# EUROPÄISCHE PATENTSCHRIFT

(11) **EP 1 037 882 B1**
(45) Veröffentlichungstag und Bekanntmachung des Hinweises auf die Patenterteilung: **05.11.2003**
(21) Anmeldenummer: 98965711.9
(22) Anmeldetag: 28.11.1998
(51) Int. Cl.: C07D 251/48, C07D 251/42, C07C 279/26, A01N 43/68

(54) **SUBSTITUIERTE 2,4-DIAMINO-1,3,5-TRIAZINE**
SUBSTITUTED 2,4-DIAMINO-1,3,5-TRIAZINES
2,4-DIAMINO-1,3,5-TRIAZINES SUBSTITUEES

(30) Priorität: 11.12.1997 DE 19755016
(43) Veröffentlichungstag der Anmeldung: 27.09.2000
(73) Patentinhaber: Bayer CropScience AG, 40789 Monheim (DE); Bayer CropScience K.K., Tokyo (JP)
(72) Erfinder: VOIGT, Katharina, D-50080 Aachen (DE); RIEBEL, Hans-Jochem, D-42113 Wuppertal (DE); LEHR, Stefan, D-51381 Leverkusen (DE); LENDER, Andreas, D-42327 Wuppertal (DE); KIRSTEN, Rolf, D-40789 Monheim (DE); DOLLINGER, Markus, Overland Park, KS 66213 (US); DREWES, Mark-Wilhelm, D-40764 Langenfeld (DE); WETCHOLOWSKY, Ingo, D-51371 Leverkusen (DE); WATANABE, Yukiyoshi, Oyama, Tochigi 323 (JP); GOTO, Toshio, Shimotsuga-gun, Tochigi 329-04 (JP); MYERS, Randy, Allen, D-40789 Düsseldorf (DE)
(74) Vertreter: Krieg, Robert Alexander, Dr.
(86) Internationale Anmeldenummer: EP9807691
(87) Internationale Veröffentlichungsnummer: WO99029677

(56) Entgegenhaltungen:
- EP-A- 0 273 328
- EP-A- 0 411 153
- WO-A-95/11237
- DE-A- 1 905 683
- DE-A- 1 911 527
- DE-A- 2 240 887
- V. OLUIC-VUKOVIC ET AL.: J. SERB. CHEM. SOC., Bd. 50, Nr. 3, 1985, Seiten 121-4, XP002102084

## Beschreibung

Die Erfindung betrifft neue substituierte 2,4-Diamino-1,3,5-triazine, Verfahren und neue Zwischenprodukte zu ihrer Herstellung und ihre Verwendung als Herbizide.

Eine Reihe von substituierten Aryloxyalkylaminotriazinen ist bereits aus der (Patent)-Literatur bekannt (vgl. EP-273 328, EP-411 153 / WO 90/09378). Diese Verbindungen haben jedoch bisher keine besondere Bedeutung erlangt.

Auch aus anderen wissenschaftlichen Publikationen sind substituierte Aryloxyalkylaminotriazine bekannt (vgl. V. Oluic-Vukovic et al., J. Serb. Chem. Soc., Bd. 50, Nr. 3, 1985, Seiten 121-124).

Es wurden nun die neuen substituierten 2,4-Diamino-1,3,5-triazine der allgemeinen Formel (I) 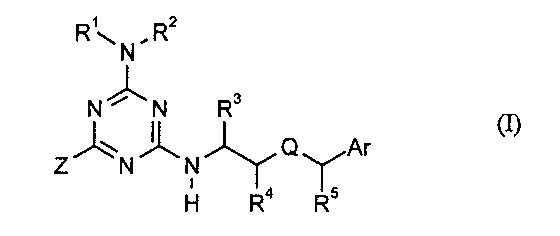 in welcher
- Q: für O (Sauerstoff), S (Schwefel), SO, SO₂, NH oder N(C₁-C₄-Alkyl) steht,
- R¹: für Wasserstoff oder für gegebenenfalls durch Hydroxy, Cyano, Halogen oder C₁-C₄-Alkoxy substituiertes Alkyl mit 1 bis 6 Kohlenstoffatomen steht,
- R²: für Wasserstoff, für Formyl oder für jeweils gegebenenfalls durch Cyano, Halogen oder C₁-C₄-Alkoxy substituiertes Alkyl, Alkylcarbonyl, Alkoxycarbonyl oder Alkylaminocarbonyl mit jeweils 1 bis 6 Kohlenstoffatomen in den Alkylgruppen steht,
- R³: für gegebenenfalls durch Hydroxy, Cyano, Halogen oder C₁-C₄-Alkoxy substituiertes Alkyl mit 1 bis 6 Kohlenstoffatomen oder für gegebenenfalls durch Cyano, Halogen oder C₁-C₄-Alkyl substituiertes Cycloalkyl mit 3 bis 6 Kohlenstoffatomen steht,
- R⁴: für Wasserstoff oder Alkyl mit 1 bis 4 Kohlenstoffatomen steht,
- R⁵: für Wasserstoff oder Alkyl mit 1 bis 4 Kohlenstoffatomen steht,
- Ar: für jeweils gegebenenfalls substituiertes Phenyl, Naphthyl, Tetralinyl oder Heteroaryl steht,
wobei die möglichen Heteroarylgruppierungen vorzugsweise aus folgender Gruppe ausgewählt sind:
Furyl, Benzofuryl, Thienyl, Benzothienyl, Thiazolyl, Benzthiazolyl, Oxazolyl, Benzoxazolyl, Isoxazolyl, Thiadiazolyl, Oxadiazolyl, Pyrazolyl, Imidazolyl, Pyrrolyl, Pyridinyl, Pyrimidinyl,
und wobei die möglichen Substituenten jeweils vorzugsweise aus folgender Gruppe ausgewählt sind:
Hydroxy, Cyano, Carbamoyl, Thiocarbamoyl, Nitro, Halogen, jeweils gegebenenfalls durch Hydroxy, Cyano oder Halogen substituiertes Alkyl oder Alkoxy mit jeweils 1 bis 6 Kohlenstoffatomen, jeweils gegebenenfalls durch Halogen substituiertes Alkylcarbonyl, Alkoxycarbonyl, Alkylthio, Alkylsulfinyl, Alkylsulfonyl, Dialkylamino, Alkylcarbonylamino, Alkylsulfonylamino, Bis-alkylcarbonyl-amino, Bis-alkylsulfonyl-amino, N-Alkyl-N-alkylcarbonyl-amino oder N-Alkyl-N-alkylsulfonyl-amino mit jeweils 1 bis 6 Kohlenstoffatomen in den Alkylgruppen, jeweils gegebenenfalls durch Hydroxy, Cyano, Nitro, Halogen, C₁-C₄-Alkyl, C₁-C₄-Halogenalkyl, C₁-C₄-Alkoxy oder C₁-C₄-Halogenalkoxy substiuiertes Phenyl oder Phenoxy, sowie jeweils gegebenenfalls durch Halogen substituiertes Methylendioxy oder Ethylendioxy,
und
- Z: für gegebenenfalls durch Hydroxy, Cyano, Halogen, C₁-C₄-Alkoxy, C₁-C₄-Alkyl-carbonyl, C₁-C₄-Alkoxy-carbonyl, C₁-C₄-Alkylthio, C₁-C₄-Alkylsulfinyl oder C₁-C₄-Alkylsulfonyl substituiertes Alkyl mit jeweils 1 bis 6 Kohlenstoffatomen in den Alkylgruppen oder für gegebenenfalls durch Cyano, Halogen oder C₁-C₄-Alkyl substituiertes Cycloalkyl mit 3 bis 6 Kohlenstoffatomen steht.

Man erhält die neuen substituierten 2,4-Diaminotriazine der allgemeinen Formel (I), wenn man
(a) Biguanide der allgemeinen Formel (II) 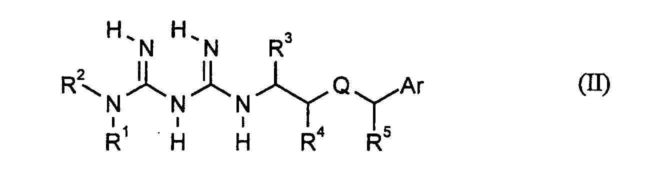 in welcher
   - Q, R¹, R², R³, R⁴, R⁵ und Ar: die oben angegebene Bedeutung haben,
   - und/oder Säureaddukte von Verbindungen der allgemeinen Formel (II) -
   mit Alkoxycarbonylverbindungen der allgemeinen Formel (III)

   Z-CO-OR' (III)

   in welcher
   - Z: die oben angegebene Bedeutung hat und
   - R': für Alkyl steht,
   gegebenenfalls in Gegenwart eines Reaktionshilfsmittels und gegebenenfalls in Gegenwart eines Verdünnungsmittels umsetzt,
   oder wenn man
(b) substituierte Halogentriazine der allgemeinen Formel (IV) 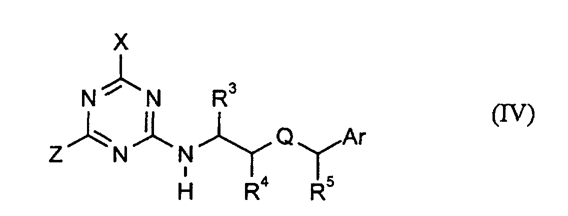 in welcher
   - Q, R³, R⁴, R⁵, Ar und Z: die oben angegebene Bedeutung haben und
   - X: für Halogen steht,
   mit Stickstoffverbindungen der allgemeinen Formel (V) 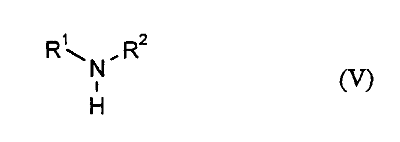 in welcher
   - R¹ und R²: die oben angegebene Bedeutung haben,
   gegebenenfalls in Gegenwart eines Reaktionshilfsmittels und gegebenenfalls in Gegenwart eines Verdünnungsmittels umsetzt,
   oder wenn man
(c) substituierte Aminotriazine der allgemeinen Formel (VI) 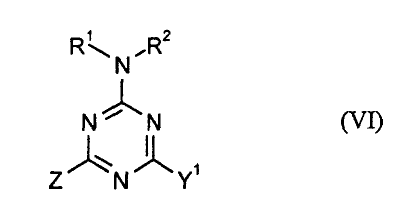 in welcher
   - R¹, R² und Z: die oben angegebene Bedeutung haben und
   - Y¹: für Halogen oder Alkoxy steht,
   mit substituierten Alkylaminen der allgemeinen Formel (VII) 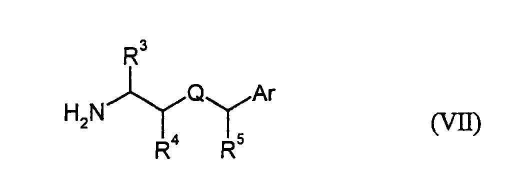 in welcher
   - Q, Ar, R³, R⁴ und R⁵: die oben angegebene Bedeutung haben,
   gegebenenfalls in Gegenwart eines Reaktionshilfsmittels und gegebenenfalls in Gegenwart eines Verdünnungsmittels umsetzt,
   oder wenn man
(d) substituierte 2,4-Diamino-1,3,5-triazine der allgemeinen Formel (Ia) 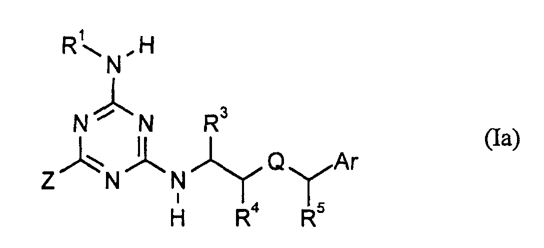 in welcher
   - Q, R¹, R³, R⁴, R⁵, Ar und Z: die oben angegebene Bedeutung haben,
   mit Alkylierungs- oder Acylierungsmitteln der allgemeinen Formel (VIII)

   Y²-R² (VIII)

   in welcher
   - R²: mit Ausnahme von Wasserstoff die oben angegebene Bedeutung hat und
   - Y²: für Halogen, -O-R² oder -O-CO-R² steht,
   gegebenenfalls in Gegenwart eines Reaktionshilfsmittels und gegebenenfalls in Gegenwart eines Verdünnungsmittels umsetzt,
und gegebenenfalls an den gemäß den unter (a), (b), (c) oder (d) beschriebenen Verfahren erhaltenen Verbindungen der allgemeinen Formel (I) im Rahmen der obigen Substituentendefinition weitere Umwandlungen nach üblichen Methoden durchführt.

Die neuen substituierten 2,4-Diamino-1,3,5-triazine der allgemeinen Formel (I) zeichnen sich durch starke und selektive herbizide Wirksamkeit aus.

Die erfindungsgemäßen Verbindungen der allgemeinen Formel (I) enthalten mindestens ein asymmetrisch substituiertes Kohlenstoffatom und können deshalb in verschiedenen enantiomeren (R- und S- konfigurierten Formen) bzw. diasteromeren Formen vorliegen. Die Erfindung betrifft sowohl die verschiedenen möglichen einzelnen enantiomeren bzw. stereoisomeren Formen der Verbindungen der allgemeinen Formel (I) wie auch die Gemische dieser isomeren Verbindungen.

In den Definitionen sind die Kohlenwasserstoffketten, wie Alkyl - auch in Verbindung mit Heteroatomen, wie in Alkoxy oder Alkylthio - jeweils geradkettig oder verzweigt. Halogen steht im allgemeinen für Fluor, Chlor, Brom oder Iod, vorzugsweise für Fluor, Chlor oder Brom, insbesondere für Fluor oder Chlor.

Aus den vorausgehend definierten Verbindungen der Formel (I) seien folgende Gruppen besonders herausgehoben:
(A) die Verbindungen der Formel (I), in welcher Q, R¹, R², R³, R⁴, R⁵ und Z die vorausgehend angegebene Bedeutung haben und Ar für jeweils gegebenenfalls substituiertes Phenyl oder Naphthyl steht, wobei die möglichen Substituenten die vorausgehend angegebene Bedeutung haben;
(B) die Verbindungen der Formel (I), in welcher Q, R¹, R², R³, R⁴, R⁵ und Z die vorausgehend angegebene Bedeutung haben und Ar für gegebenenfalls substituiertes Heterocyclyl steht, wobei die möglichen Heterocyclylgruppierungen und die möglichen Substituenten die vorausgehend angegebene Bedeutung haben.

Die Erfindung betrifft insbesondere Verbindungen der Formel (I), in welcher
- Q: für O (Sauerstoff), S (Schwefel) oder NH steht,
- R¹: für Wasserstoff oder für jeweils gegebenenfalls durch Hydroxy, Cyano, Fluor, Chlor, Methoxy oder Ethoxy substituiertes Methyl, Ethyl, n- oder i-Propyl, n-, i-, s- oder t-Butyl steht,
- R²: für Wasserstoff, für Formyl, oder für jeweils gegebenenfalls durch Cyano, Fluor, Chlor, Brom, Methoxy oder Ethoxy substituiertes Methyl, Ethyl, Acetyl, Propionyl, n- oder i-Butyroyl, Methoxycarbonyl, Ethoxycarbonyl, n- oder i-Propoxycarbonyl, Methylaminocarbonyl, Ethylaminocarbonyl, n- oder i-Propylaminocarbonyl steht,
- R³: für jeweils gegebenenfalls durch Hydroxy, Cyano, Fluor, Chlor, Methoxy oder Ethoxy substituiertes Ethyl, n- oder i-Propyl, n-, i-, s- oder t-Butyl oder für die Substituenten des Kohlenstoffatoms, an das R³ gebunden ist, in S-Konfiguration angeordnet sind.

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgangs- oder Zwischenprodukte. Diese Restedefinitionen können untereinander, also auch zwischen den angegebenen bevorzugten Bereichen beliebig kombiniert werden.

Beispiele für die erfindungsgemäßen Verbindungen der Formel (I) sind in den nachstehenden Gruppen aufgeführt. Die allgemeinen Formeln stehen hierbei jeweils für die R-Enantiomeren, die S-Enantiomeren und die Racemate.

### Gruppe 1

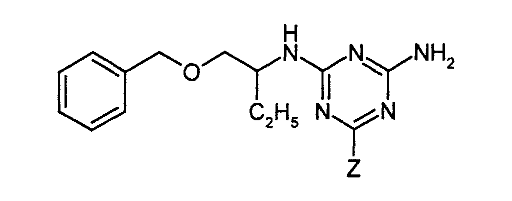

Z hat hierbei beispielhaft die in der nachstehenden Aufzählung angegeben Bedeutungen:
Methyl, Ethyl, n- oder i-Propyl, n-, i-, s- oder t-Butyl, Fluormethyl, Difluormethyl, Trifluormethyl, Chlormethyl, Dichlormethyl, Chlorfluormethyl, Chlorbrommethyl, Chlordifluormethyl, Fluordichlormethyl, Bromdifluormethyl, Trichlormethyl, 1-Fluor-ethyl, 2-Fluor-ethyl, 1-Chlor-ethyl, 2-Chlor-ethyl, 1-Brom-ethyl, 1-Chlor-1-fluor-ethyl, 1-Fluor-propyl, 2-Fluor-propyl, 3-Fluor-propyl, 1-Chlor-propyl, 2-Chlor-propyl, 3-Chlor-propyl, 1-Brom-propyl, 1-Fluor-1-methyl-ethyl, 2-Fluor-1-methylethyl, 1-Chlor-1-methyl-ethyl, 1-Fluor-1-methyl-propyl, 1-Chlor-1-ethyl-propyl, 1-Fluor-1-ethyl-propyl, 1-Fluor-2-methyl-propyl, 1-Chlor-2-methyl-propyl, 2-Chlor-1-methyl-ethyl, 1,1-Difluor-ethyl, 1,2-Difluor-ethyl, 1,1-Dichlor-ethyl, 2,2,2-Trifluorethyl, 1,2,2,2-Tetrafluor-ethyl, Perfluorethyl, 1,1-Difluor-propyl, 1,1-Dichlor-propyl, Perfluorpropyl, 1-Fluor-butyl, 1-Chlor-butyl, Perfluorbutyl, Perfluorpentyl, Perfluorhexyl, 1-Hydroxy-ethyl, 1-Hydroxy-1-methyl-ethyl, 1-Hydroxy-propyl, Methoxymethyl, Ethoxymethyl, Dimethoxy-methyl, 1-Methoxyethyl, 2-Methoxy-ethyl, 1,1-Dimethoxy-ethyl, 1-Ethoxyethyl, 2-Ethoxy-ethyl, 2,2-Dimethoxy-ethyl, 2,2-Diethoxy-ethyl, 2-Methoxy-1-methyl-ethyl, 2-Methoxy-1-ethyl-ethyl, 2-Ethoxy-1-methyl-ethyl, 2-Ethoxy-1-ethyl-ethyl, 2,2-Bis-methoxy-methyl, Methylthiomethyl, Ethylthiomethyl, 1-Methylthio-ethyl, 2-Methylthioethyl, 1-Ethylthio-ethyl, 2-Ethylthioethyl, Methylsulfinylmethyl, Ethylsulfinylmethyl, Methylsulfonylmethyl, Ethylsulfonylmethyl, Cyclopropyl, 1-Cyano-cyclopropyl, 1-Fluor-cyclopropyl, 1-Chlor-cyclopropyl, 2-Cyano-cyclopropyl, 2-Fluorcyclopropyl, 2-Chlor-cyclopropyl, 2,2-Difluor-cyclopropyl,
Cyclobutyl, 2,2-Difluor-cyclobutyl, 2,2,3-Trifluor-cyclobutyl, 2,2-Difluor-3-chlorcyclobutyl, Cyclopentyl, Cyclohexyl.

### Gruppe 2

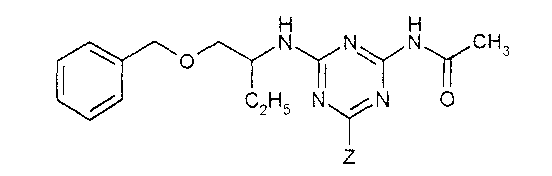

Z hat hierbei beispielhaft die oben in Gruppe 1 angegebenen Bedeutungen.

### Gruppe 3

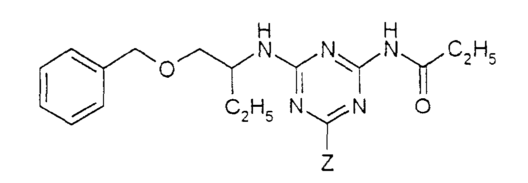

Z hat hierbei beispielhaft die oben in Gruppe 1 angegebenen Bedeutungen.

### Gruppe 4

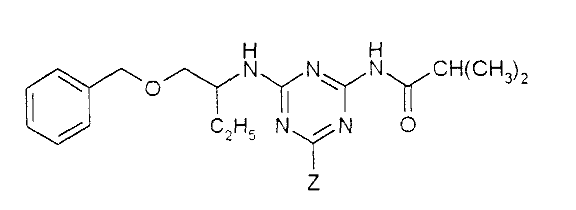

Z hat hierbei beispielhaft die oben in Gruppe 1 angegebenen Bedeutungen.

### Gruppe 5

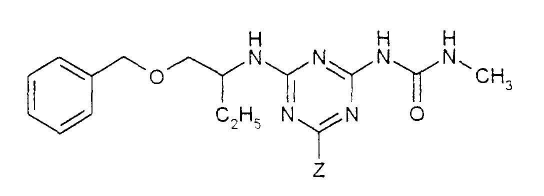

Z hat hierbei beispielhaft die oben in Gruppe 1 angegebenen Bedeutungen.

### Gruppe 6

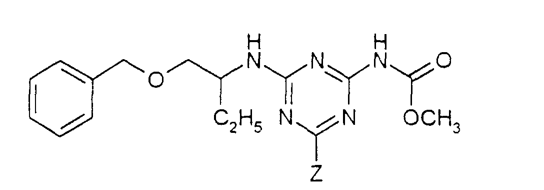

Z hat hierbei beispielhaft die oben in Gruppe 1 angegebenen Bedeutungen.

### Gruppe 7

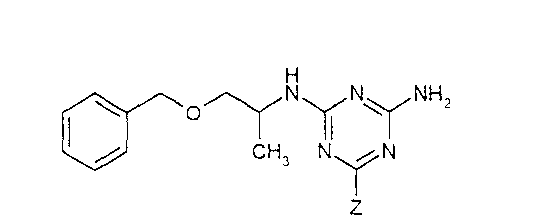

Z hat hierbei beispielhaft die oben in Gruppe 1 angegebenen Bedeutungen

### Gruppe 8

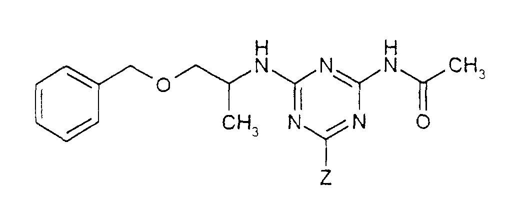

Z hat hierbei beispielhaft die oben in Gruppe 1 angegebenen Bedeutungen.

### Gruppe 9

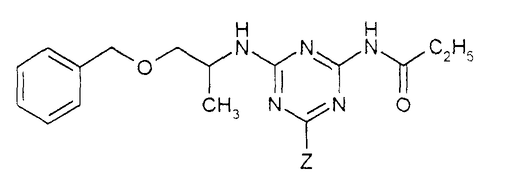

Z hat hierbei beispielhaft die oben in Gruppe 1 angegebenen Bedeutungen

### Gruppe 10

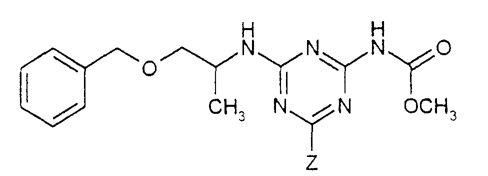

Z hat hierbei beispielhaft die oben in Gruppe 1 angegebenen Bedeutungen.

### Gruppe 11

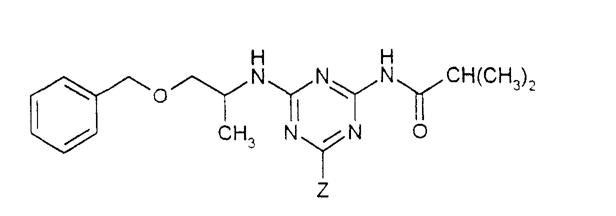

Z hat hierbei beispielhaft die oben in Gruppe 1 angegebenen Bedeutungen.

### Gruppe 12

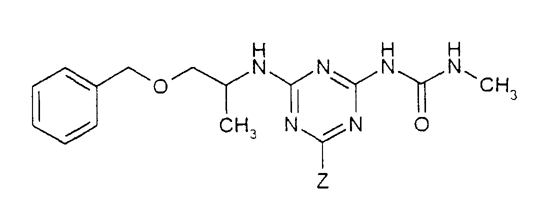

Z hat hierbei beispielhaft die oben in Gruppe 1 angegebenen Bedeutungen.

### Gruppe 13

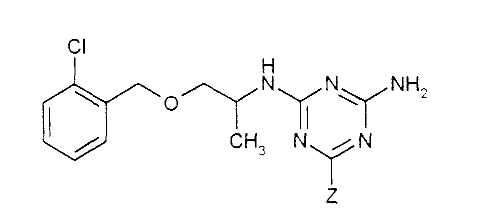

Z hat hierbei beispielhaft die oben in Gruppe 1 angegebenen Bedeutungen.

### Gruppe 14

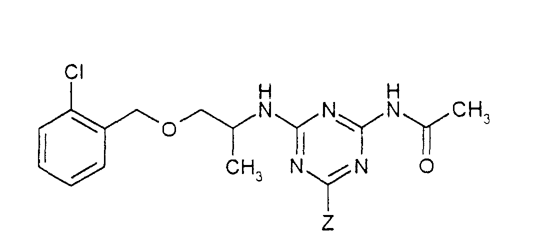

Z hat hierbei beispielhaft die oben in Gruppe 1 angegebenen Bedeutungen

### Gruppe 15

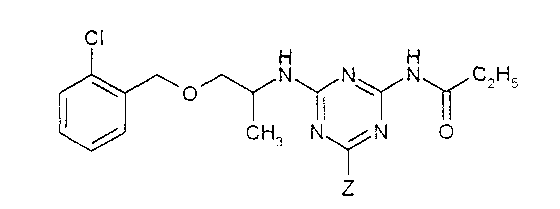

Z hat hierbei beispielhaft die oben in Gruppe 1 angegebenen Bedeutungen.

### Gruppe 16

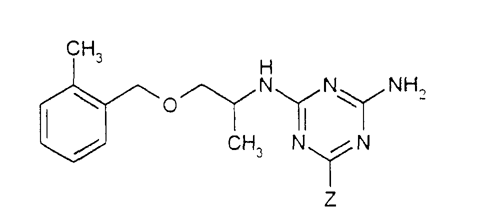

Z hat hierbei beispielhaft die oben in Gruppe 1 angegebenen Bedeutungen.

### Gruppe 17

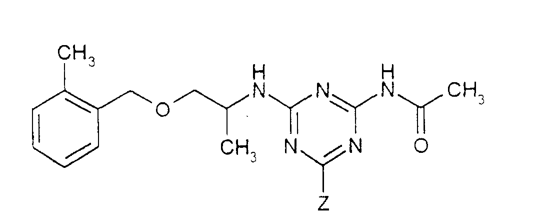

Z hat hierbei beispielhaft die oben in Gruppe 1 angegebenen Bedeutungen

### Gruppe 18

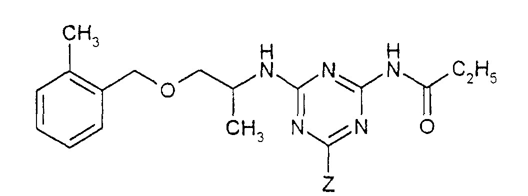

Z hat hierbei beispielhaft die oben in Gruppe 1 angegebenen Bedeutungen.

### Gruppe 19

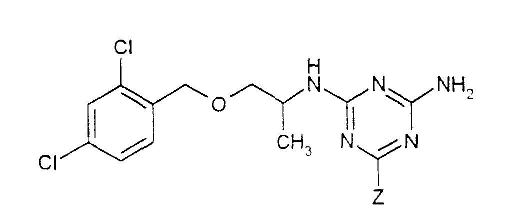

Z hat hierbei beispielhaft die oben in Gruppe 1 angegebenen Bedeutungen.

### Gruppe 20

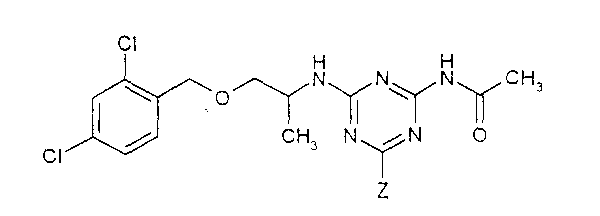

Z hat hierbei beispielhaft die oben in Gruppe 1 angegebenen Bedeutungen.

### Gruppe 21

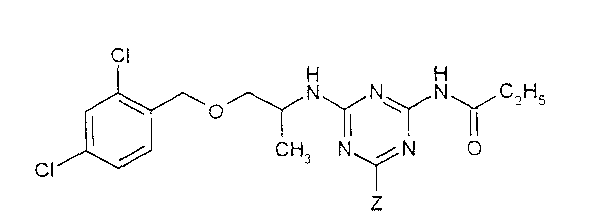

Z hat hierbei beispielhaft die oben in Gruppe 1 angegebenen Bedeutungen.

### Gruppe 22

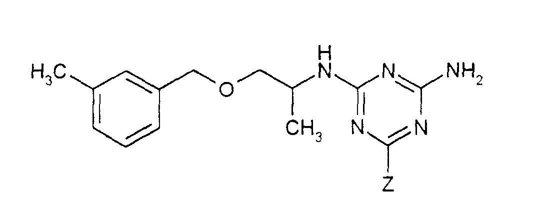

Z hat hierbei beispielhaft die oben in Gruppe 1 angegebenen Bedeutungen.

### Gruppe 23

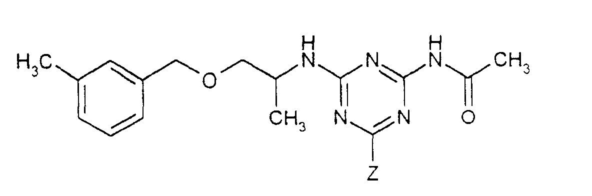

Z hat hierbei beispielhaft die oben in Gruppe 1 angegebenen Bedeutungen

### Gruppe 24

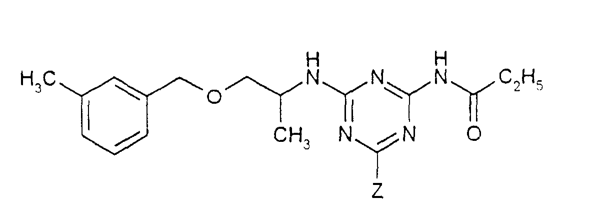

Z hat hierbei beispielhaft die oben in Gruppe 1 angegebenen Bedeutungen

### Gruppe 25

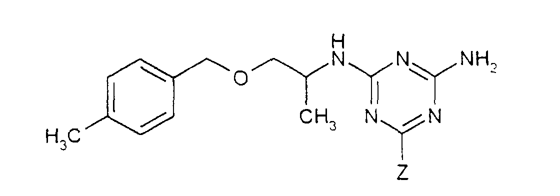

Z hat hierbei beispielhaft die oben in Gruppe 1 angegebenen Bedeutungen

### Gruppe 26

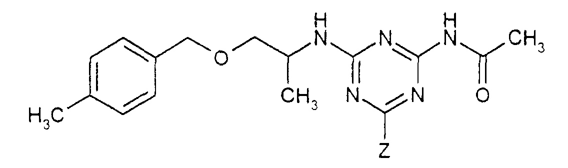

Z hat hierbei beispielhaft die oben in Gruppe 1 angegebenen Bedeutungen.

### Gruppe 27

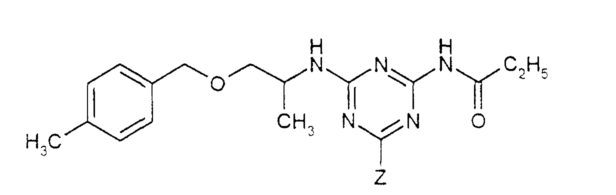

Z hat hierbei beispielhaft die oben in Gruppe 1 angegebenen Bedeutungen.

### Gruppe 28

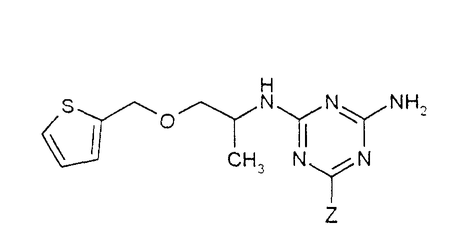

Z hat hierbei beispielhaft die oben in Gruppe 1 angegebenen Bedeutungen

### Gruppe 29

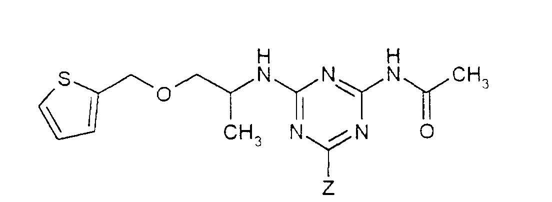

Z hat hierbei beispielhaft die oben in Gruppe 1 angegebenen Bedeutungen.

### Gruppe 30

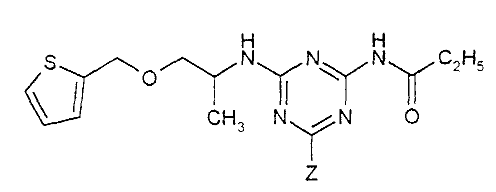

Z hat hierbei beispielhaft die oben in Gruppe 1 angegebenen Bedeutungen.

### Gruppe 31

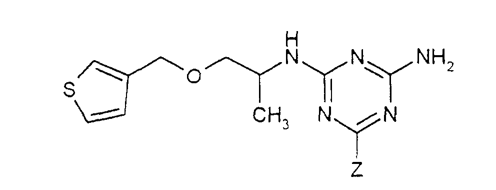

Z hat hierbei beispielhaft die oben in Gruppe 1 angegebenen Bedeutungen.

### Gruppe 32

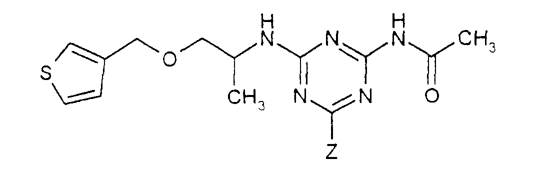

Z hat hierbei beispielhaft die oben in Gruppe 1 angegebenen Bedeutungen.

### Gruppe 33

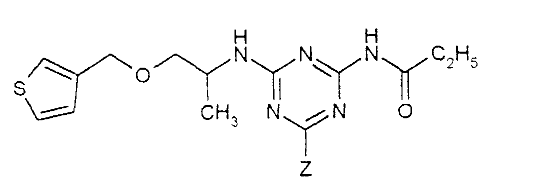

Z hat hierbei beispielhaft die oben in Gruppe 1 angegebenen Bedeutungen.

### Gruppe 34

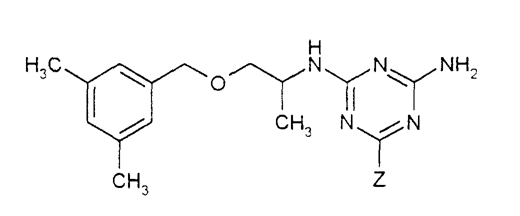

Z hat hierbei beispielhaft die oben in Gruppe 1 angegebenen Bedeutungen.

### Gruppe 35

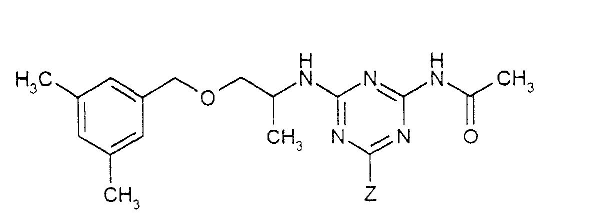

Z hat hierbei beispielhaft die oben in Gruppe 1 angegebenen Bedeutungen

### Gruppe 36

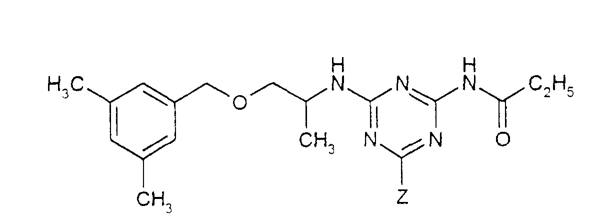

Z hat hierbei beispielhaft die oben in Gruppe 1 angegebenen Bedeutungen

### Gruppe 37

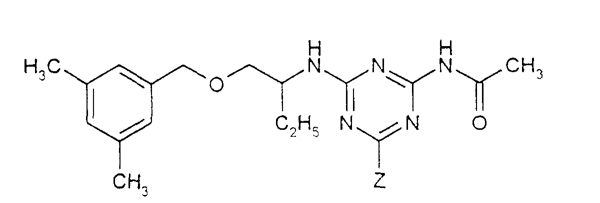

Z hat hierbei beispielhaft die oben in Gruppe 1 angegebenen Bedeutungen

### Gruppe 38

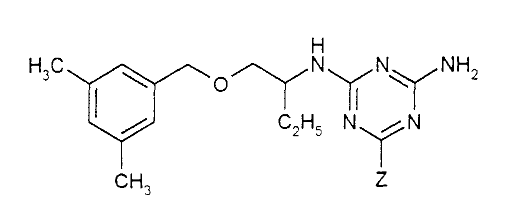

Z hat hierbei beispielhaft die oben in Gruppe 1 angegebenen Bedeutungen.

### Gruppe 39

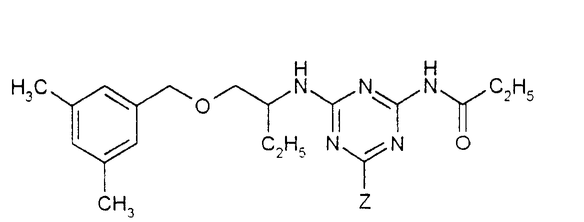

Z hat hierbei beispielhaft die oben in Gruppe 1 angegebenen Bedeutungen.

### Gruppe 40

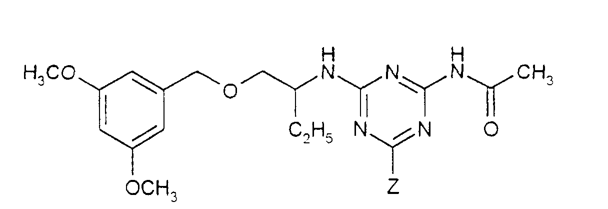

Z hat hierbei beispielhaft die oben in Gruppe 1 angegebenen Bedeutungen.

### Gruppe 41

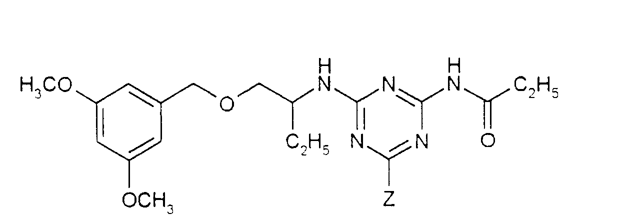

Z hat hierbei beispielhaft die oben in Gruppe 1 angegebenen Bedeutungen.

### Gruppe 42

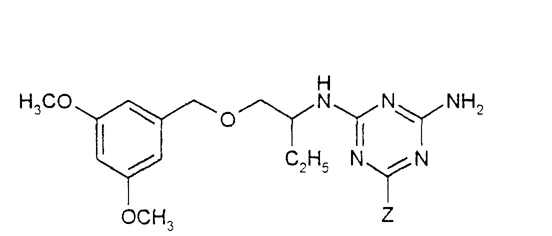

Z hat hierbei beispielhaft die oben in Gruppe 1 angegebenen Bedeutungen.

### Gruppe 43

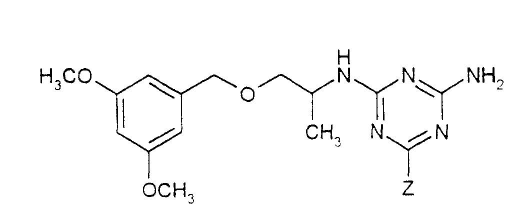

Z hat hierbei beispielhaft die oben in Gruppe 1 angegebenen Bedeutungen.

### Gruppe 44

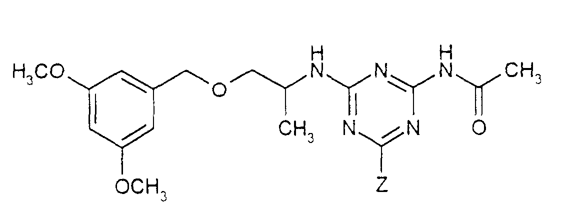

Z hat hierbei beispielhaft die oben in Gruppe 1 angegebenen Bedeutungen.

### Gruppe 45

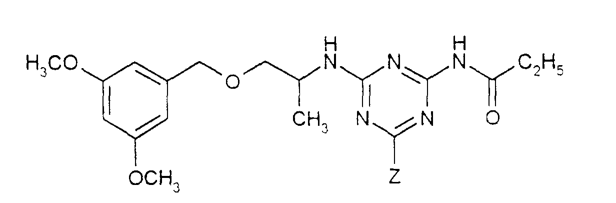

Z hat hierbei beispielhaft die oben in Gruppe 1 angegebenen Bedeutungen.

### Gruppe 46

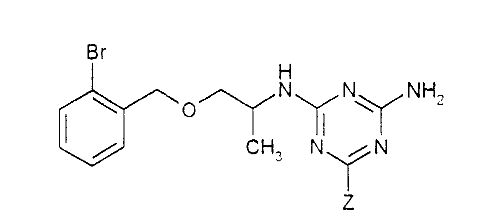

Z hat hierbei beispielhaft die oben in Gruppe 1 angegebenen Bedeutungen

### Gruppe 47

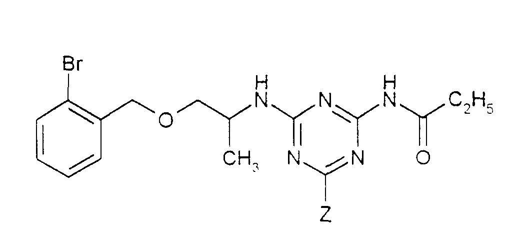

Z hat hierbei beispielhaft die oben in Gruppe 1 angegebenen Bedeutungen.

### Gruppe 48

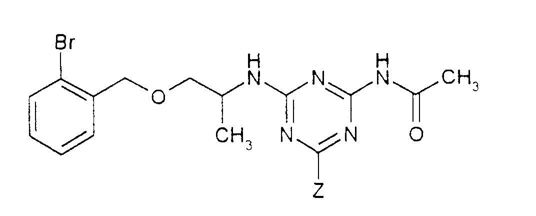

Z hat hierbei beispielhaft die oben in Gruppe 1 angegebenen Bedeutungen.

### Gruppe 49

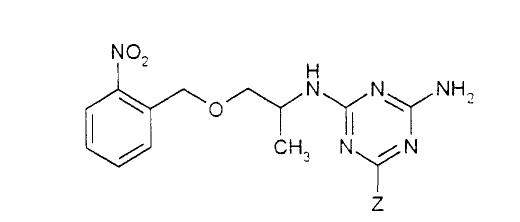

Z hat hierbei beispielhaft die oben in Gruppe 1 angegebenen Bedeutungen.

### Gruppe 50

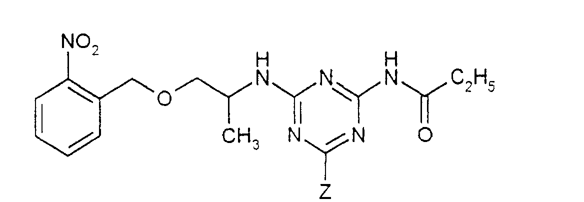

Z hat hierbei beispielhaft die oben in Gruppe 1 angegebenen Bedeutungen.

### Gruppe 51

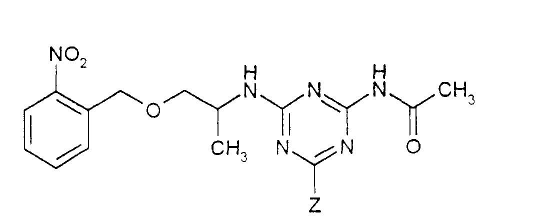

Z hat hierbei beispielhaft die oben in Gruppe 1 angegebenen Bedeutungen.

### Gruppe 52

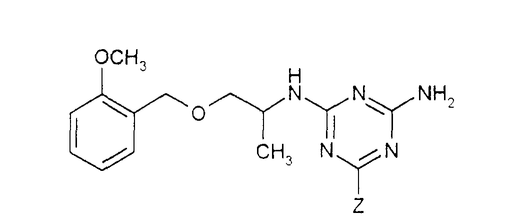

Z hat hierbei beispielhaft die oben in Gruppe 1 angegebenen Bedeutungen.

### Gruppe 53

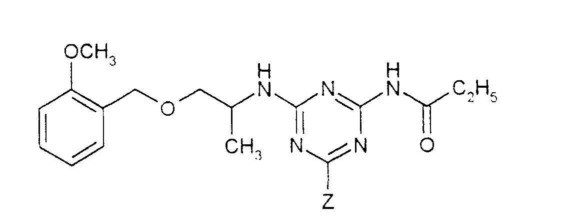

Z hat hierbei beispielhaft die oben in Gruppe 1 angegebenen Bedeutungen.

### Gruppe 54

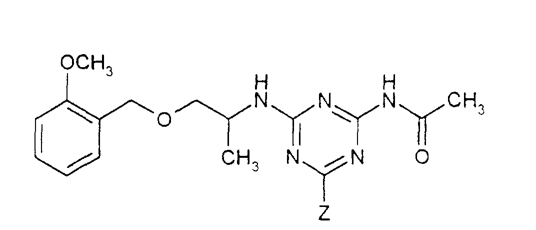

Z hat hierbei beispielhaft die oben in Gruppe 1 angegebenen Bedeutungen.

### Gruppe 55

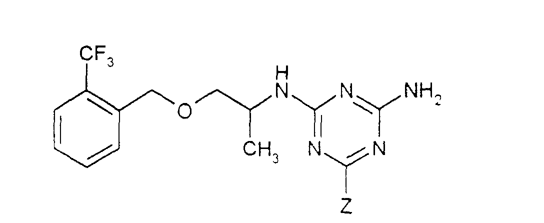

Z hat hierbei beispielhaft die oben in Gruppe 1 angegebenen Bedeutungen.

### Gruppe 56

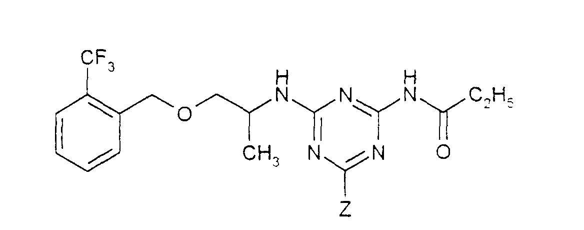

Z hat hierbei beispielhaft die oben in Gruppe 1 angegebenen Bedeutungen.

### Gruppe 57

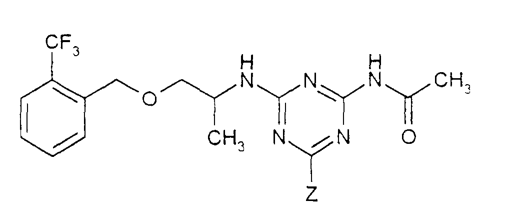

Z hat hierbei beispielhaft die oben in Gruppe 1 angegebenen Bedeutungen.

### Gruppe 58

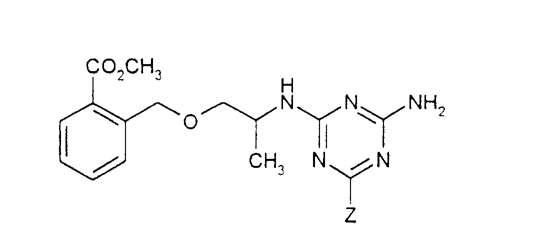

Z hat hierbei beispielhaft die oben in Gruppe 1 angegebenen Bedeutungen.

### Gruppe 59

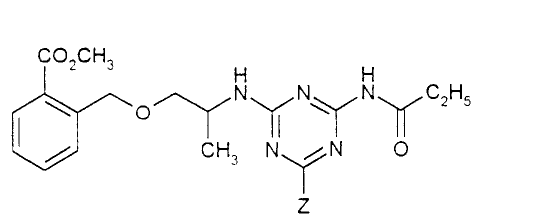

Z hat hierbei beispielhaft die oben in Gruppe 1 angegebenen Bedeutungen.

### Gruppe 60

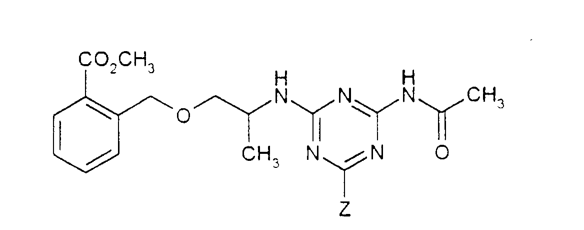

Z hat hierbei beispielhaft die oben in Gruppe 1 angegebenen Bedeutungen.

### Gruppe 61

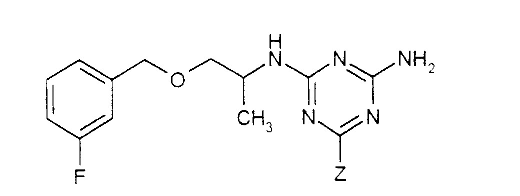

Z hat hierbei beispielhaft die oben in Gruppe 1 angegebenen Bedeutungen.

### Gruppe 62

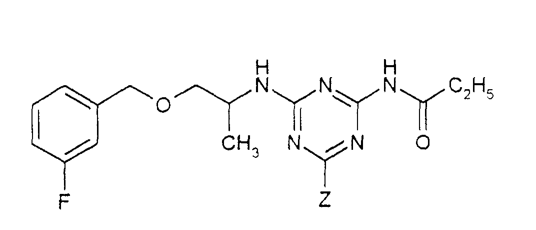

Z hat hierbei beispielhaft die oben in Gruppe 1 angegebenen Bedeutungen.

### Gruppe 63

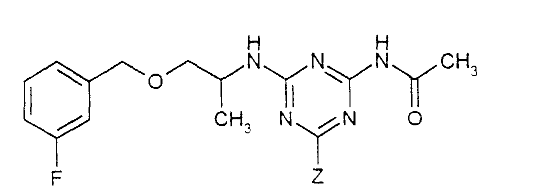

Z hat hierbei beispielhaft die oben in Gruppe 1 angegebenen Bedeutungen.

### Gruppe 64

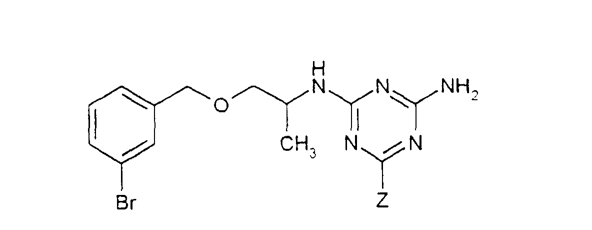

Z hat hierbei beispielhaft die oben in Gruppe 1 angegebenen Bedeutungen.

### Gruppe 65

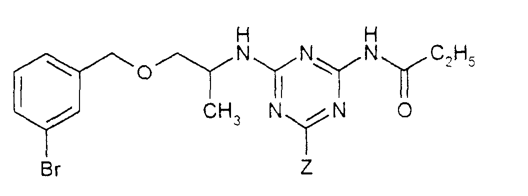

Z hat hierbei beispielhaft die oben in Gruppe 1 angegebenen Bedeutungen

### Gruppe 66

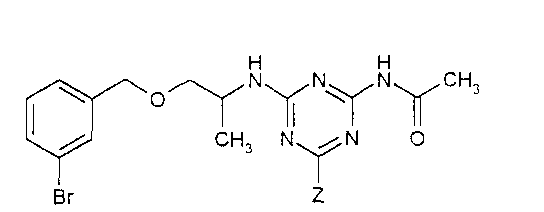

Z hat hierbei beispielhaft die oben in Gruppe 1 angegebenen Bedeutungen.

### Gruppe 67

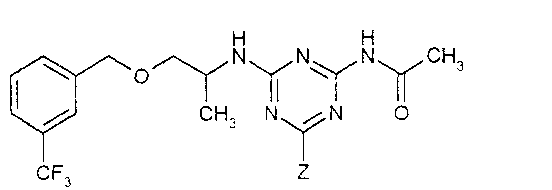

Z hat hierbei beispielhaft die oben in Gruppe 1 angegebenen Bedeutungen.

### Gruppe 68

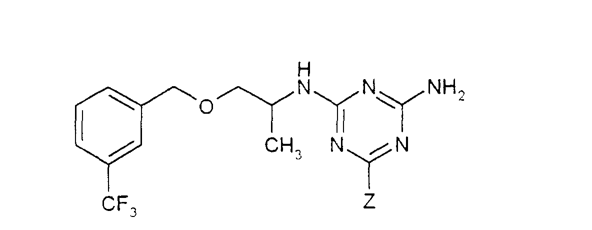

Z hat hierbei beispielhaft die oben in Gruppe 1 angegebenen Bedeutungen.

### Gruppe 69

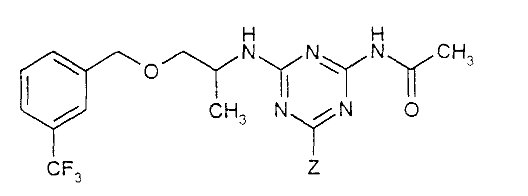

Z hat hierbei beispielhaft die oben in Gruppe 1 angegebenen Bedeutungen

### Gruppe 70

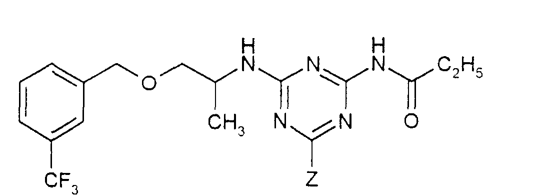

Z hat hierbei beispielhaft die oben in Gruppe 1 angegebenen Bedeutungen.

### Gruppe 71

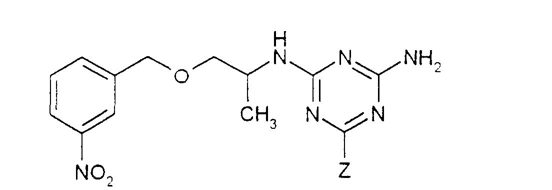

Z hat hierbei beispielhaft die oben in Gruppe 1 angegebenen Bedeutungen.

### Gruppe 72

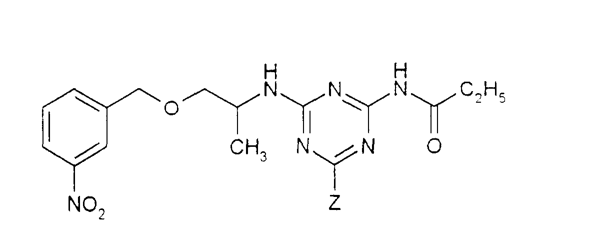

Z hat hierbei beispielhaft die oben in Gruppe 1 angegebenen Bedeutungen.

### Gruppe 73

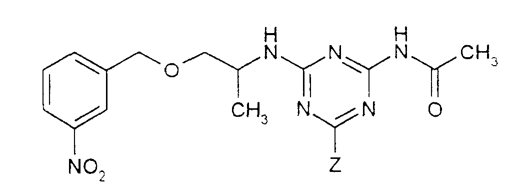

Z hat hierbei beispielhaft die oben in Gruppe 1 angegebenen Bedeutungen.

### Gruppe 74

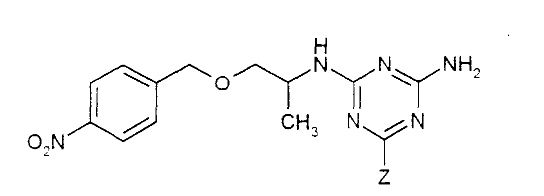

Z hat hierbei beispielhaft die oben in Gruppe 1 angegebenen Bedeutungen

### Gruppe 75

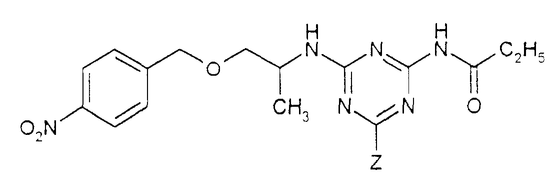

Z hat hierbei beispielhaft die oben in Gruppe 1 angegebenen Bedeutungen.

### Gruppe 76

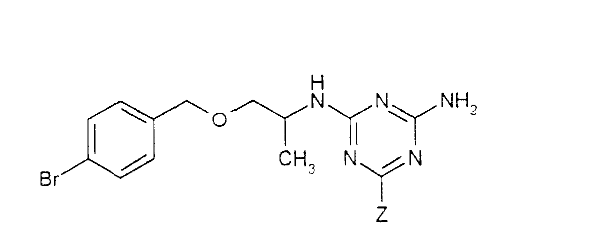

Z hat hierbei beispielhaft die oben in Gruppe 1 angegebenen Bedeutungen.

### Gruppe 77

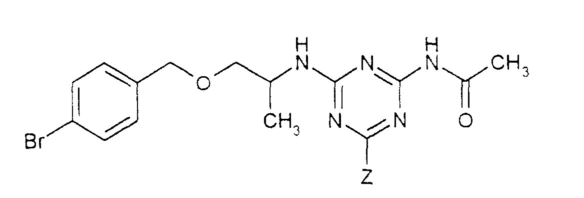

Z hat hierbei beispielhaft die oben in Gruppe 1 angegebenen Bedeutungen.

### Gruppe 78

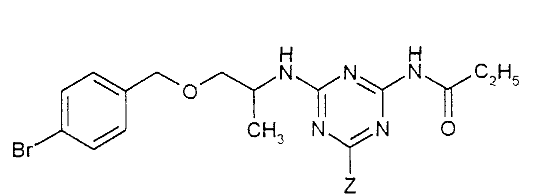

Z hat hierbei beispielhaft die oben in Gruppe 1 angegebenen Bedeutungen

### Gruppe 79

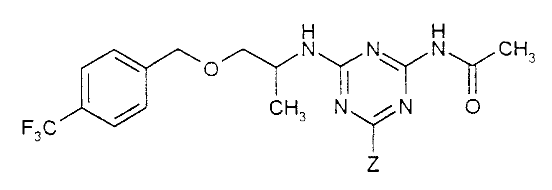

Z hat hierbei beispielhaft die oben in Gruppe 1 angegebenen Bedeutungen

### Gruppe 80

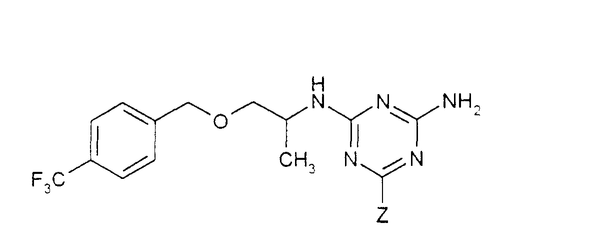

Z hat hierbei beispielhaft die oben in Gruppe 1 angegebenen Bedeutungen.

### Gruppe 81

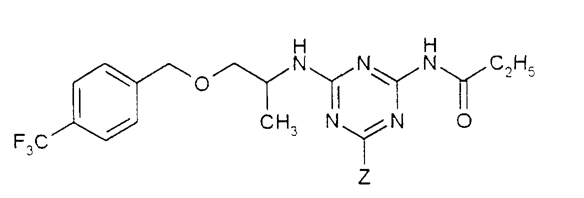

Z hat hierbei beispielhaft die oben in Gruppe 1 angegebenen Bedeutungen

### Gruppe 82

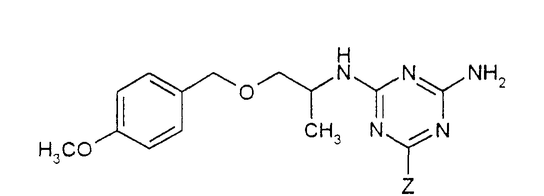

Z hat hierbei beispielhaft die oben in Gruppe 1 angegebenen Bedeutungen.

### Gruppe 83

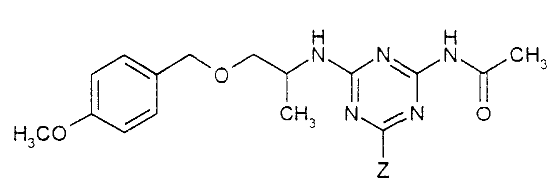

Z hat hierbei beispielhaft die oben in Gruppe 1 angegebenen Bedeutungen.

### Gruppe 84

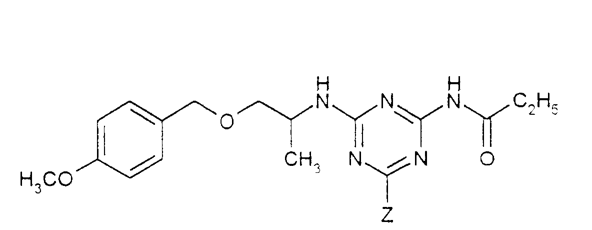

Z hat hierbei beispielhaft die oben in Gruppe 1 angegebenen Bedeutungen.

### Gruppe 85

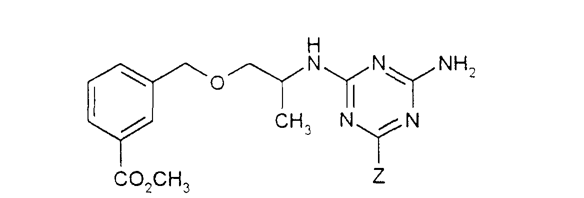

Z hat hierbei beispielhaft die oben in Gruppe 1 angegebenen Bedeutungen.

### Gruppe 86

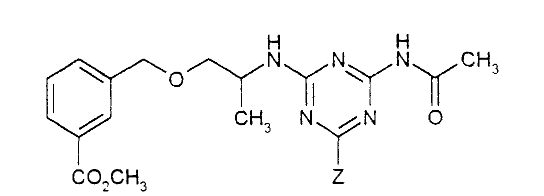

Z hat hierbei beispielhaft die oben in Gruppe 1 angegebenen Bedeutungen.

### Gruppe 87

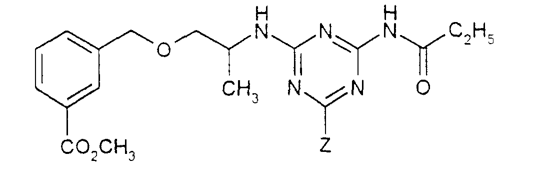

Z hat hierbei beispielhaft die oben in Gruppe 1 angegebenen Bedeutungen.

### Gruppe 88

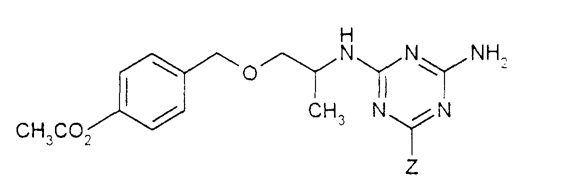

Z hat hierbei beispielhaft die oben in Gruppe 1 angegebenen Bedeutungen.

### Gruppe 89

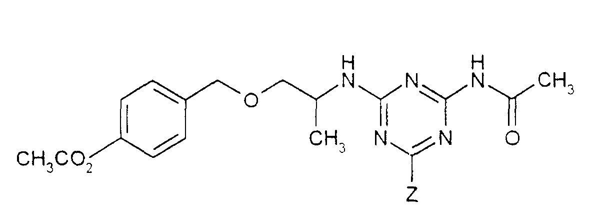

Z hat hierbei beispielhaft die oben in Gruppe 1 angegebenen Bedeutungen.

### Gruppe 90

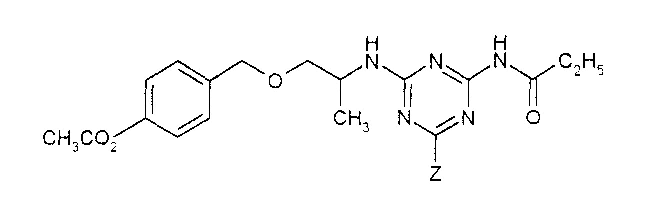

Z hat hierbei beispielhaft die oben in Gruppe 1 angegebenen Bedeutungen.

### Gruppe 91

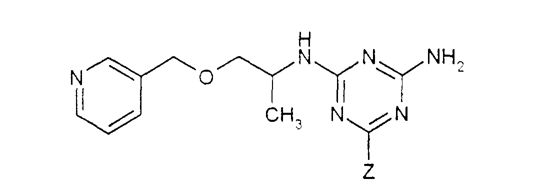

Z hat hierbei beispielhaft die oben in Gruppe 1 angegebenen Bedeutungen.

### Gruppe 92

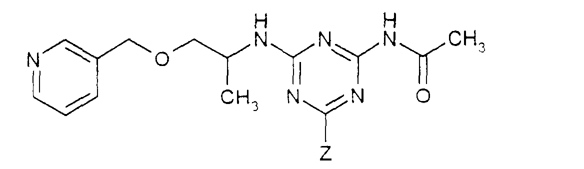

Z hat hierbei beispielhaft die oben in Gruppe 1 angegebenen Bedeutungen.

### Gruppe 93

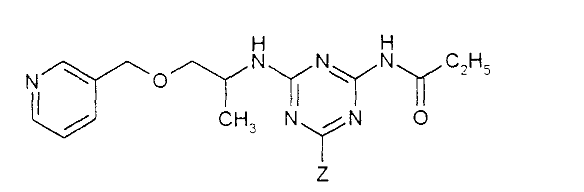

Z hat hierbei beispielhaft die oben in Gruppe 1 angegebenen Bedeutungen.

### Gruppe 94

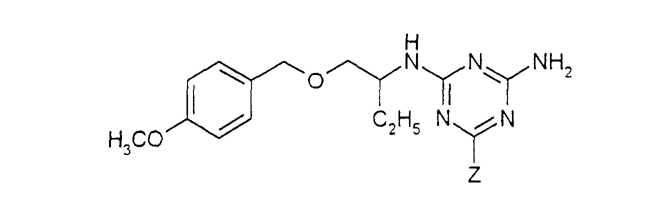

Z hat hierbei beispielhaft die oben in Gruppe 1 angegebenen Bedeutungen.

### Gruppe 95

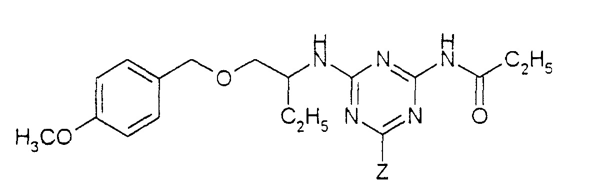

Z hat hierbei beispielhaft die oben in Gruppe 1 angegebenen Bedeutungen.

### Gruppe 96

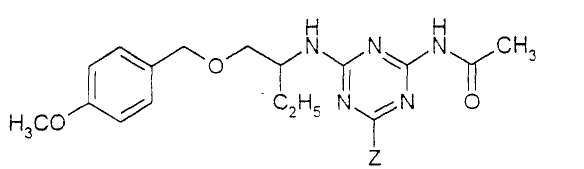

Z hat hierbei beispielhaft die oben in Gruppe 1 angegebenen Bedeutungen.

### Gruppe 97

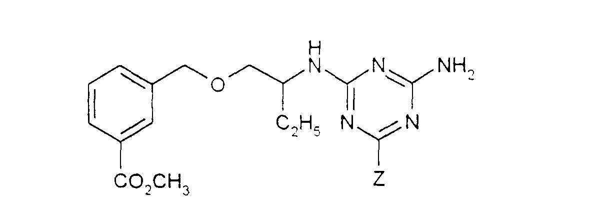

Z hat hierbei beispielhaft die oben in Gruppe 1 angegebenen Bedeutungen.

### Gruppe 98

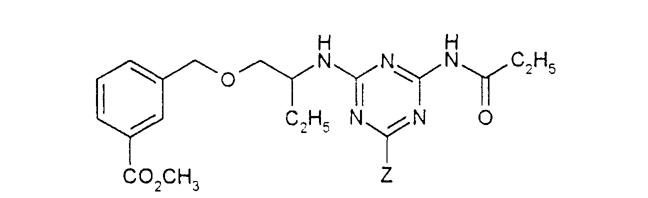

Z hat hierbei beispielhaft die oben in Gruppe 1 angegebenen Bedeutungen.

### Gruppe 99

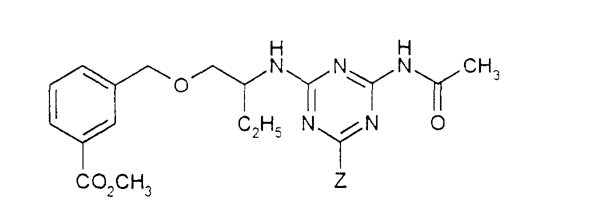

Z hat hierbei beispielhaft die oben in Gruppe 1 angegebenen Bedeutungen.

### Gruppe 100

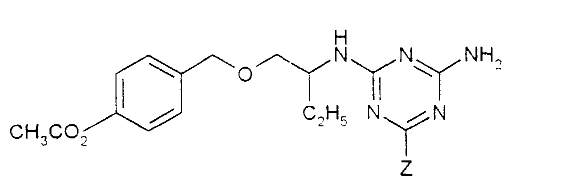

Z hat hierbei beispielhaft die oben in Gruppe 1 angegebenen Bedeutungen

### Gruppe 101

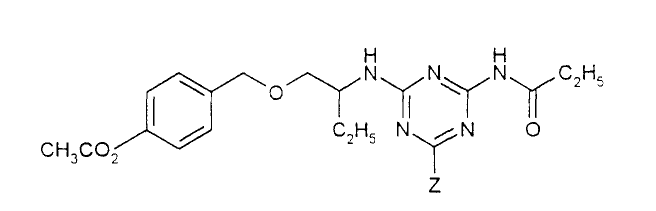

Z hat hierbei beispielhaft die oben in Gruppe 1 angegebenen Bedeutungen.

### Gruppe 102

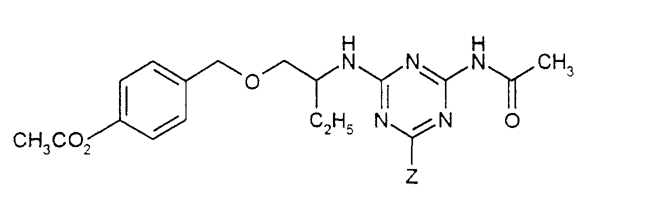

Z hat hierbei beispielhaft die oben in Gruppe 1 angegebenen Bedeutungen.

### Gruppe 103

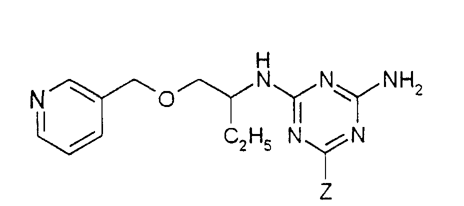

Z hat hierbei beispielhaft die oben in Gruppe 1 angegebenen Bedeutungen.

### Gruppe 104

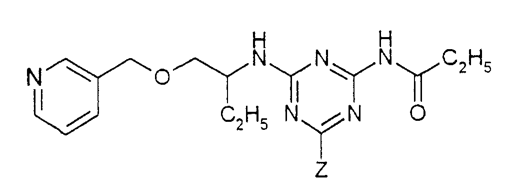

Z hat hierbei beispielhaft die oben in Gruppe 1 angegebenen Bedeutungen.

### Gruppe 105

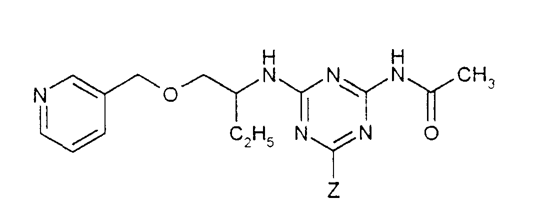

Z hat hierbei beispielhaft die oben in Gruppe 1 angegebenen Bedeutungen.

### Gruppe 106

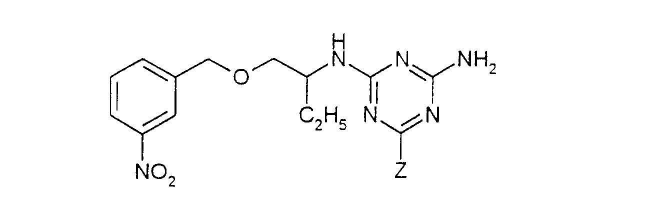

Z hat hierbei beispielhaft die oben in Gruppe 1 angegebenen Bedeutungen.

### Gruppe 107

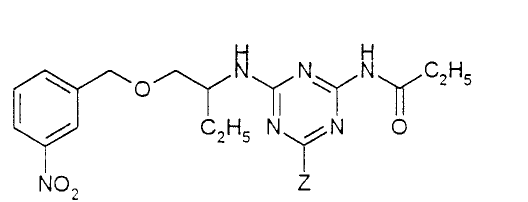

Z hat hierbei beispielhaft die oben in Gruppe 1 angegebenen Bedeutungen.

### Gruppe 108

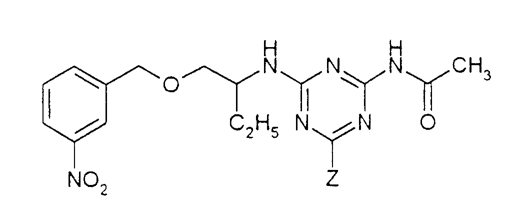

Z hat hierbei beispielhaft die oben in Gruppe 1 angegebenen Bedeutungen.

### Gruppe 109

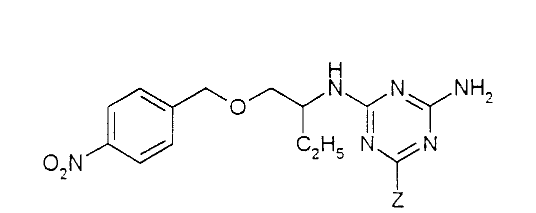

Z hat hierbei beispielhaft die oben in Gruppe 1 angegebenen Bedeutungen.

### Gruppe 110

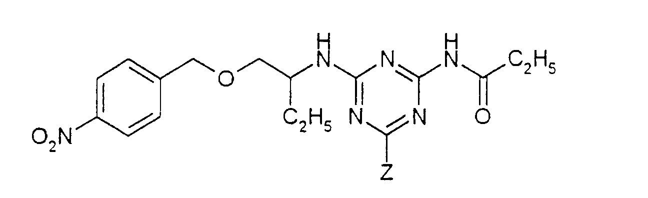

Z hat hierbei beispielhaft die oben in Gruppe 1 angegebenen Bedeutungen.

### Gruppe 111

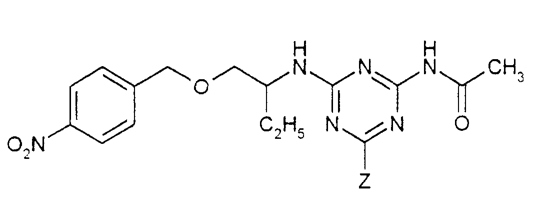

Z hat hierbei beispielhaft die oben in Gruppe 1 angegebenen Bedeutungen.

### Gruppe 112

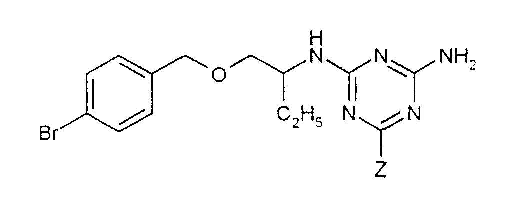

Z hat hierbei beispielhaft die oben in Gruppe 1 angegebenen Bedeutungen.

### Gruppe 113

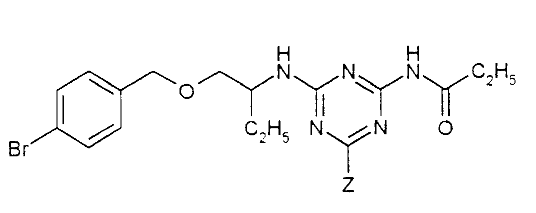

Z hat hierbei beispielhaft die oben in Gruppe 1 angegebenen Bedeutungen.

### Gruppe 114

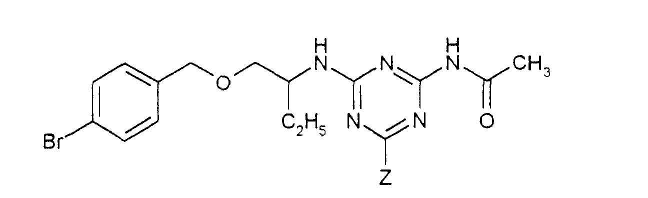

Z hat hierbei beispielhaft die oben in Gruppe 1 angegebenen Bedeutungen

### Gruppe 115

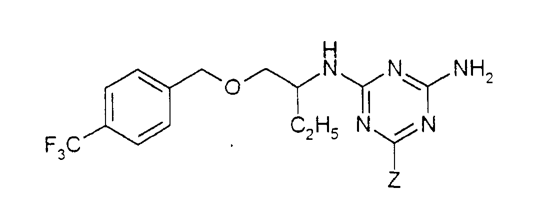

Z hat hierbei beispielhaft die oben in Gruppe 1 angegebenen Bedeutungen.

### Gruppe 116

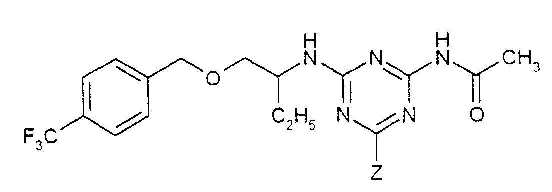

Z hat hierbei beispielhaft die oben in Gruppe 1 angegebenen Bedeutungen.

### Gruppe 117

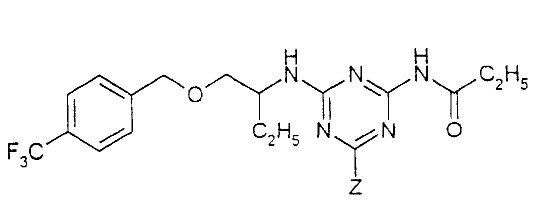

Z hat hierbei beispielhaft die oben in Gruppe angegebenen Bedeutungen.

### Gruppe 118

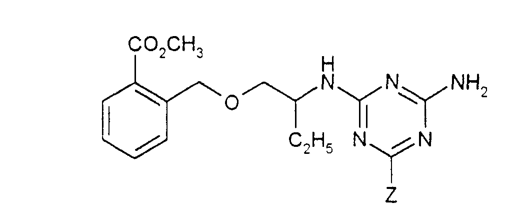

Z hat hierbei beispielhaft die oben in Gruppe 1 angegebenen Bedeutungen.

### Gruppe 119

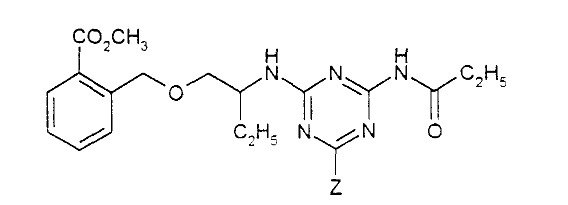

Z hat hierbei beispielhaft die oben in Gruppe 1 angegebenen Bedeutungen

### Gruppe 120

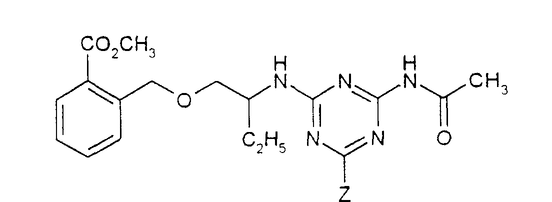

Z hat hierbei beispielhaft die oben in Gruppe 1 angegebenen Bedeutungen

### Gruppe 121

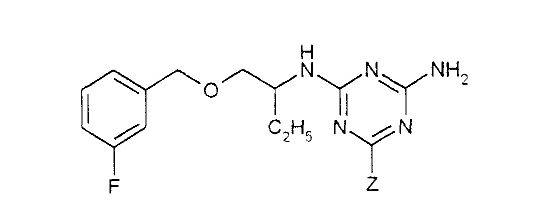

Z hat hierbei beispielhaft die oben in Gruppe 1 angegebenen Bedeutungen.

### Gruppe 122

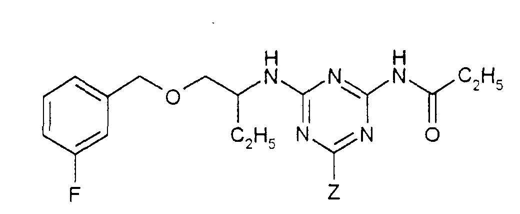

Z hat hierbei beispielhaft die oben in Gruppe 1 angegebenen Bedeutungen.

### Gruppe 123

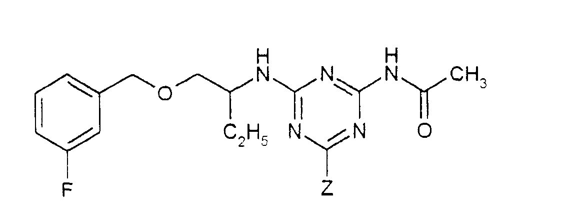

Z hat hierbei beispielhaft die oben in Gruppe 1 angegebenen Bedeutungen.

### Gruppe 124

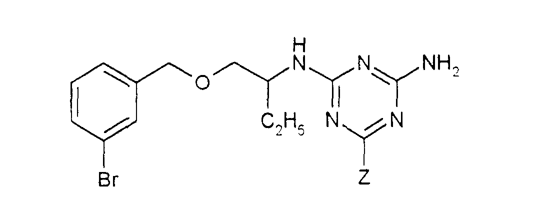

Z hat hierbei beispielhaft die oben in Gruppe 1 angegebenen Bedeutungen.

### Gruppe 125

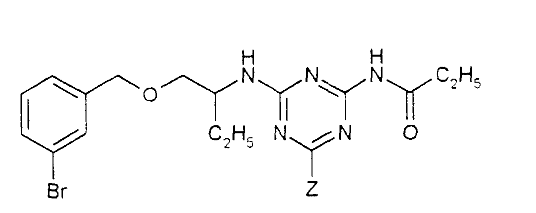

Z hat hierbei beispielhaft die oben in Gruppe 1 angegebenen Bedeutungen.

### Gruppe 12

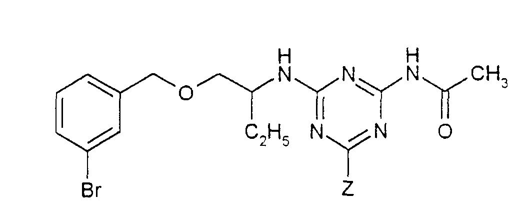

Z hat hierbei beispielhaft die oben in Gruppe 1 angegebenen Bedeutungen.

### Gruppe 127

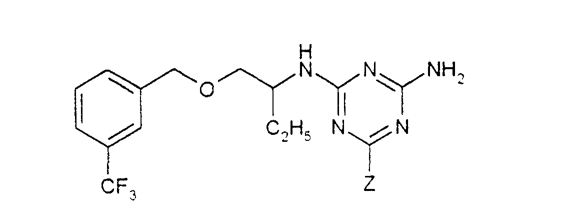

Z hat hierbei beispielhaft die oben in Gruppe 1 angegebenen Bedeutungen.

### Gruppe 128

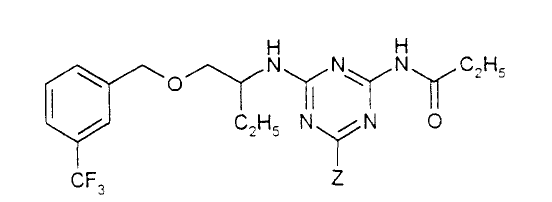

Z hat hierbei beispielhaft die oben in Gruppe 1 angegebenen Bedeutungen.

### Gruppe 129

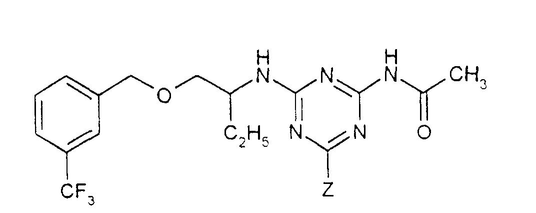

Z hat hierbei beispielhaft die oben in Gruppe 1 angegebenen Bedeutungen.

### Gruppe 130

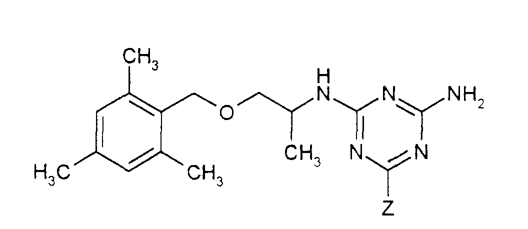

Z hat hierbei beispielhaft die oben in Gruppe 1 angegebenen Bedeutungen.

### Gruppe 131

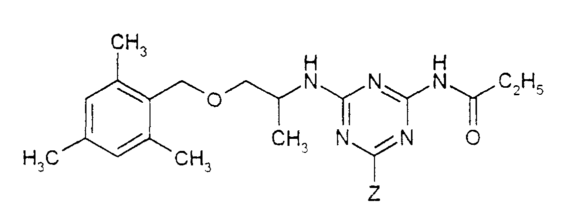

Z hat hierbei beispielhaft die oben in Gruppe 1 angegebenen Bedeutungen.

### Gruppe 132

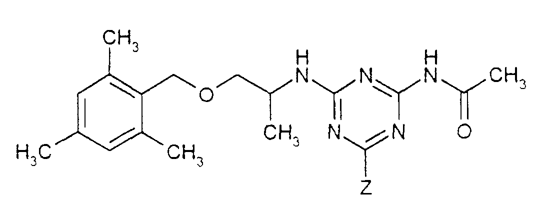

Z hat hierbei beispielhaft die oben in Gruppe 1 angegebenen Bedeutungen.

### Gruppe 133

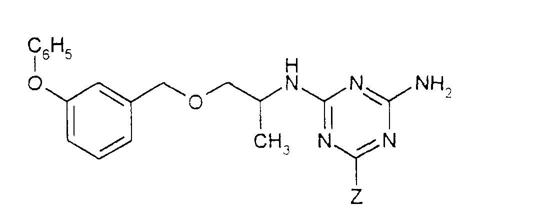

Z hat hierbei beispielhaft die oben in Gruppe 1 angegebenen Bedeutungen.

### Gruppe 134

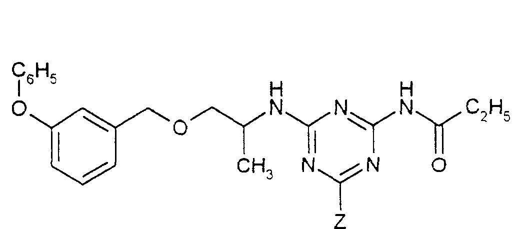

Z hat hierbei beispielhaft die oben in Gruppe 1 angegebenen Bedeutungen.

### Gruppe 135

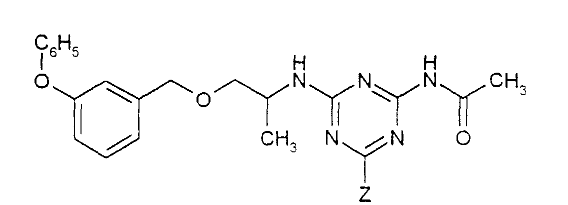

Z hat hierbei beispielhaft die oben in Gruppe 1 angegebenen Bedeutungen.

### Gruppe 136

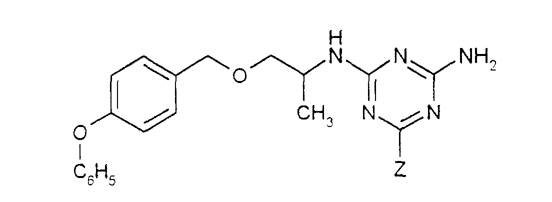

Z hat hierbei beispielhaft die oben in Gruppe 1 angegebenen Bedeutungen.

### Gruppe 137

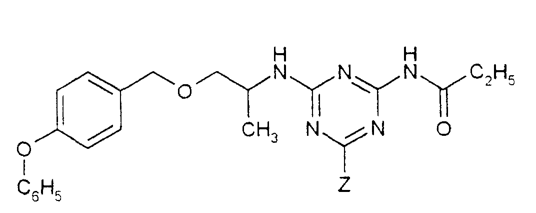

Z hat hierbei beispielhaft die oben in Gruppe 1 angegebenen Bedeutungen.

### Gruppe 138

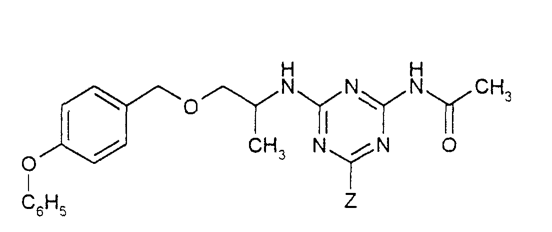

Z hat hierbei beispielhaft die oben in Gruppe 1 angegebenen Bedeutungen.

### Gruppe 139

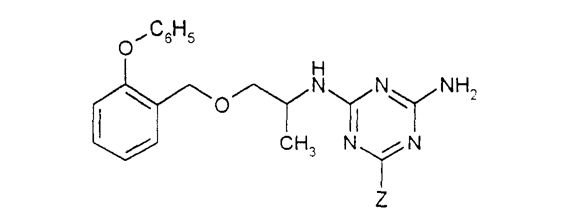

Z hat hierbei beispielhaft die oben in Gruppe 1 angegebenen Bedeutungen.

### Gruppe 140

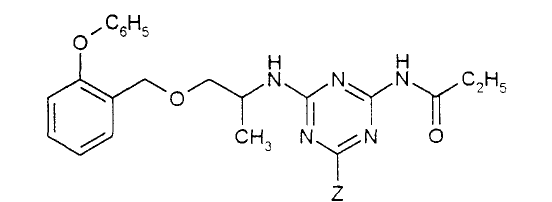

Z hat hierbei beispielhaft die oben in Gruppe 1 angegebenen Bedeutungen.

### Gruppe 141

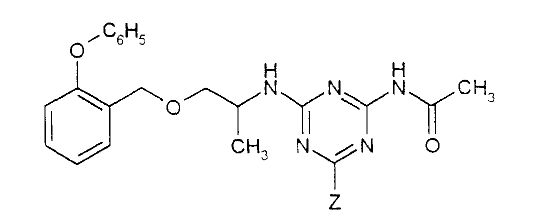

Z hat hierbei beispielhaft die oben in Gruppe 1 angegebenen Bedeutungen.

### Gruppe 142

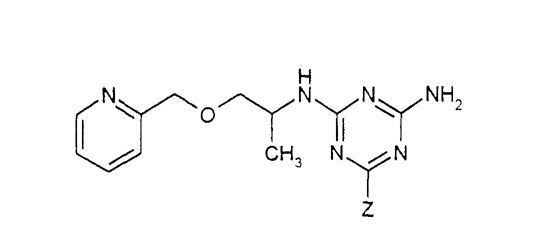

Z hat hierbei beispielhaft die oben in Gruppe 1 angegebenen Bedeutungen.

### Gruppe 143

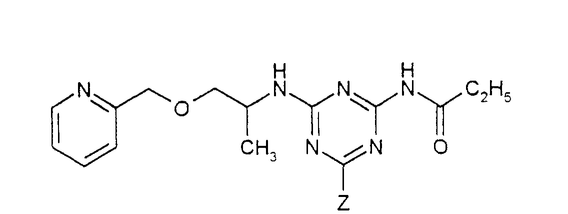

Z hat hierbei beispielhaft die oben in Gruppe 1 angegebenen Bedeutungen.

### Gruppe 144

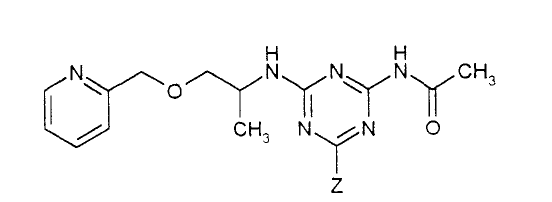

Z hat hierbei beispielhaft die oben in Gruppe 1 angegebenen Bedeutungen.

### Gruppe 145

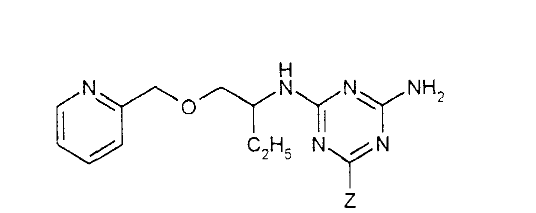

Z hat hierbei beispielhaft die oben in Gruppe 1 angegebenen Bedeutungen

### Gruppe 146

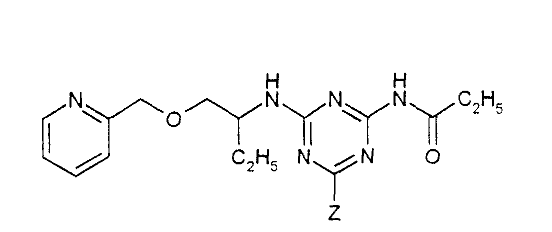

Z hat hierbei beispielhaft die oben in Gruppe 1 angegebenen Bedeutungen.

### Gruppe 147

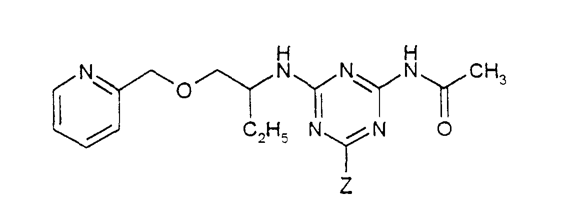

Z hat hierbei beispielhaft die oben in Gruppe 1 angegebenen Bedeutungen.

### Gruppe 148

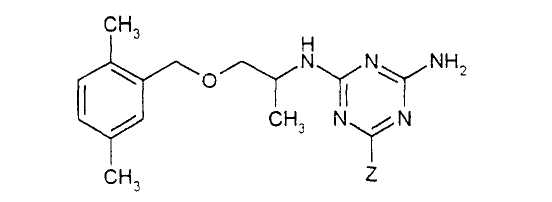

Z hat hierbei beispielhaft die oben in Gruppe 1 angegebenen Bedeutungen.

### Gruppe 149

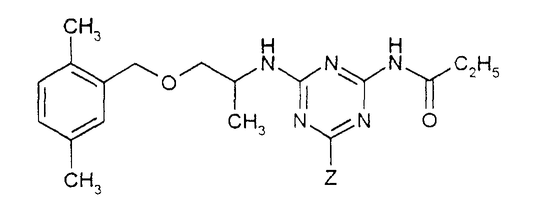

Z hat hierbei beispielhaft die oben in Gruppe 1 angegebenen Bedeutungen.

### Gruppe 150

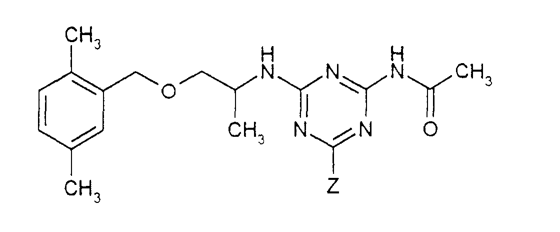

Z hat hierbei beispielhaft die oben in Gruppe 1 angegebenen Bedeutungen.

Verwendet man beispielsweise 1-(1-Benzyloxymethyl-propyl)-biguanid und Trifluoressigsäuremethylester als Ausgangsstoffe, so kann der Reaktionsablauf beim erfindungsgemäßen Verfahren (a) durch das folgende Formelschema skizziert werden:

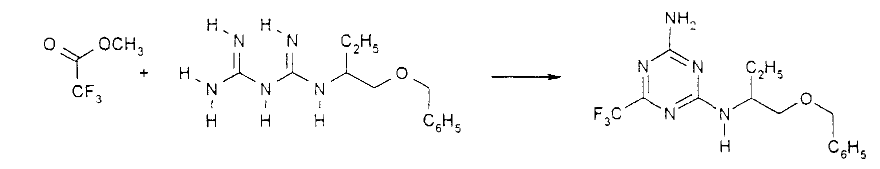

Verwendet man beispielsweise 2-Chlor-4-(1-benzylthiomethyl-propylamino)-6-trifluormethyl-1,3,5-triazin und Ethylamin als Ausgangsstoffe, so kann der Reaktionsablauf beim erfindungsgemäßen Verfahren (b) durch das folgende Formelschema skizziert werden

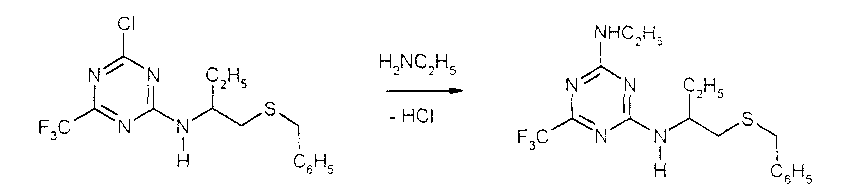

Verwendet man beispielsweise 2-Amino-4-methoxy-6-trifluormethyl-1,3,5-triazin und 1-Benzyloxymethyl-propylamin als Ausgangsstoffe, so kann der Reaktionsablauf beim erfindungsgemäßen Verfahren (c) durch das folgende Formelschema skizziert werden:

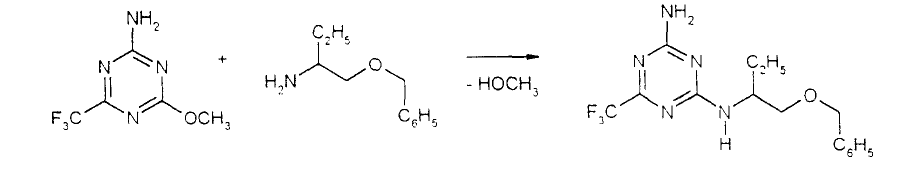

Verwendet man beispielsweise 2-Amino-4-(1-benzyloxymethyl-propylamino)-6-trifluormethyl-1,3,5-triazin und Acetylchlorid als Ausgangsstoffe, so kann der Reaktionsablauf beim erfindungsgemäßen Verfahren (d) durch das folgende Formelschema skizziert werden:

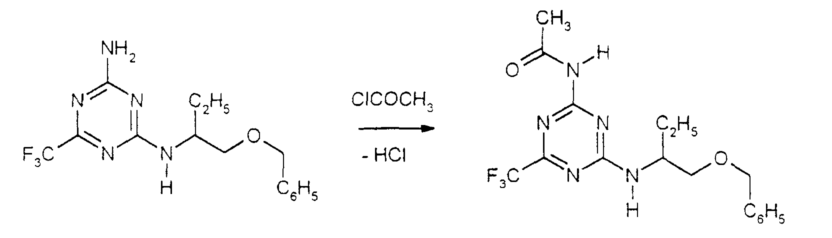

Die beim erfindungsgemäßen Verfahren (a) zur Herstellung von Verbindungen der Formel (I) als Ausgangsstoffe zu verwendenden Biguanide sind durch die Formel (II) allgemein definiert. In der Formel (II) haben Q, R¹, R², R³, R⁴, R⁵ und Ar vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für Q, R¹, R², R³, R⁴, R⁵ und Ar angegeben wurden.

Geeignete Säureaddukte von Verbindungen der Formel (II) sind deren Additionsprodukte mit Protonensäuren, wie z.B. mit Chlorwasserstoff (Hydrogenchlorid), Bromwasserstoff (Hydrogenbromid), Schwefelsäure, Methansulfonsaure, Benzolsulfonsäure und p-Toluolsulfonsäure.

Die Ausgangsstoffe der allgemeinen Formel (II) sind noch nicht aus der Literatur bekannt; sie sind als neue Stoffe auch Gegenstand der vorliegenden Anmeldung.

Man erhält die neuen Biguanide der allgemeinen Formel (II), wenn man substituierte Alkylamine der allgemeinen Formel (VII) 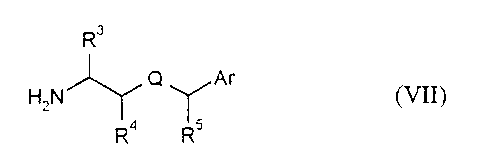 in welcher
Q, R³, R⁴, R⁵ und Ardie oben angegebene Bedeutung haben,
- und/oder Säureaddukte von Verbindungen der allgemeinen Formel (VII), wie z.B. die Hydrochloride -
mit Cyanoguanidin ("Dicyandiamid") der Formel (IX) 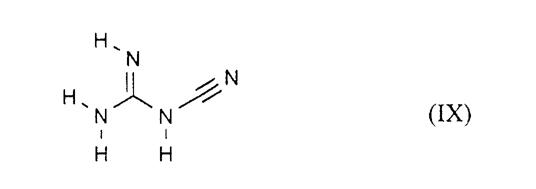 gegebenenfalls in Gegenwart eines Reaktionshilfsmittels, wie z.B. Hydrogenchlorid, und gegebenenfalls in Gegenwart eines Verdünnungsmittels, wie z.B. n-Decan oder 1.2-Dichlor-benzol, bei Temperaturen zwischen 100°C und 200°C umsetzt (vgl. EP-492 615, Herstellungsbeispiele).

Die hierfür als Vorprodukte benötigten substituierten Alkylamine der allgemeinen Formel (VII) sind bekannt und/oder können nach an sich bekannten Verfahren hergestellt werden (vgl. J. Org. Chem. 34 (1969), 466-468; J. Heterocycl. Chem. 11 (1974), 985-989; Liebigs Ann. Chem. 1980, 786-790; Can. J. Chem. 60 (1982), 1836-1841; Tetrahedron 40 (1984), 1255-1268; J. Am. Chem. Soc. 109 (1987), 236-239 und 1798-1805; loc. cit. 110 (1988), 3862-3869, Tetrahedron Lett. 30 (1989), 731-734; Tetrahedron: Asymmetry 3 (1992), 587-590; loc. cit. 7 (1996), 3397-3406; Tetrahedron Lett. 34 (1993), 2957-2960; Tetrahedron 51 (1995), 1709-1720; J Org. Chem. 61 (1996), 7285-7290; Tetrahedron 52 (1996), 4199-4214; EP-192 060; EP-199 845; WO 9213823; Herstellungsbeispiele).

Die beim erfindungsgemäßen Verfahren (a) zur Herstellung von Verbindungen der Formel (I) weiter als Ausgangsstoffe zu verwendenden Alkoxycarbonylverbindungen sind durch die Formel (III) allgemein definiert. In der Formel (III) hat Z vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für Z angegeben wurde; R' steht vorzugsweise für Alkyl mit 1 bis 4 Kohlenstoffatomen, insbesondere für Methyl oder Ethyl.

Die Ausgangsstoffe der Formel (III) sind bekannte Synthesechemikalien.

Die beim erfindungsgemäßen Verfahren (b) zur Herstellung von Verbindungen der Formel (I) als Ausgangsstoffe zu verwendenden substituierten Halogentriazine sind durch die Formel (IV) allgemein definiert. In der Formel (IV) haben Q, R³, R⁴, R⁵, Ar und Z vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für Q, R³, R⁴, R⁵, Ar und Z angegeben wurden; X steht vorzugsweise für Fluor oder Chlor, insbesondere für Chlor.

Die Ausgangsstoffe der allgemeinen Formel (IV) sind noch nicht aus der Literatur bekannt; sie sind als neue Stoffe ebenfalls Gegenstand der vorliegenden Anmeldung.

Man erhält die neuen substituierten Halogentriazine der allgemeinen Formel (IV), wenn man entsprechende Dihalogentriazine der allgemeinen Formel (X) 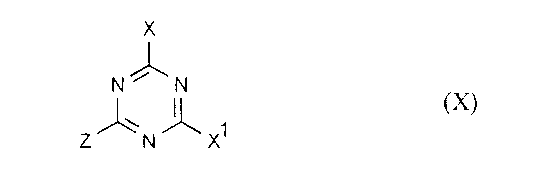 in welcher
- X und Z: die oben angegebene Bedeutung haben und
- X¹: für Halogen steht,
mit substituierten Alkylaminen der allgemeinen Formel (VII) 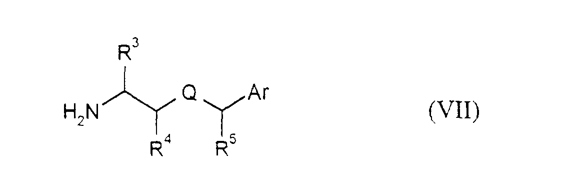 in welcher
- Q, R³, R⁴, R⁵ und Ar: die oben angegebene Bedeutung haben,
gegebenenfalls in Gegenwart eines Säureakzeptors, wie z.B. Ethyldiisopropylamin, und gegebenenfalls in Gegenwart eines Verdünnungsmittels, wie z.B. Tetrahydrofuran oder Dioxan, bei Temperaturen zwischen -50°C und +50°C umsetzt (vgl. die Herstellungsbeispiele).
Die beim erfindungsgemäßen Verfahren (b) zur Herstellung von Verbindungen der Formel (I) weiter als Ausgangsstoffe zu verwendenden Stickstoffverbindungen sind durch die Formel (V) allgemein definiert. In der Formel (V) haben R¹ und R² vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für R¹ und R² angegeben wurden.

Die Ausgangsstoffe der allgemeinen Formel (V) sind bekannte Synthesechemikalien. Die beim erfindungsgemäßen Verfahren (c) zur Herstellung von Verbindungen der Formel (I) als Ausgangsstoffe zu verwendenden substituierten Aminotriazine sind durch die Formel (VI) allgemein definiert. In der Formel (VI) haben R¹, R² und Z vorzugsweise bzw. insbesondere diejenigen Bedeutungen, die bereits oben im Zusammenhang mit der Beschreibung der erfindungsgemäßen Verbindungen der Formel (I) vorzugsweise bzw als insbesondere bevorzugt für R¹, R² und Z angegeben wurden; Y¹ steht vorzugsweise für Fluor, Chlor, Methoxy oder Ethoxy, insbesondere für Chlor oder Methoxy.

Die Ausgangsstoffe der allgemeinen Formel (VI) sind bekannt und/oder können nach an sich bekannten Verfahren hergestellt werden (vgl. WO 95/11237).

Die beim erfindungsgemäßen Verfahren (c) weiter als Ausgangsstoffe zu verwendenden substituierten Alkylamine sind durch die Formel (VII) allgemein definiert. In der Formel (VII) haben Q, R³, R⁴, R⁵ und Ar vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für Q, R³, R⁴, R⁵ und Ar angegeben wurden.

Die Ausgangsstoffe der allgemeinen Formel (VII) sind bekannt und/oder können nach an sich bekannten Verfahren hergestellt werden (vgl. J. Org. Chem. 34 (1969), 466-468; J. Heterocycl. Chem. 11 (1974), 985-989; Liebigs Ann. Chem. 1980, 786-790; Can. J. Chem. 60 (1982), 1836-1841; Tetrahedron 40 (1984), 1255-1268; J. Am. Chem. Soc. 109 (1987), 236-239; loc. cit. 110 (1988), 3862-3869; Tetrahedron Lett. 30 (1989), 731-734; Tetrahedron: Asymmetry 3 (1992), 587-590; Tetrahedron Lett. 34 (1993), 2957-2960; EP-192 060; EP-199 845; WO 9213823; Herstellungsbeispiele).

Die beim erfindungsgemäßen Verfahren (d) zur Herstellung von Verbindungen der Formel (I) als Ausgangsstoffe zu verwendenden substituierten 2,4-Diamino-1,3,5-triazine sind durch die Formel (Ia) allgemein definiert. In der Formel (Ia) haben Q, R¹, R³, R⁴, R⁵, Ar und Z vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für Q, R¹, R³, R⁴, R⁵, Ar und Z angegeben wurden.

Die Ausgangsstoffe der allgemeinen Formel (Ia) sind als neue Stoffe auch Gegenstand der vorliegenden Anmeldung; sie können nach den erfindungsgemäßen Verfahren (a), (b) oder (c) hergestellt werden (vgl. die Herstellungsbeispiele).

Die beim erfindungsgemäßen Verfahren (d) weiter als Ausgangsstoffe zu verwendenden Alkylierungs- oder Acylierungs-mittel sind durch die Formel (VIII) allgemein definiert. In der Formel (VIII) hat R² mit Ausnahme von Wasserstoff vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für R² angegeben wurde; Y² steht vorzugsweise für Fluor, Chlor, Brom, Methoxy, Ethoxy, Acetyloxy oder Propionyloxy, insbesondere für Chlor, Methoxy oder Acetyloxy.

Die Ausgangsstoffe der allgemeinen Formel (VIII) sind bekannte Synthesechemikalien.

Die erfindungsgemäßen Verfahren zur Herstellung der Verbindungen der Formel (I) werden gegebenenfalls unter Verwendung eines Reaktionshilfsmittels durchgeführt. Als Reaktionshilfsmittel für die Verfahren (a), (b), (c) und (d) kommen im allgemeinen die üblichen anorganischen oder organischen Basen oder Säureakzeptoren in Betracht. Hierzu gehören vorzugsweise Alkalimetall- oder Erdalkalimetall- -acetate, -amide, -carbonate, -hydrogencarbonate, -hydride, -hydroxide oder -alkanolate, wie beispielsweise Natrium-, Kalium- oder Calcium-acetat, Lithium-, Natrium-, Kaliumoder Calcium-amid, Natrium-, Kalium- oder Calcium-carbonat, Natrium-, Kaliumoder Calcium-hydrogencarbonat, Lithium-, Natrium-, Kalium- oder Calcium-hydrid, Lithium-, Natrium-, Kalium- oder Calcium-hydroxid, Natrium- oder Kalium-methanolat, -ethanolat, -n- oder -i-propanolat, -n-, -i-, -s- oder -t-butanoiat; weiterhin auch basische organische Stickstoffverbindungen, wie beispielsweise Trimethylamin, Triethylamin, Tripropylamin, Tributylamin, Ethyl-diisopropylamin, N,N-Dimethyl-cyclohexylamin, Dicyclohexylamin, Ethyl-dicyclohexylamin, N,N-Dimethylanilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl- und 3,5-Dimethyl-pyridin, 5-Ethyl-2-methylpyridin, 4-Dimethylamino-pyridin, N-Methyl-piperidin, 1,4-Diazabicyclo [2,2,2]-octan (DABCO), 1,5-Diazabicyclo[4,3,0]-non-5-en (DBN), oder 1,8-Diazabicyclo[5,4,0]-undec-7-en (DBU).

Als Reaktionshilfsmittel können gegebenenfalls auch Molekularsiebe eingesetzt werden.

Als Verdünnungsmittel zur Durchführung der erfindungsgemäßen Verfahren (a), (b), (c) und (d) kommen neben Wasser vor allem inerte organische Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Ketone, wie Methyl-isopropyl-keton oder Methyl-isobutyl-keton; Nitrile, wie Acetonitril, Propionitril oder Butyronitril; Amide, wie N,N-Dimethylformamid, N,N-Dimethylacetamid, N-Methyl-formanilid, N-Methyl-pyrrolidon oder Hexamethylphosphorsäuretriamid; Ester wie Essigsäuremethylester oder Essigsäureethylester, Sulfoxide, wie Dimethylsulfoxid; Alkohole, wie Methanol, Ethanol, n- oder i-Propanol, Ethylenglykolmonomethylether, Ethylenglykolmonoethylether, Diethylenglykolmonomethylether, Diethylenglykolmonoethylether, deren Gemische mit Wasser oder reines Wasser.

Die Reaktionstemperaturen können bei der Durchführung der erfindungsgemäßen Verfahren (a), (b), (c) und (d) in einem größeren Bereich variiert werden. Im allgemeinen arbeitet man bei Temperaturen zwischen -20°C und +300°C, vorzugsweise zwischen -10°C und +250°C.

Die erfindungsgemäßen Verfahren (a), (b), (c) und (d) werden im allgemeinen unter Normaldruck durchgeführt. Es ist jedoch auch möglich, die erfindungsgemäßen Verfahren unter erhöhtem oder vermindertem Druck - im allgemeinen zwischen 0,1 bar und 10 bar - durchzuführen.

Zur Durchführung der erfindungsgemäßen Verfahren werden die Ausgangsstoffe im allgemeinen in angenähert äquimolaren Mengen eingesetzt. Es ist jedoch auch möglich, jeweils eine der Komponenten in einem größeren Überschuß zu verwenden. Die Umsetzung wird im allgemeinen in einem geeigneten Verdünnungsmittel in Gegenwart eines Reaktionshilfsmittels durchgeführt und das Reaktionsgemisch wird im allgemeinen mehrere Stunden bei der erforderlichen Temperatur gerührt. Die Aufarbeitung wird nach üblichen Methoden durchgeführt (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.
Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.
Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen und dikotylen Unkräutern in monokotylen Kulturen sowohl im Vorauflauf- als auch im Nachauflauf-Verfahren.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsaure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es konnen Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Acetochlor, Acifluorfen(-sodium), Aclonifen, Alachlor, Alloxydim(-sodium), Ametryne, Amidochlor, Amidosulfuron, Anilofos, Asulam, Atrazine, Azafenidin, Azimsulfuron, Benazolin(-ethyl), Benfuresate, Bensulfuron(-methyl), Bentazon, Benzofenap, Benzoylprop(-ethyl), Bialaphos, Bifenox, Bispyribac(-sodium), Bromobutide, Bromofenoxim, Bromoxynil, Butachlor, Butroxydim, Butylate, Cafenstrole, Caloxydim, Carbetamide, Carfentrazone(-ethyl), Chlomethoxyfen, Chloramben, Chloridazon, Chlorimuron(-ethyl), Chlornitrofen, Chlorsulfuron, Chlortoluron, Cinmethylin, Cinosulfuron, Clethodim, Clodinafop(-propargyl), Clomazone, Clomeprop, Clopyralid, Clopyrasulfuron(-methyl), Cloransulam(-methyl), Cumyluron, Cyanazine, Cycloate, Cyclosulfamuron, Cycloxydim, Cyhalofop(-butyl), 2,4-D, 2,4-DB, 2,4-DP, Desmedipham, Diallate, Dicamba, Diclofop(-methyl), Diclosulam, Diethatyl(-ethyl), Difenzoquat, Diflufenican, Diflufenzopyr, Dimefuron, Dimepiperate, Dimethachlor, Dimethametryn, Dimethenamid, Dimexyflam, Dinitramine, Diphenamid, Diquat, Dithiopyr, Diuron, Dymron, Epoprodan, EPTC, Esprocarb, Ethalfluralin, Ethametsulfuron(-methyl), Ethofumesate, Ethoxyfen, Ethoxysulfuron, Etobenzanid, Fenoxaprop(-P-ethyl), Flamprop(-isopropyl), Flamprop(-isopropyl-L), Flamprop(-methyl), Flazasulfuron, Fluazifop(-P-butyl), Flumetsulam, Flumiclorac(-pentyl), Flumioxazin, Flumipropyn, Flumetsulam, Fluometuron, Fluorochloridone, Fluoroglycofen(-ethyl), Flupoxam, Flupropacil, Flurpyrsulfuron(-methyl, -sodium), Flurenol(-butyl), Fluridone, Fluroxypyr(-meptyl), Flurprimidol, Flurtamone, Fluthiacet(-methyl), Fluthiamide, Fomesafen, Glufosinate(-ammonium), Glyphosate(-isopropylammonium), Halosafen, Haloxyfop(-ethoxyethyl), Haloxyfop(-P-methyl), Hexazinone, Imazamethabenz(-methyl), Imazamethapyr, Imazamox, Imazapyr, Imazaquin, Imazethapyr, Imazosulfuron, Ioxynil, Isopropalin, Isoproturon, Isouron, Isoxaben, Isoxaflutole, Isoxapyrifop, Lactofen, Lenacil, Linuron, MCPA, MCPP, Mefenacet, Metamitron, Metazachlor, Methabenzthiazuron, Metobenzuron, Metobromuron, (alpha-)Metolachlor, Metosulam, Metoxuron, Metribuzin, Metsulfuron(-methyl), Molinate, Monolinuron, Naproanilide, Napropamide, Neburon, Nicosulfuron, Norflurazon, Orbencarb, Oryzalin, Oxadiargyl, Oxadiazon, Oxasulfuron, Oxaziclomefone, Oxyfluorfen, Paraquat, Pelargonsäure, Pendimethalin, Pentoxazone, Phenmedipham, Piperophos, Pretilachlor, Primisulfuron(-methyl), Prometryn, Propachlor, Propanil, Propaquizafop, Propisochlor, Propyzamide, Prosulfocarb, Prosulfuron, Pyraflufen(-ethyl), Pyrazolate, Pyrazosulfuron(-ethyl), Pyrazoxyfen, Pyribenzoxim, Pyributicarb, Pyridate, Pyriminobac(-methyl), Pyrithiobac(-sodium), Quinchlorac, Quinmerac, Quinoclamine, Quizalofop(-P-ethyl), Quizalofop(-P-tefuryl), Rimsulfuron, Sethoxydim, Simazine, Simetryn, Sulcotrione, Sulfentrazone, Sulfometuron(-methyl), Sulfosate, Sulfosulfuron, Tebutam, Tebuthiuron, Terbuthylazine, Terbutryn, Thenylchlor, Thiafluamide, Thiazopyr, Thidiazimin, Thifensulfuron(-methyl), Thiobencarb, Tiocarbazil, Tralkoxydim, Triallate, Triasulfuron, Tribenuron(-methyl), Triclopyr, Tridiphane, Trifluralin und Triflusulfuron.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstruktur-verbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

### Herstellungsbeispiele:

### Beispiel 1

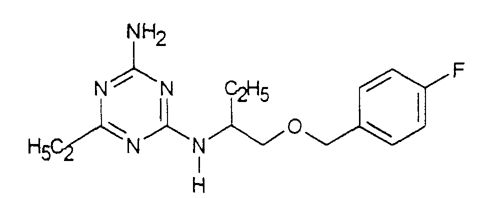

### (Verfahren (a))

Eine Lösung von 1,08 g (20 mmol) Natriummethylat in 5 ml Methanol wird tropfenweise unter Rühren bei -10°C zu einer Mischung aus 3,18 g (10 mmol) 1-[1-(4-Fluorbenzyloxymethyl)-propyl]-biguanid-Hydrochlorid (racemisch) und 2,8 g gepulvertem Molekularsieb (4 Å) in 10 ml Methanol gegeben. Danach werden tropfenweise 1,14 ml (10 mmol) Propionsäureethylester hinzugegeben. Man läßt die Reaktionsmischung auf Raumtemperatur (ca. 20°C) kommen und rührt noch 15 Stunden weiter. Dann wird von der ausgefallenen Mischung aus Feststoff und Molekularsieb abgesaugt, das Filtrat eingeengt und der Rückstand in Essigsäureethylester aufgenommen. Nach Waschen mit Wasser, Trocknen über Natriumsulfat und Einengen im Wasserstrahlvakuum wird der Rückstand säulenchromatografisch aufgearbeitet (Kieselgel, Essigsaureethylester/Petrolether).

Man erhält 1,4 g (44% der Theorie) 2-Amino-4-ethyl-6-[1-(4-fluor-benzyloxymethyl)-propylamino]-1,3,5-triazin (Racemat) vom Schmelzpunkt 96°C.

### Beispiel 2

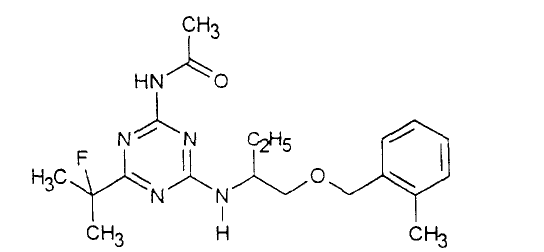

### (Verfahren (d))

2-Amino-4-(1-fluor-1-methyl-ethyl)-6-[1-(2-methyl-benzyloxymethyl-propylamino]-1,3,5-triazin (racemisch, 260 mg, 0,75 mmol) wird in Acetanhydrid (5 ml) für 1 Stunde auf 100°C erhitzt. Nach dem Abkühlen auf Raumtemperatur wird die Mischung mit Wasser (10 ml) versetzt, eine Stunde gerührt und das ausfallende Produkt durch Absaugen isoliert.

Man erhält 140 mg (50 % der Theorie) 2-Acetylamino-4-(1-fluor-1-methyl-ethyl-6-[1-(2-methyl-benzyloxymethyl)-propylamino]-1,3,5-triazin (Racemat) vom Schmelzpunkt 108°C.

Analog zu den Beispielen 1 und 2 sowie entsprechend der allgemeinen Beschreibung der erfindungsgemäßen Herstellungsverfahren können beispielsweise auch die in der nachstehenden Tabelle 1 aufgeführten Verbindungen der Formel (I) hergestellt werden.

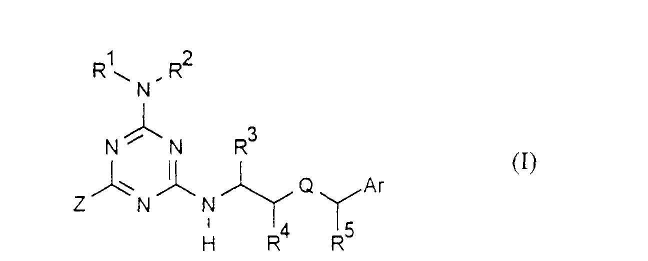

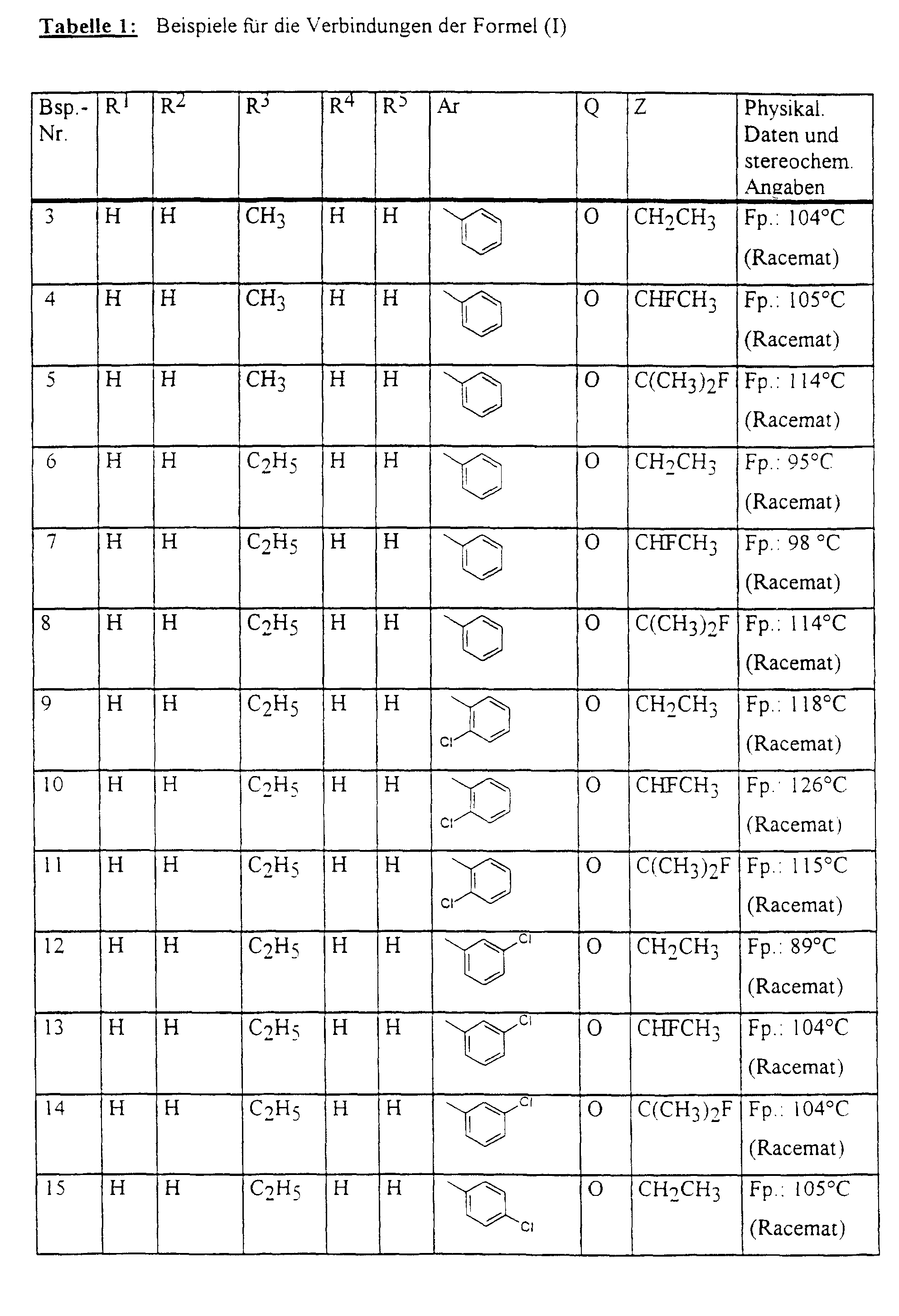

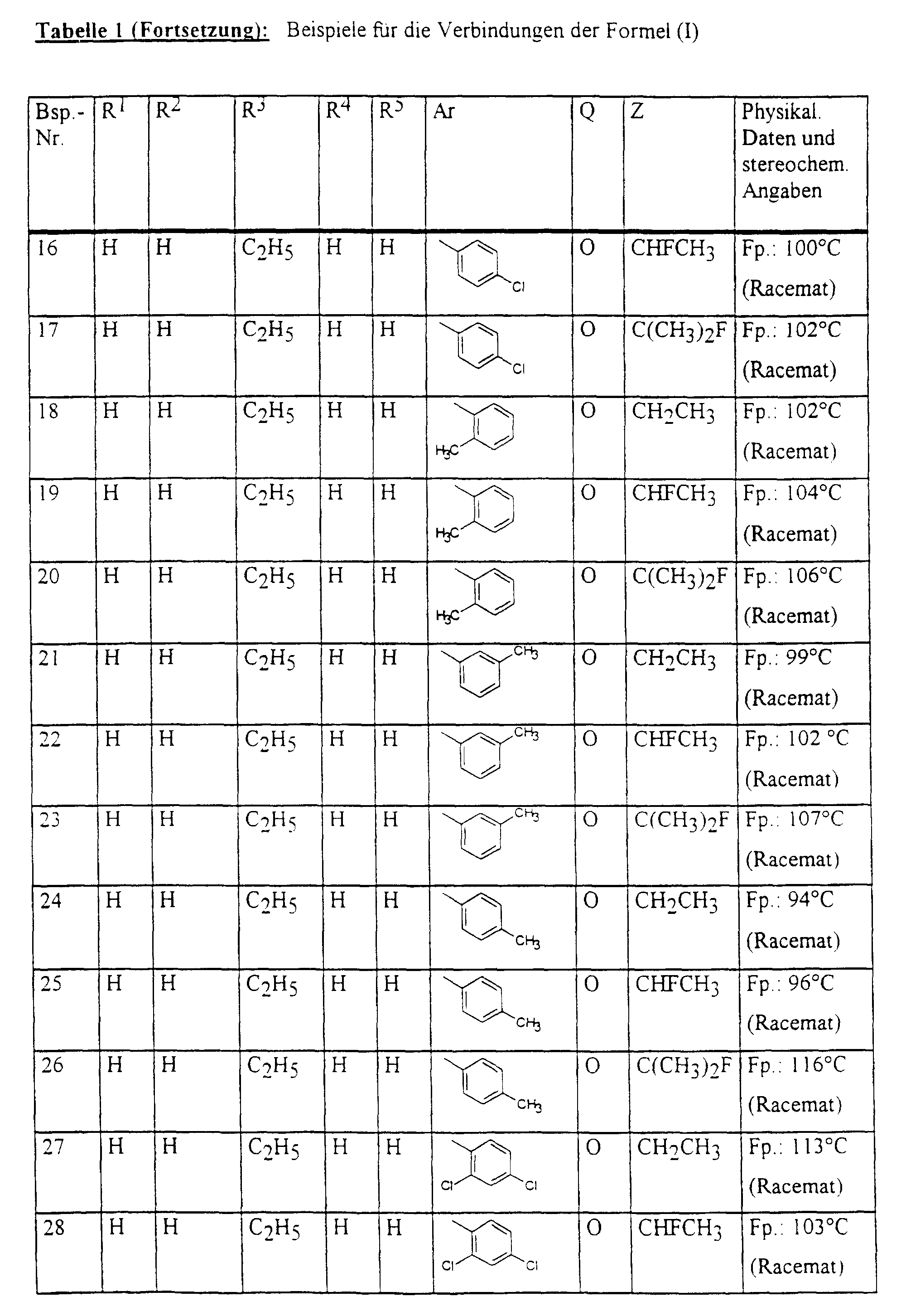

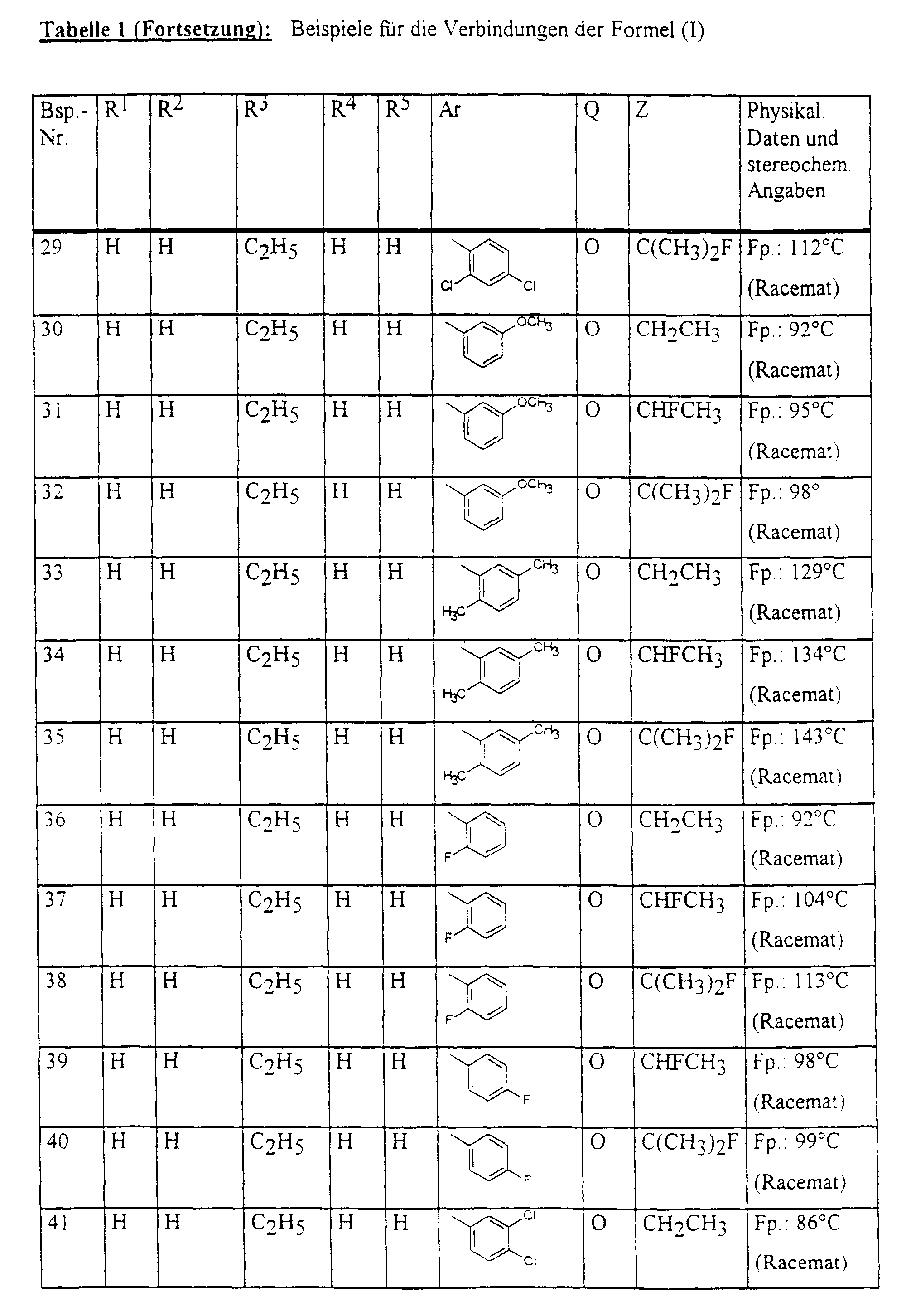

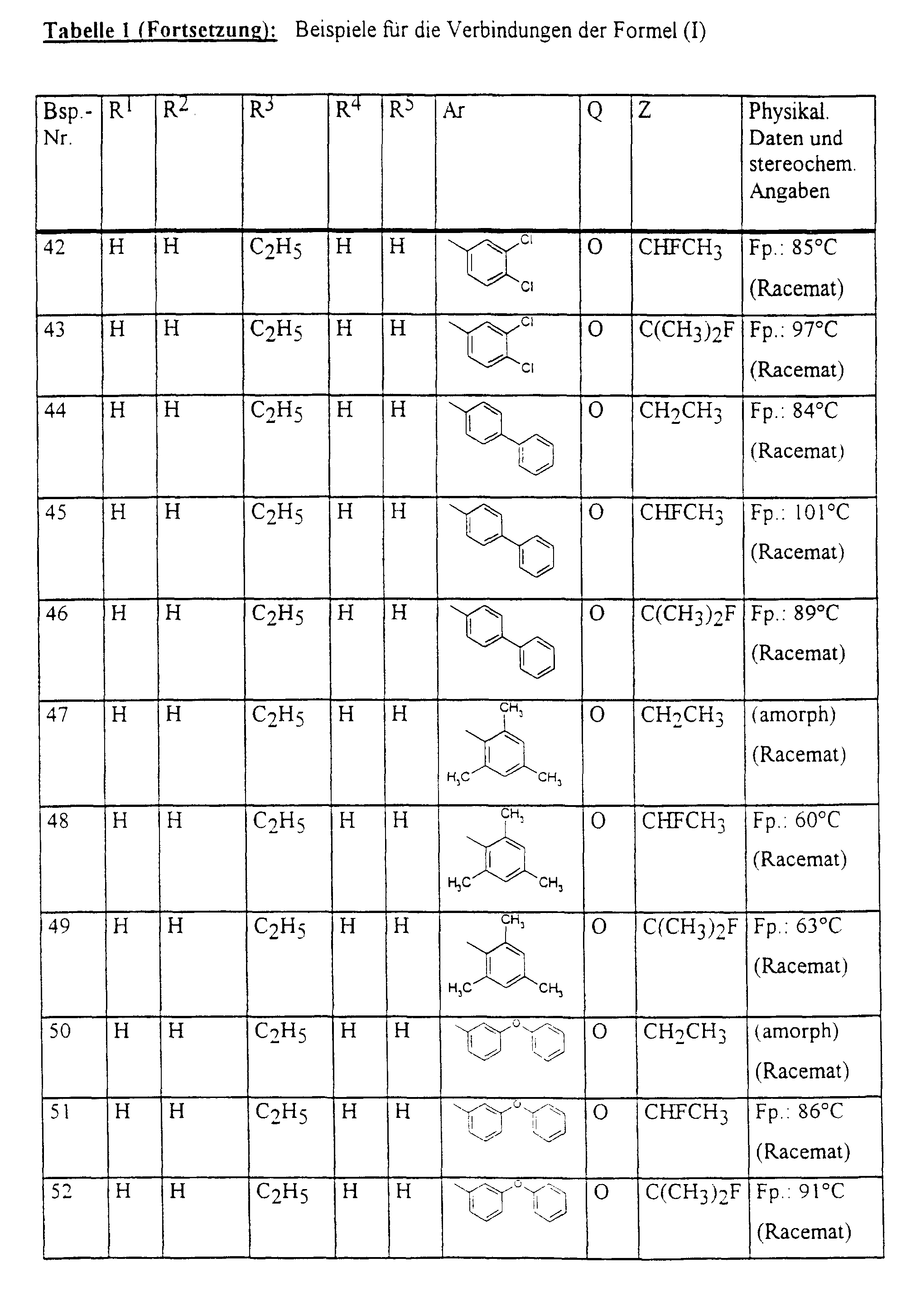

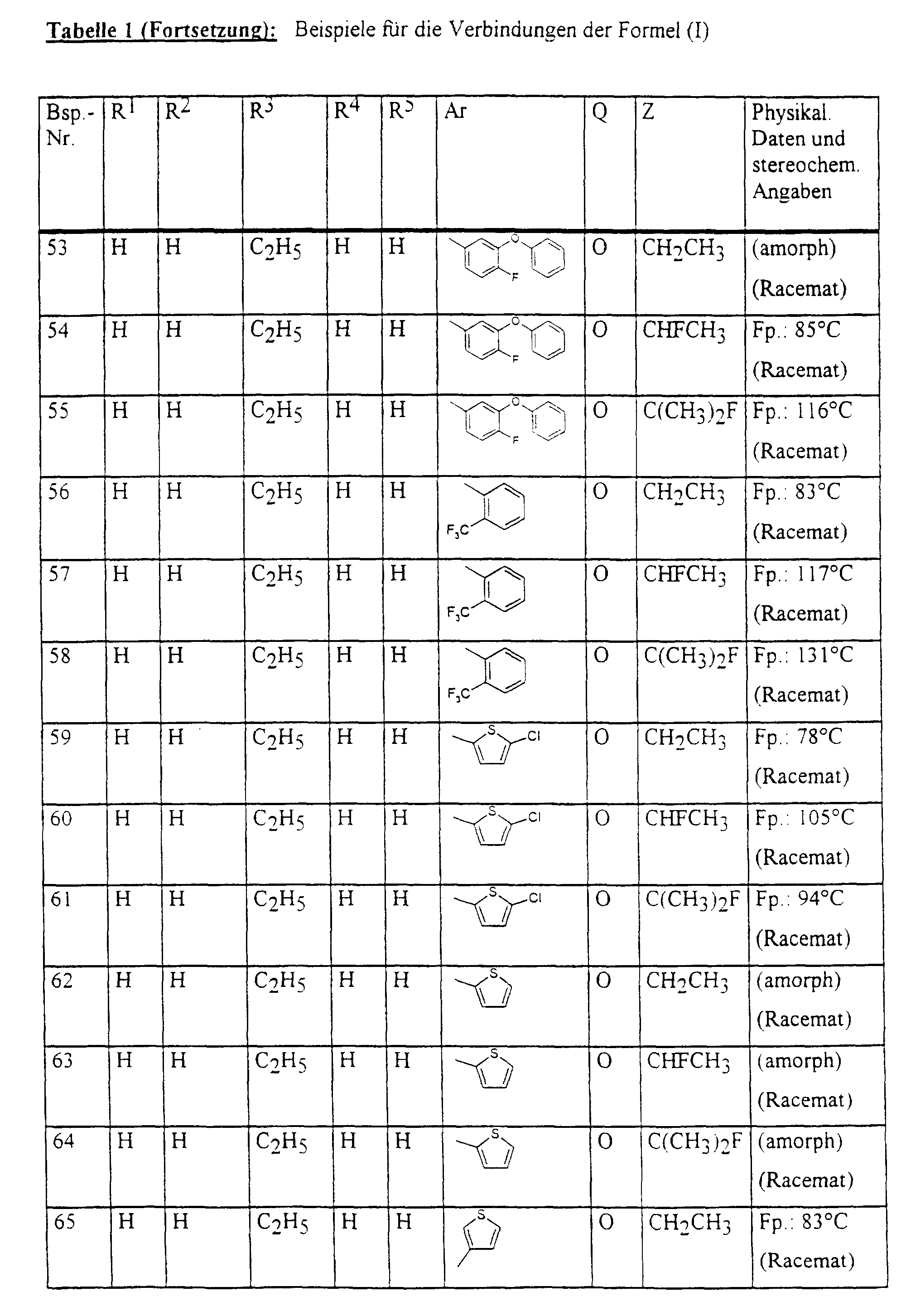

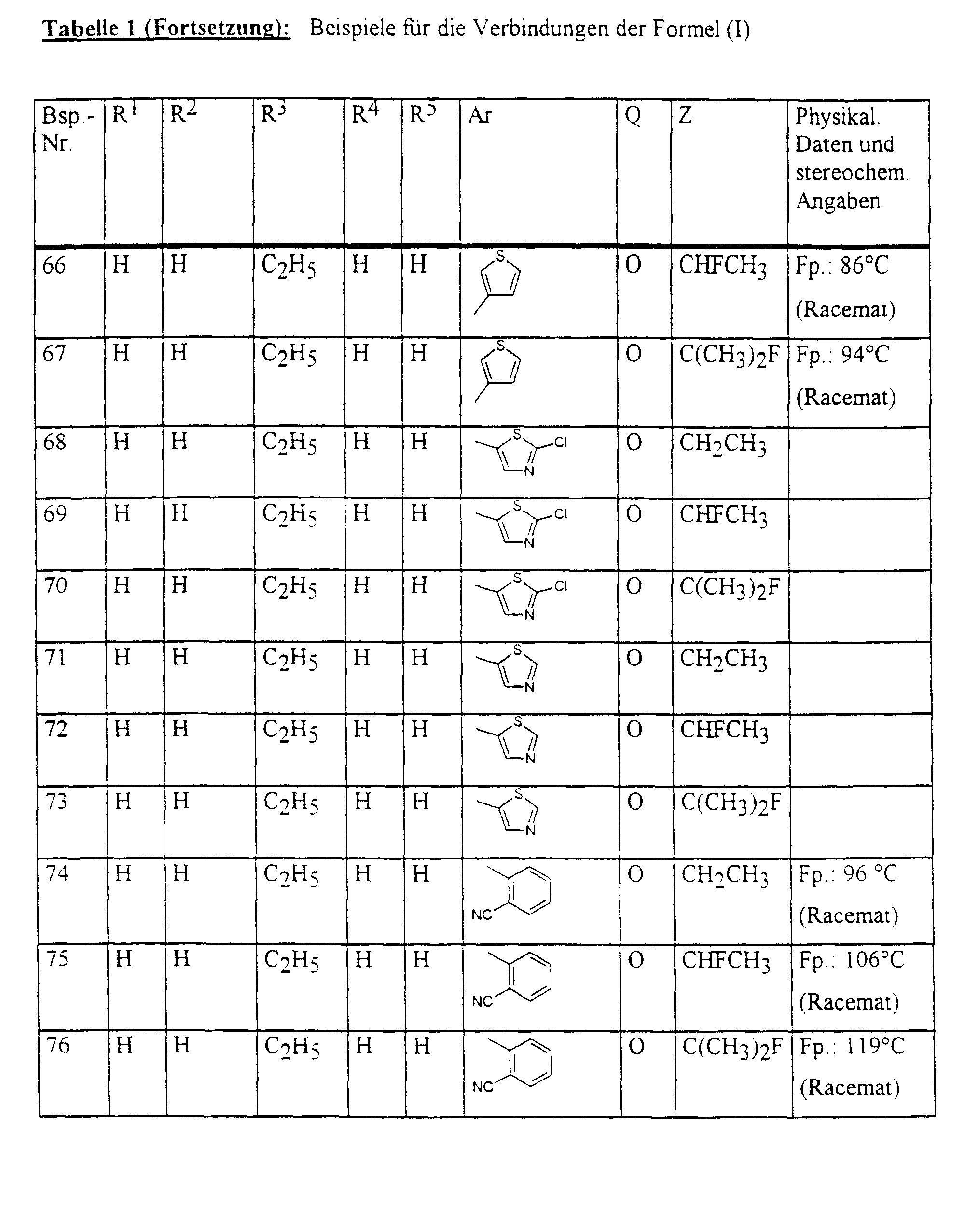

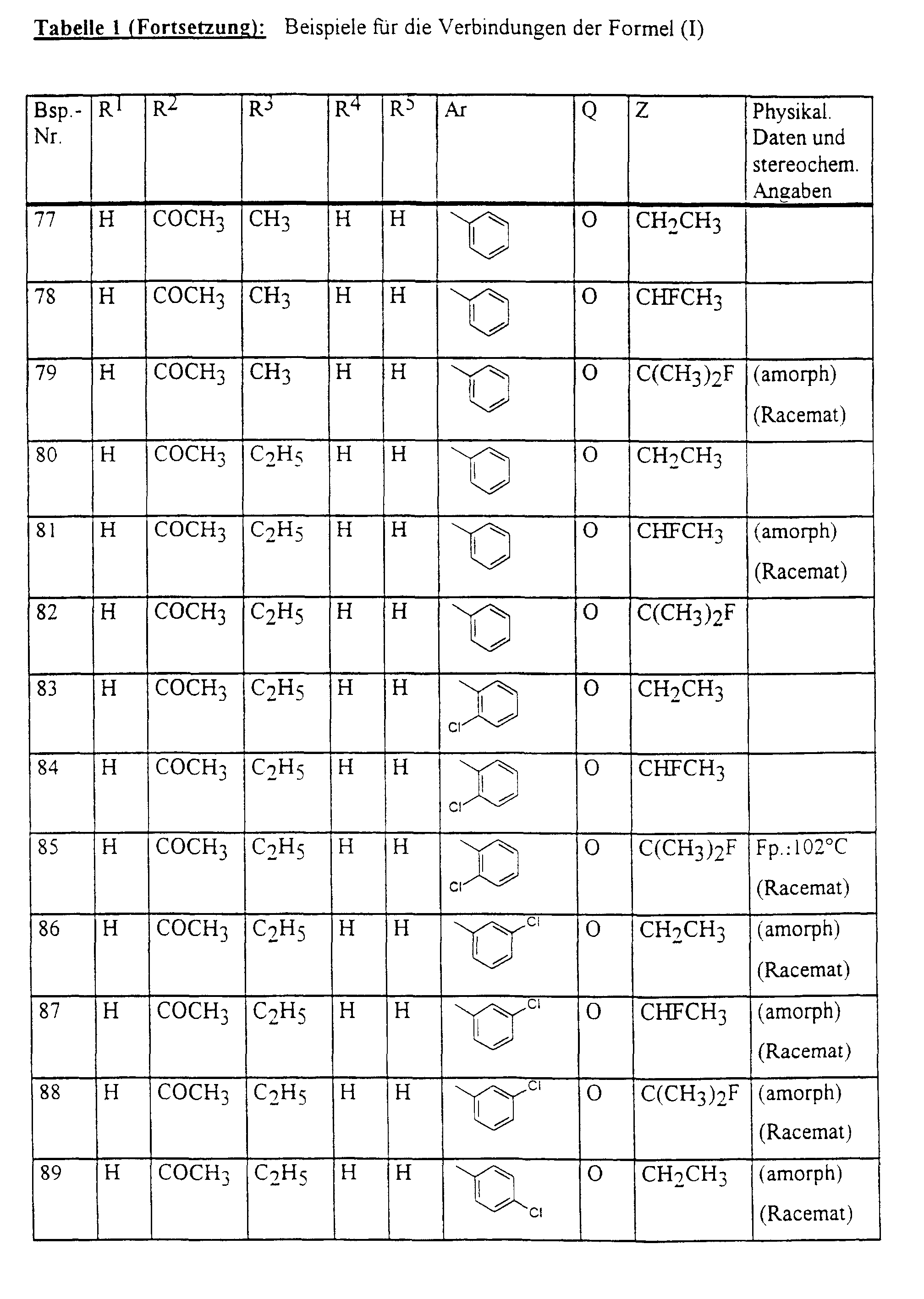

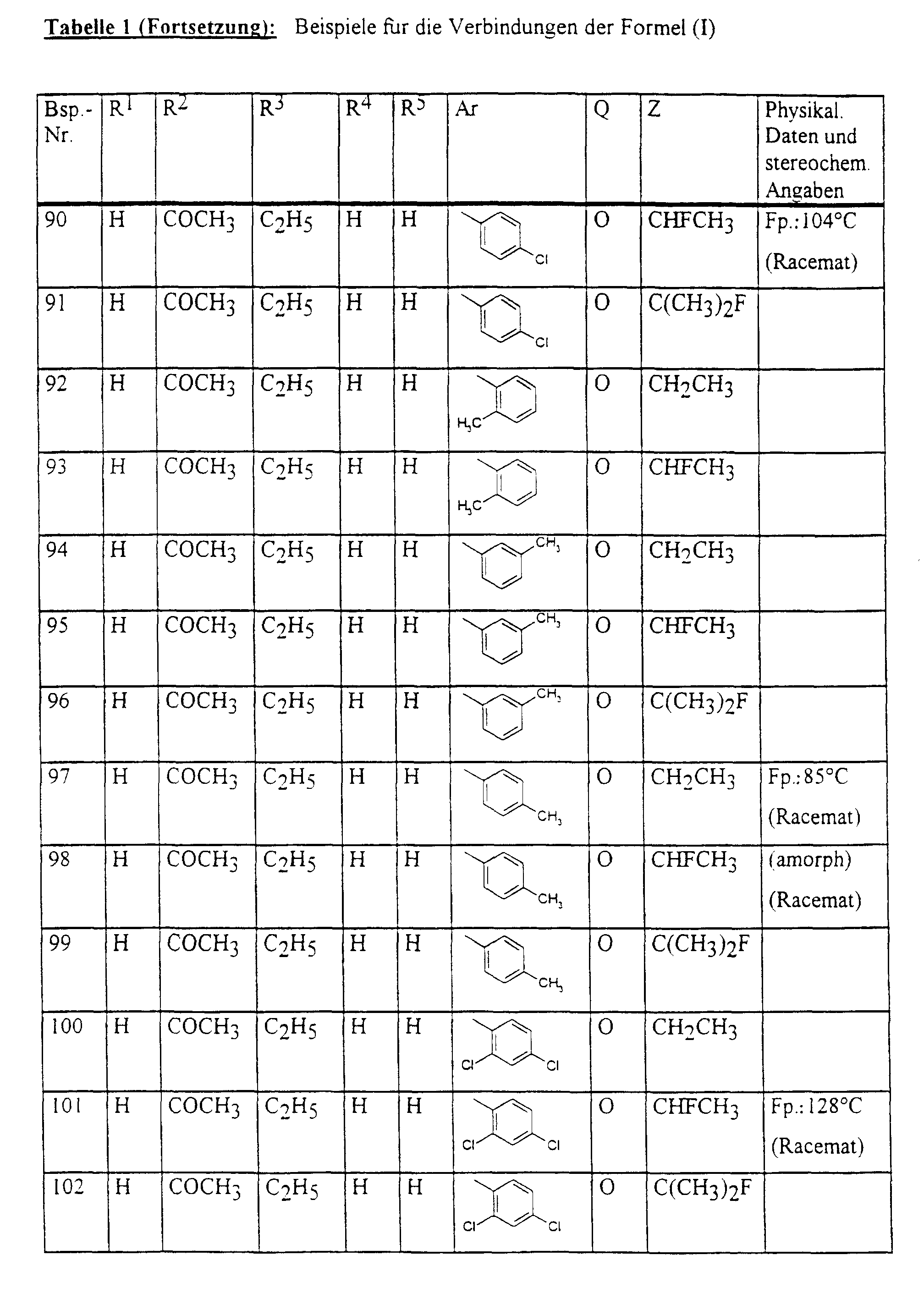

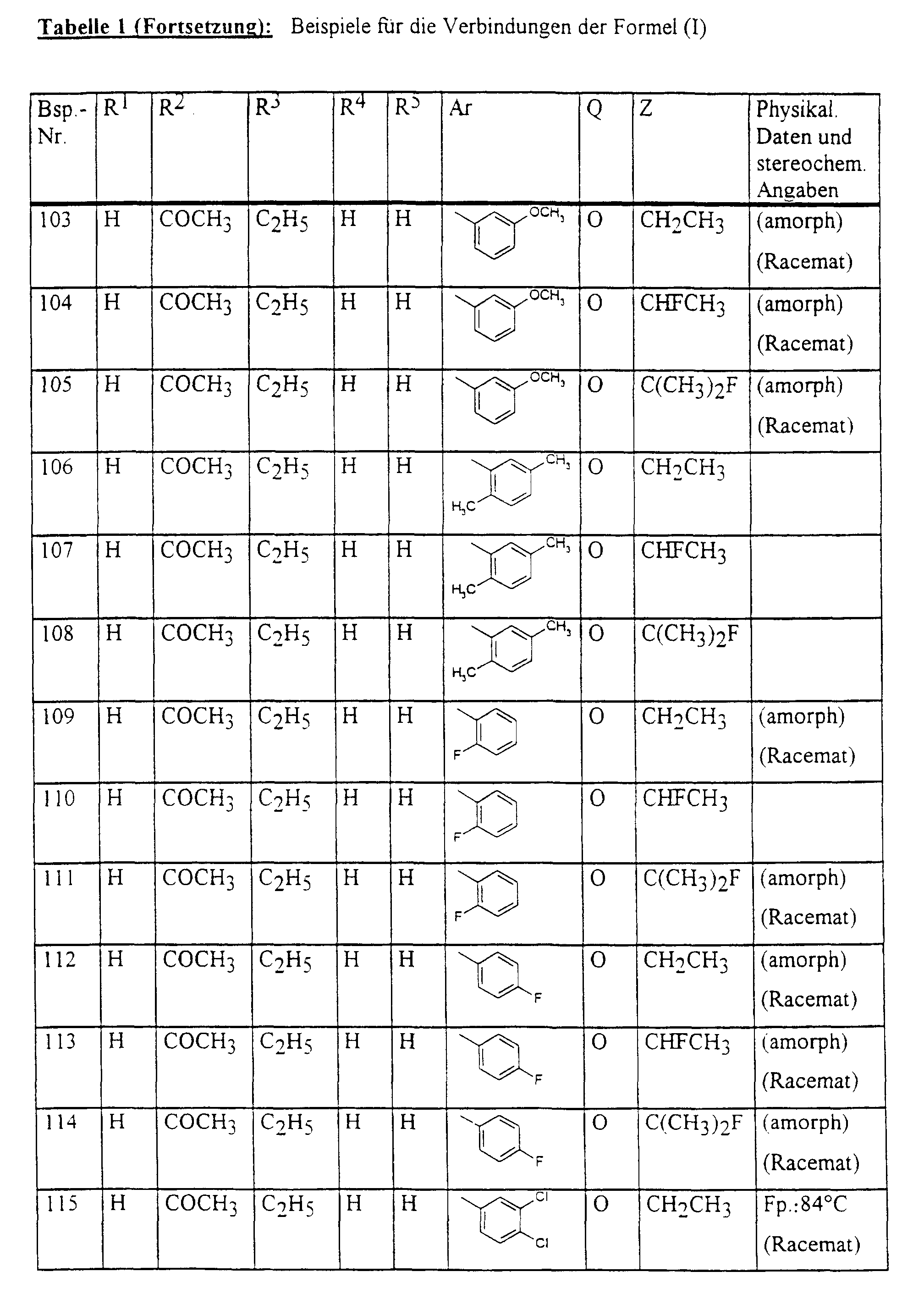

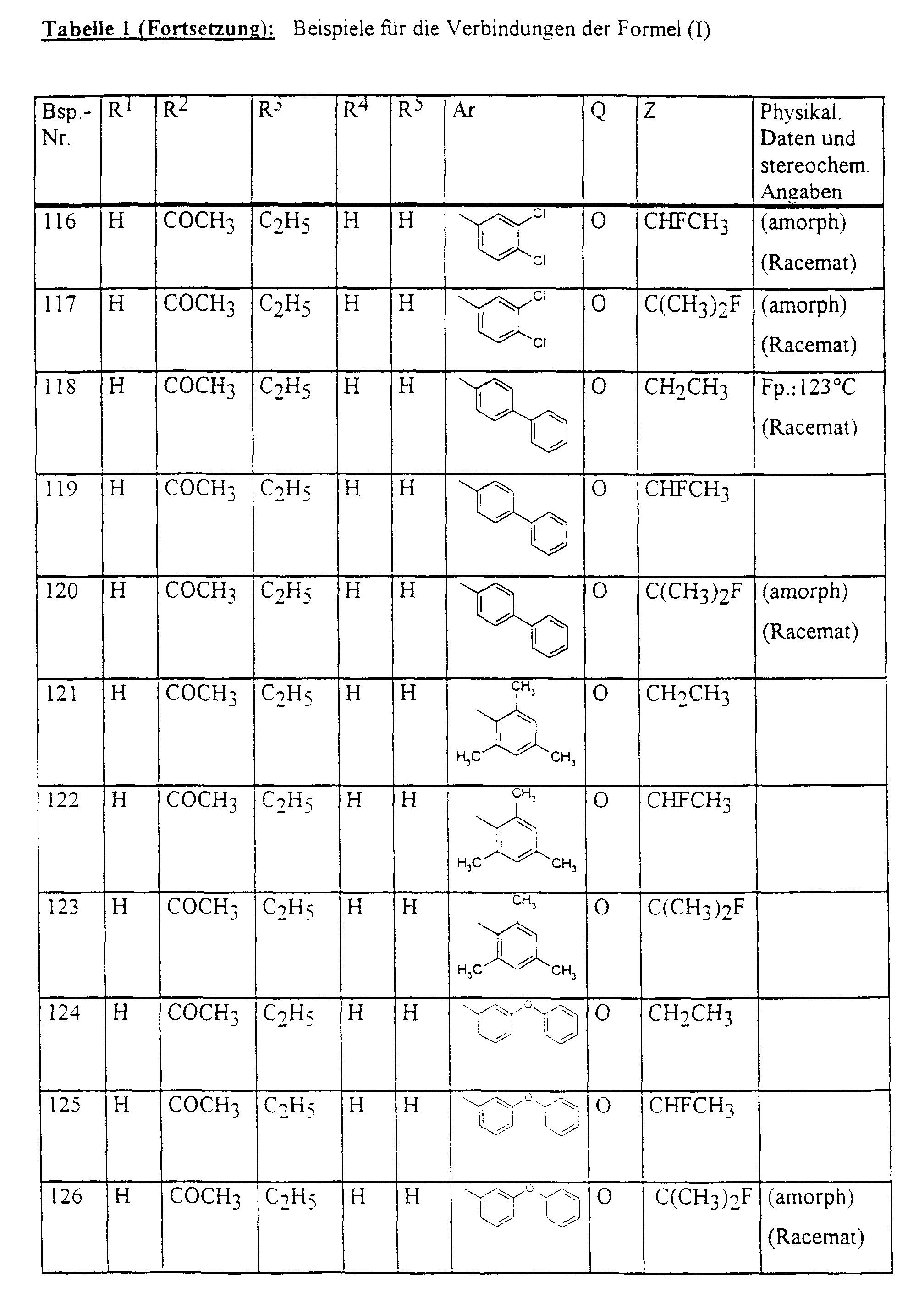

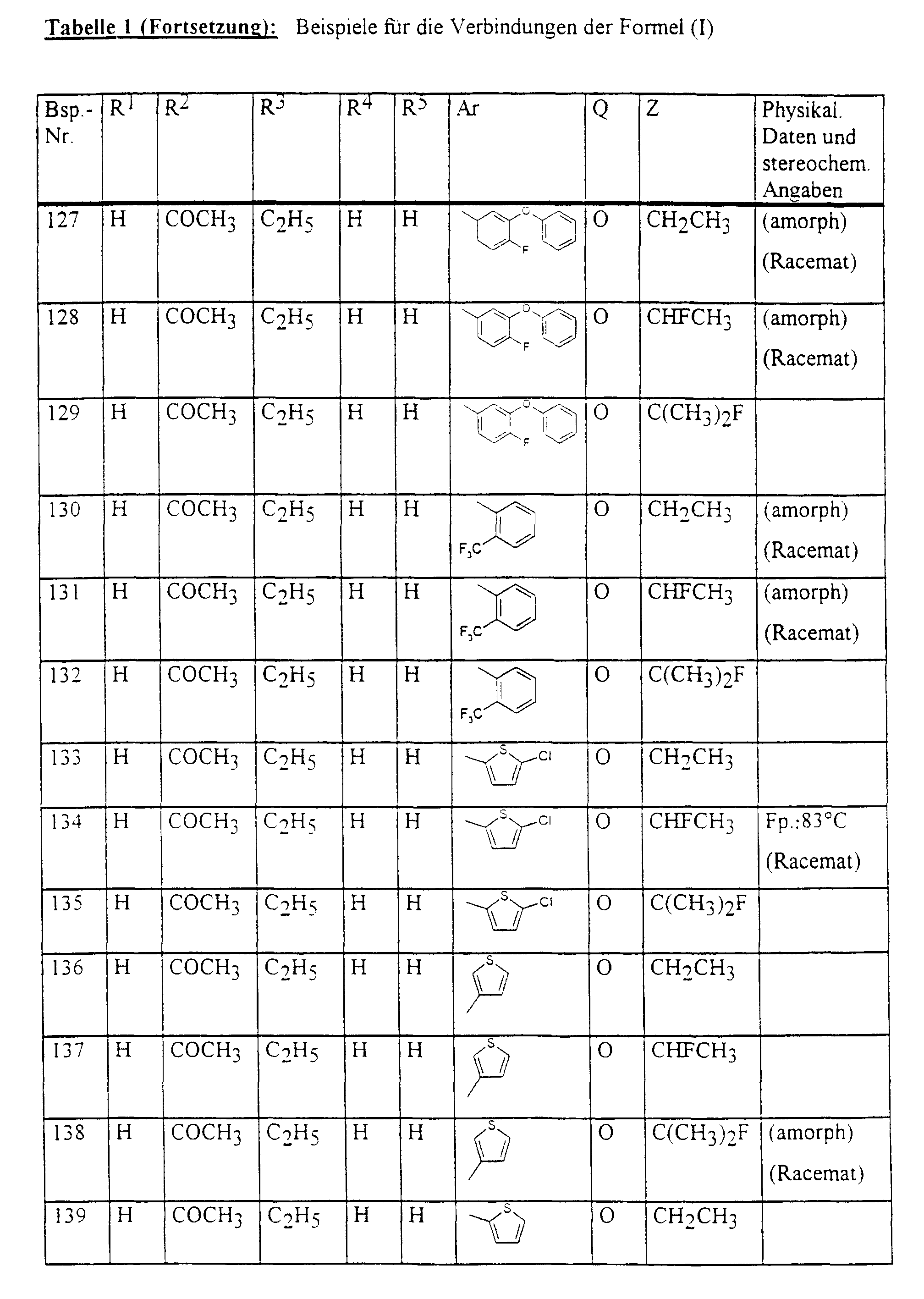

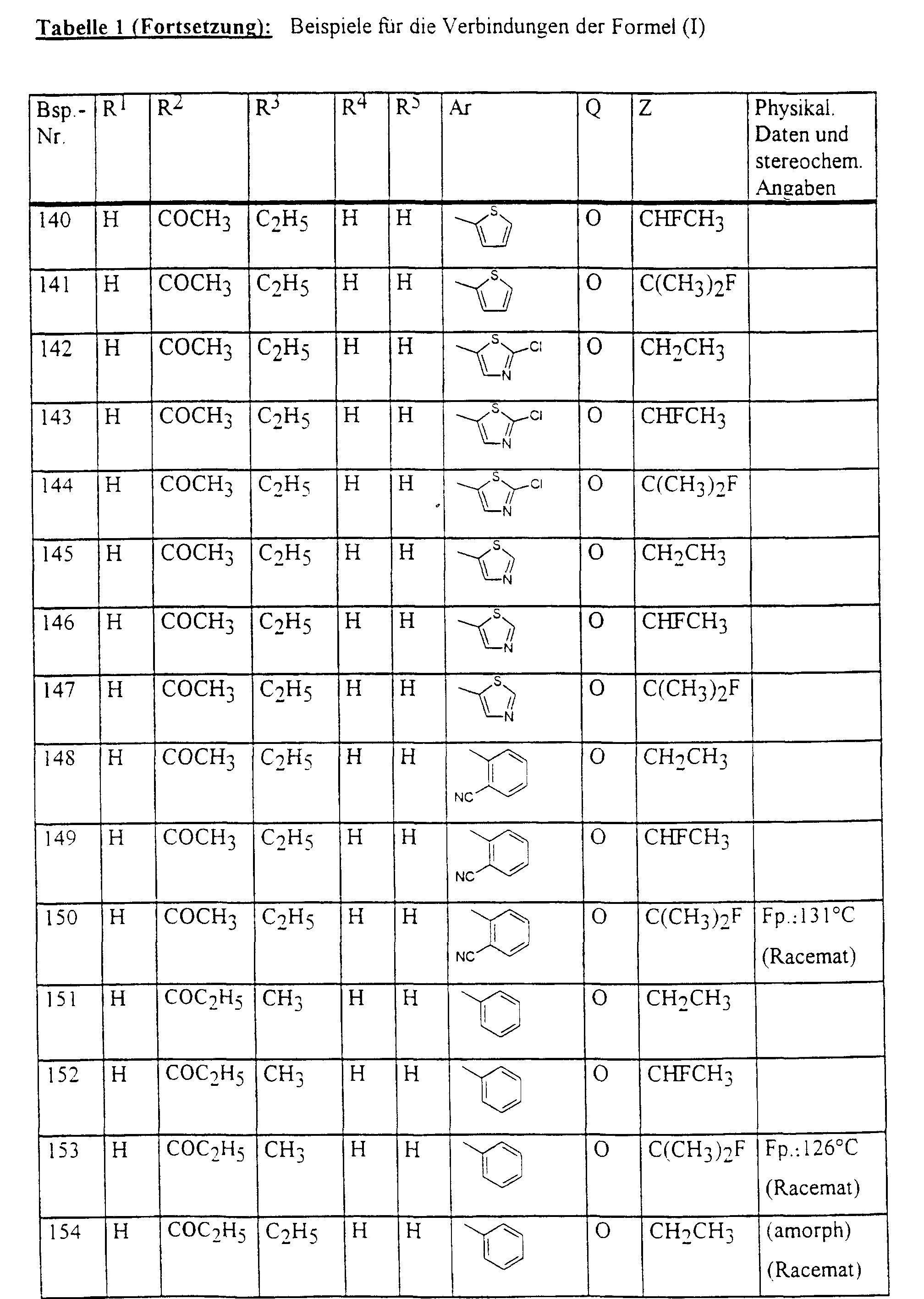

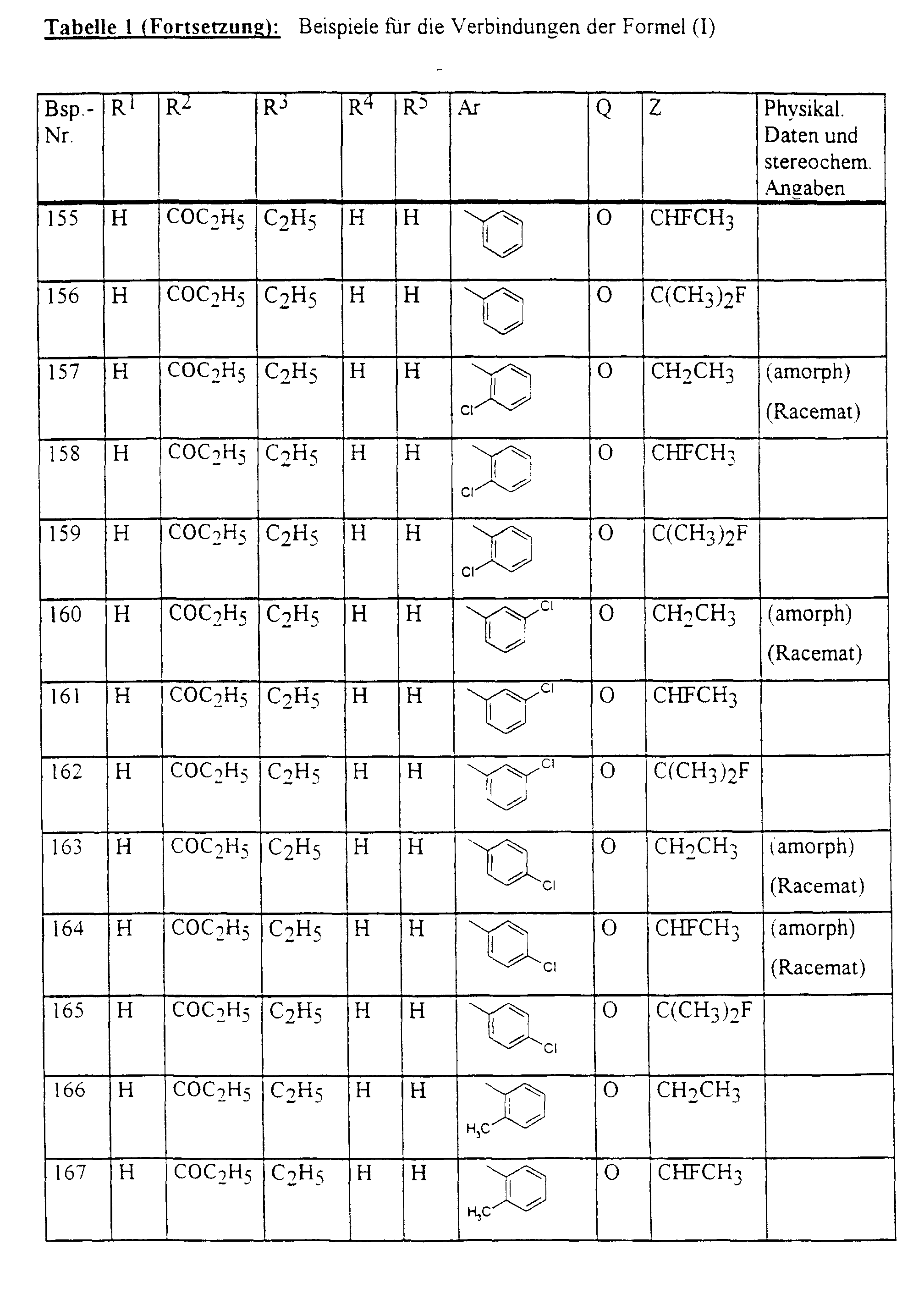

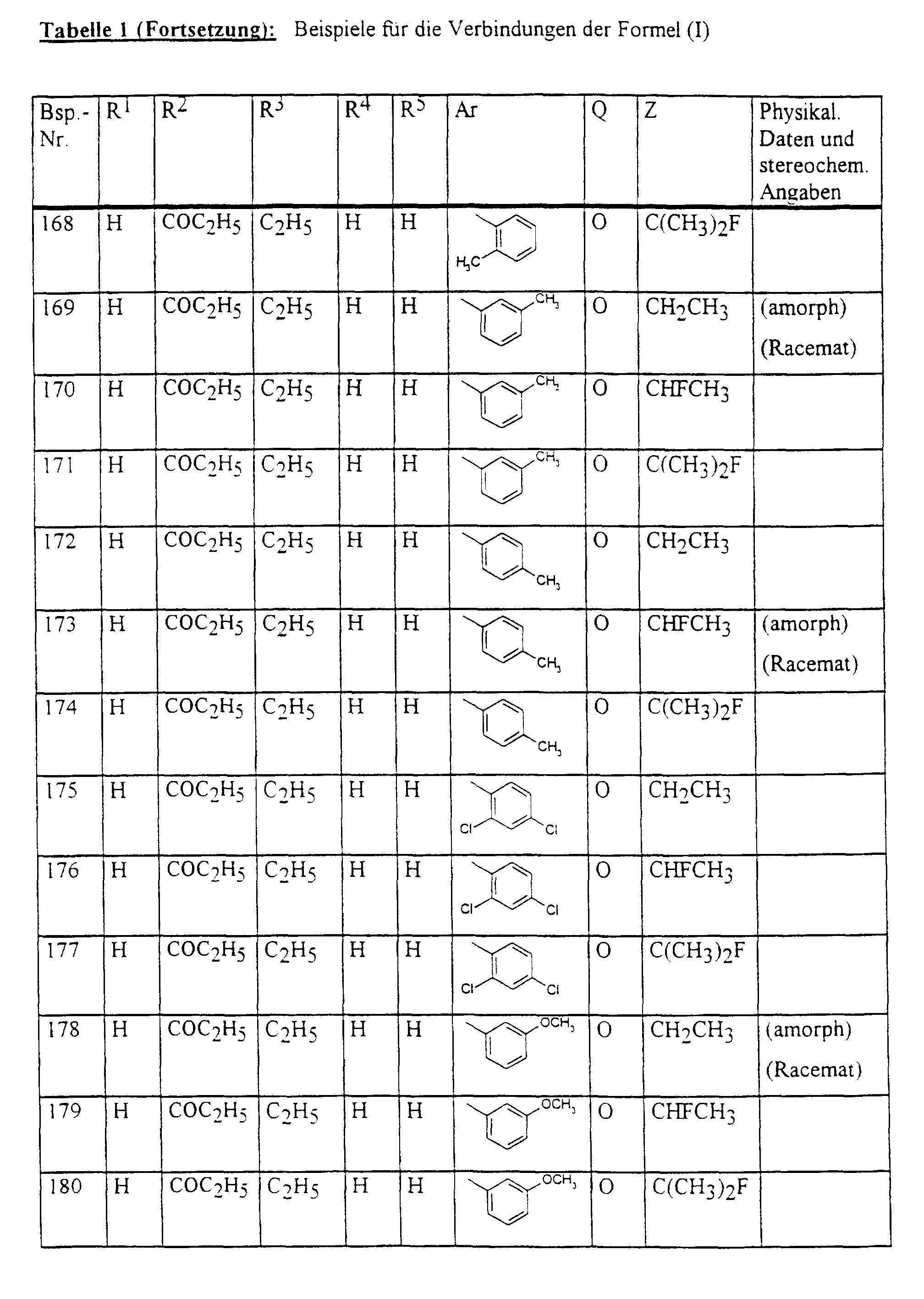

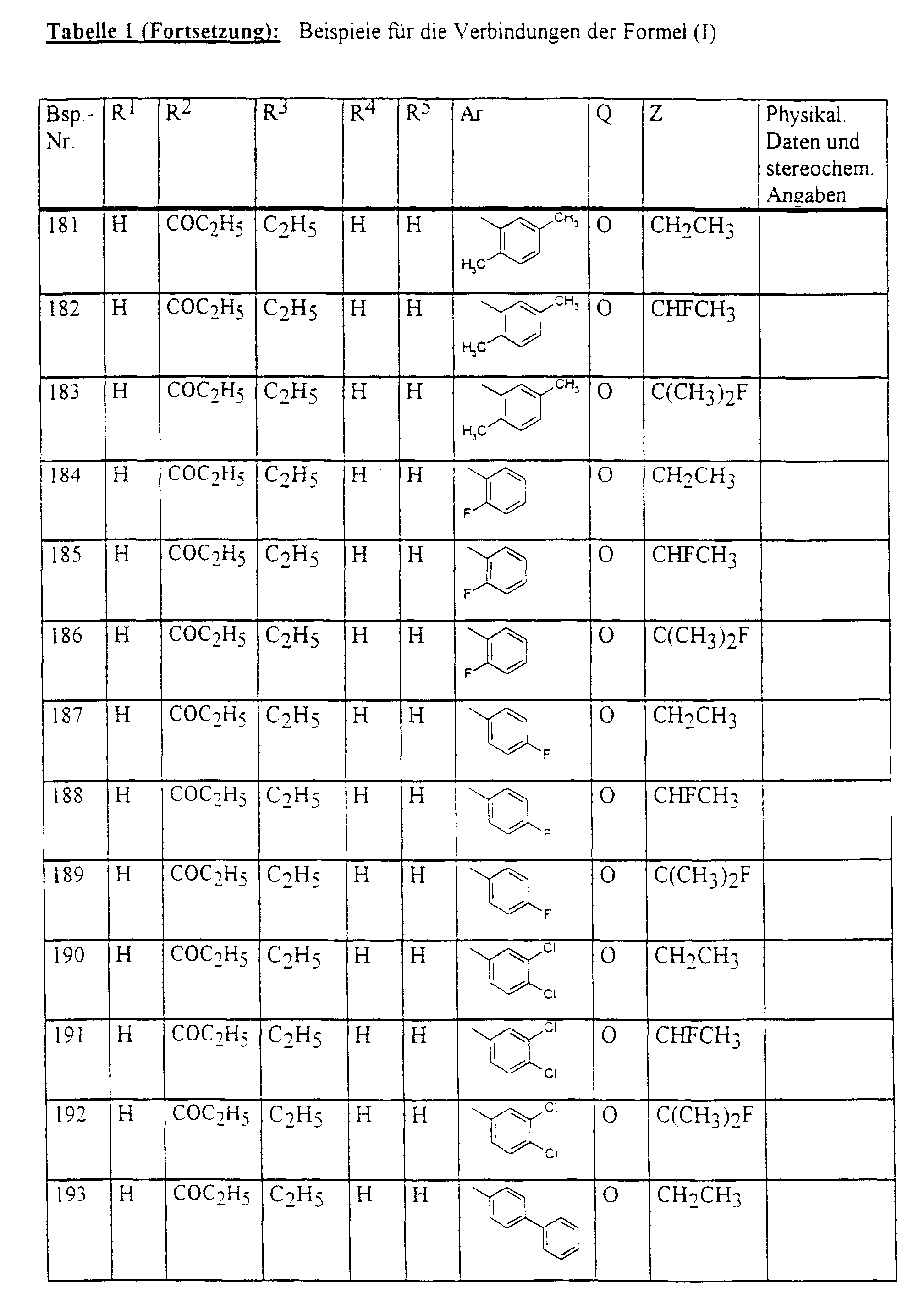

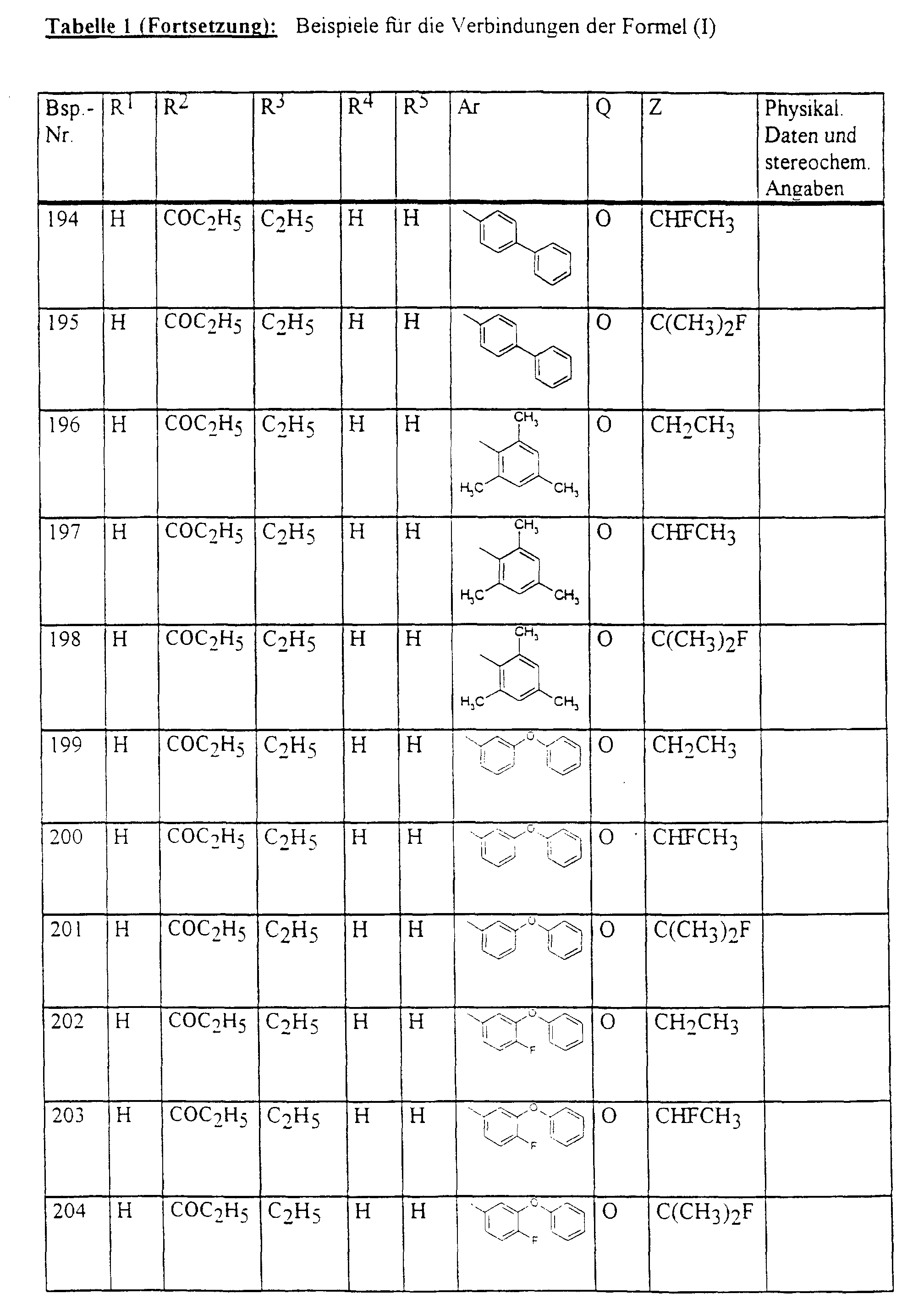

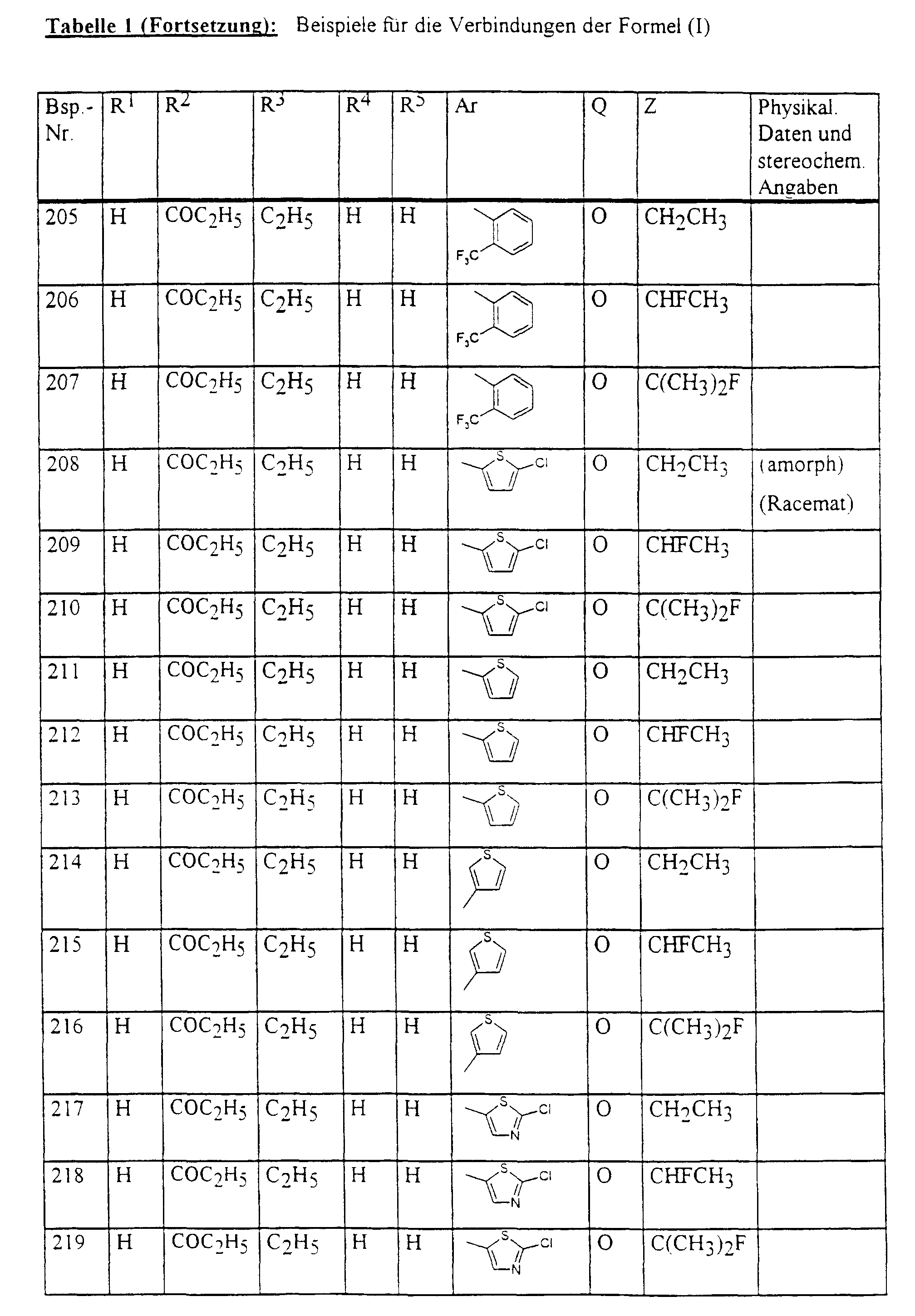

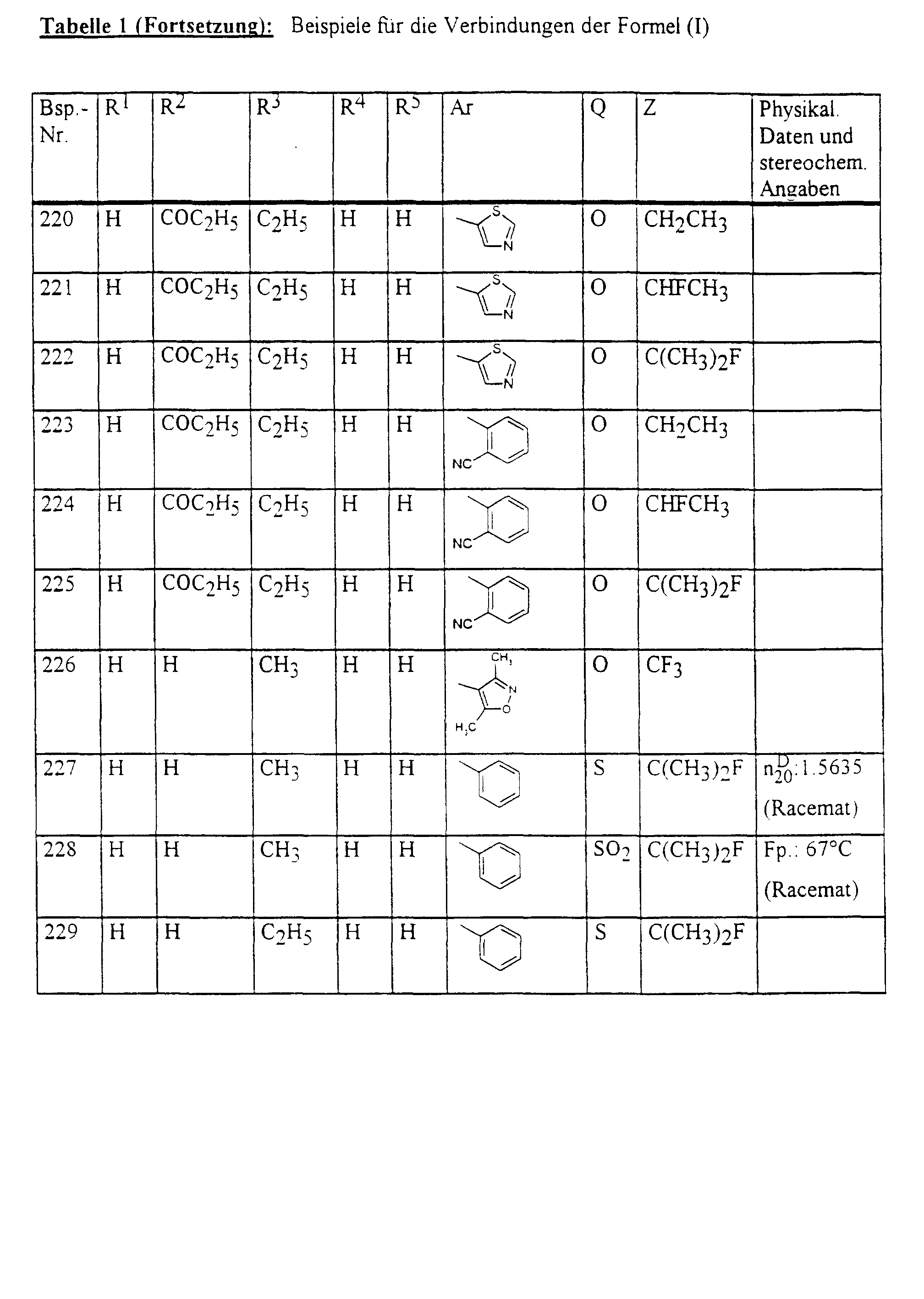

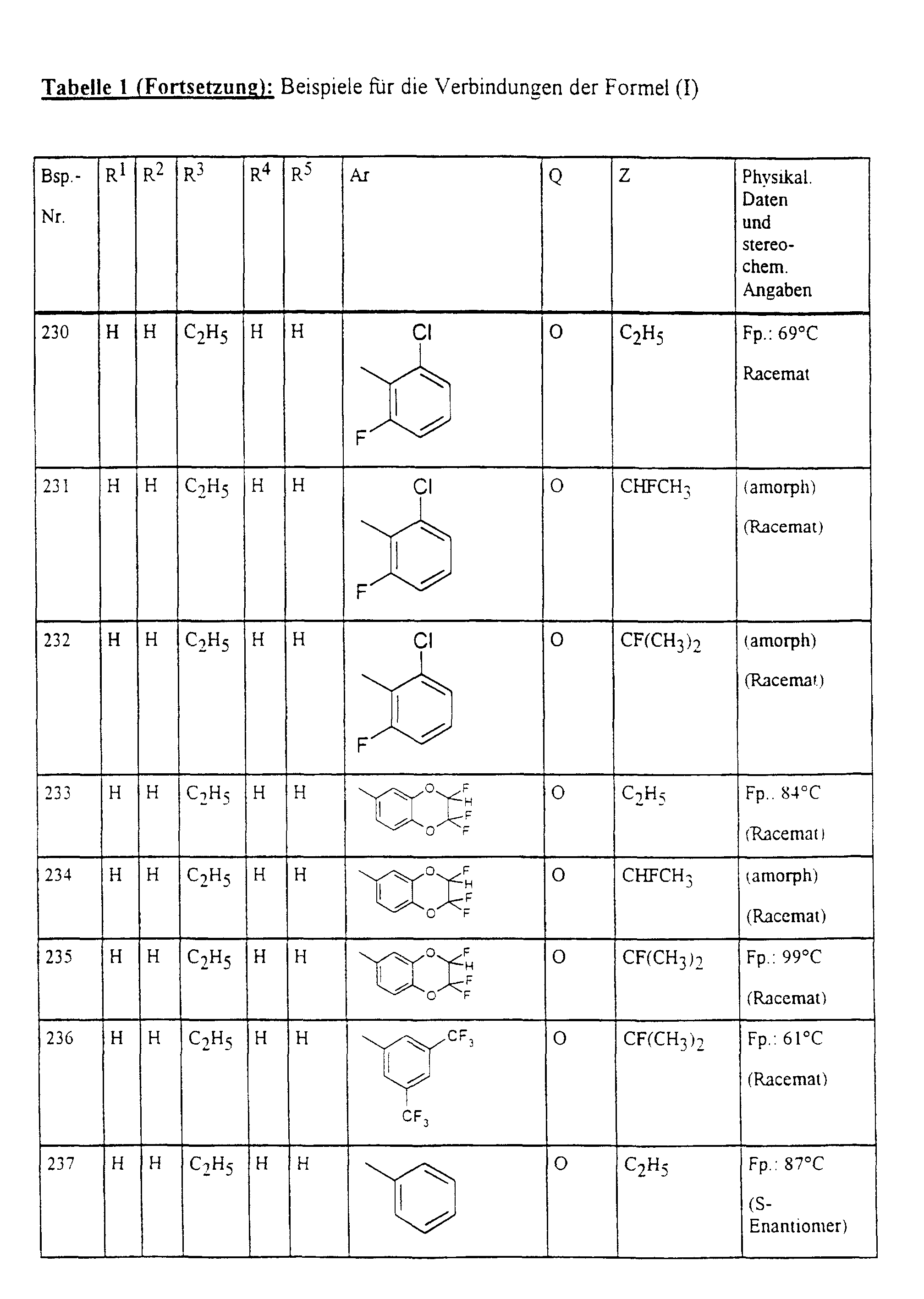

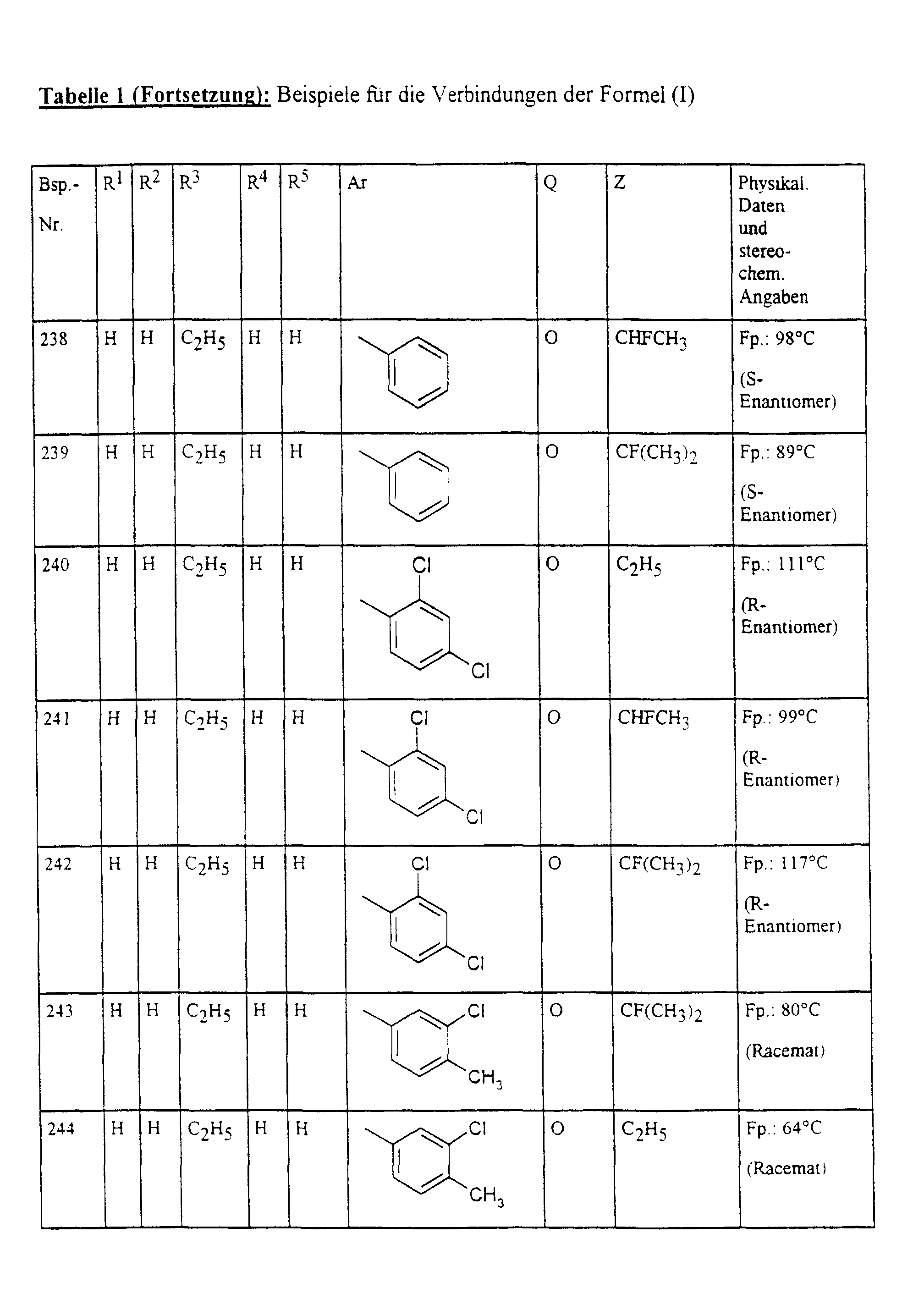

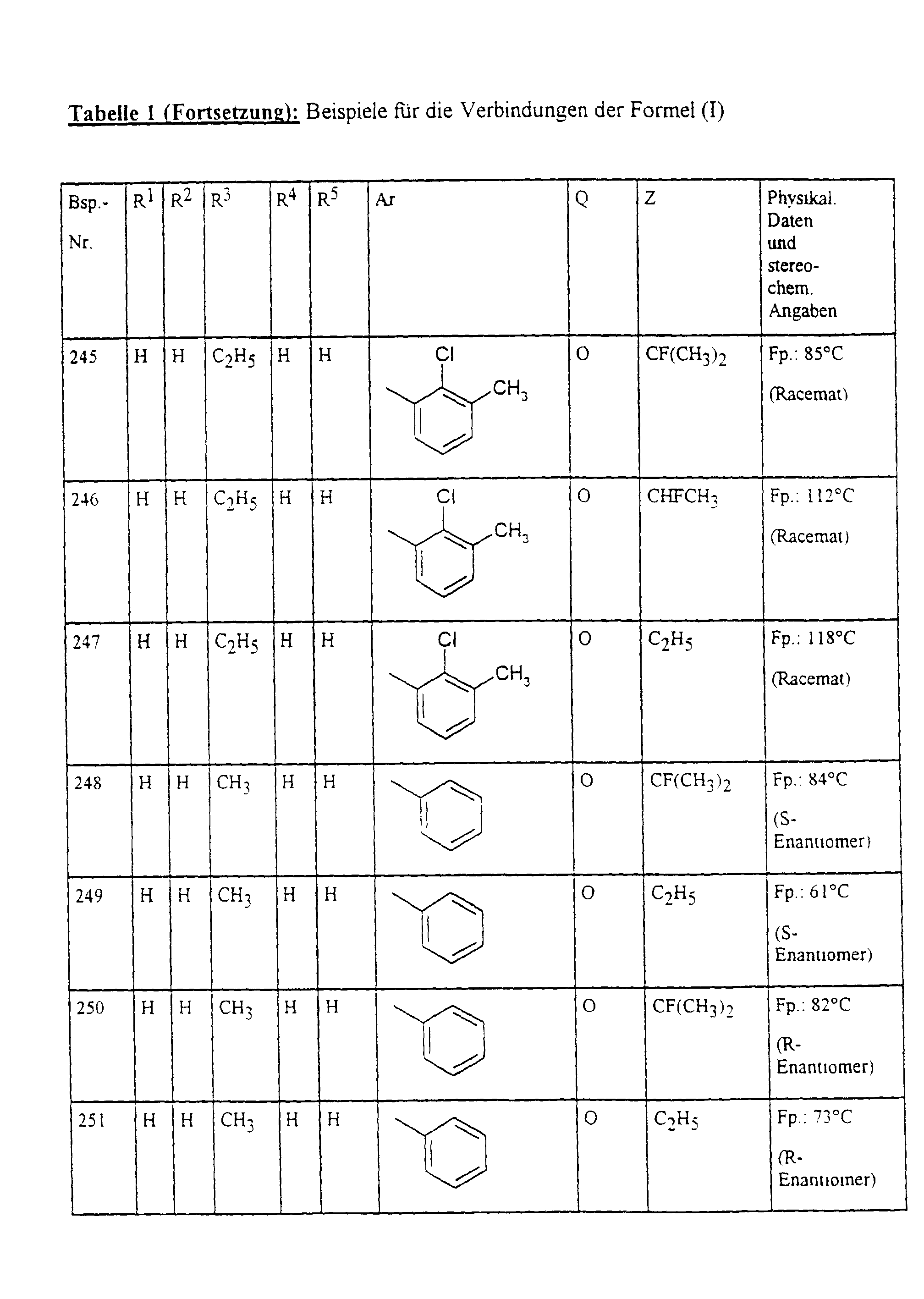

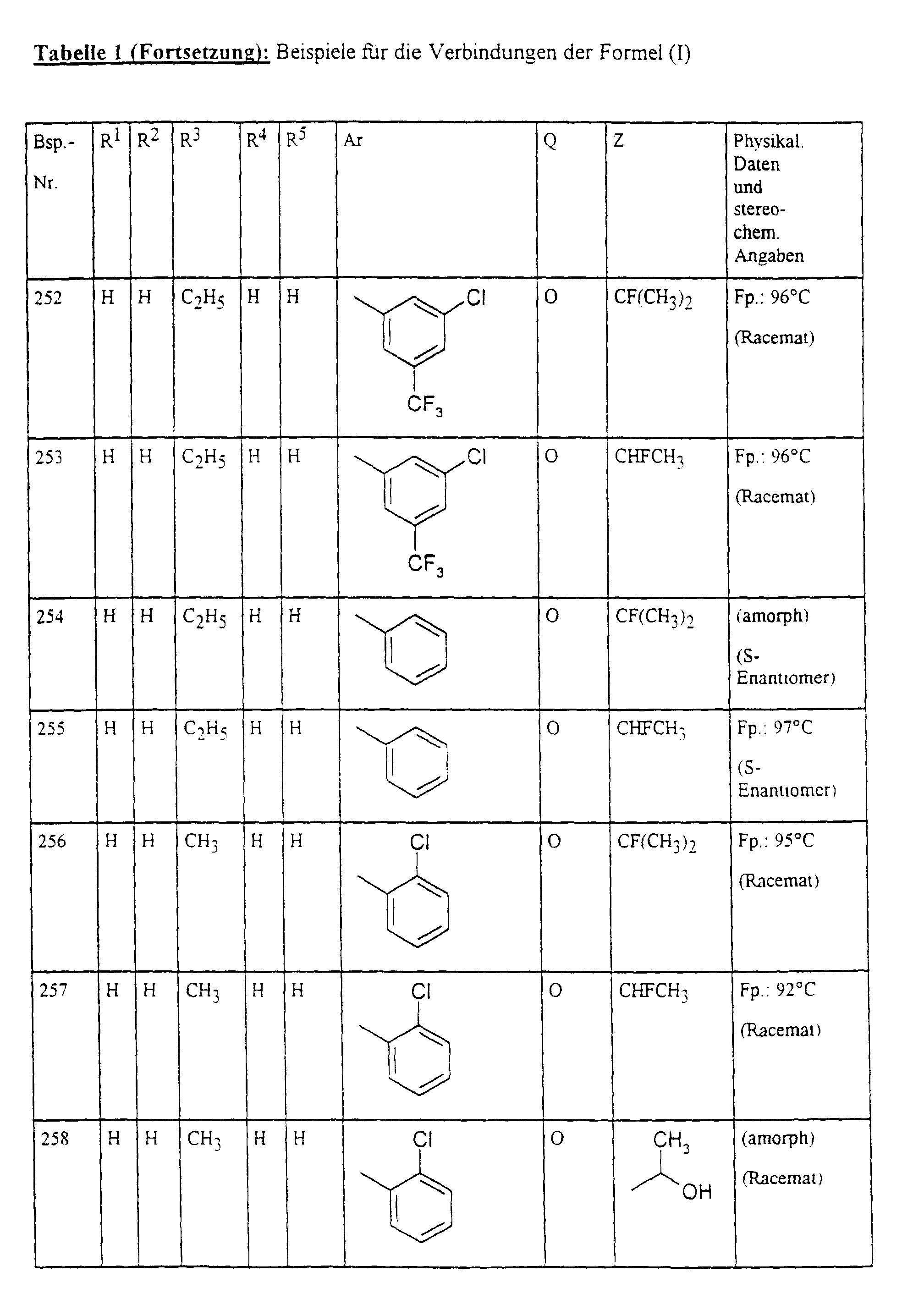

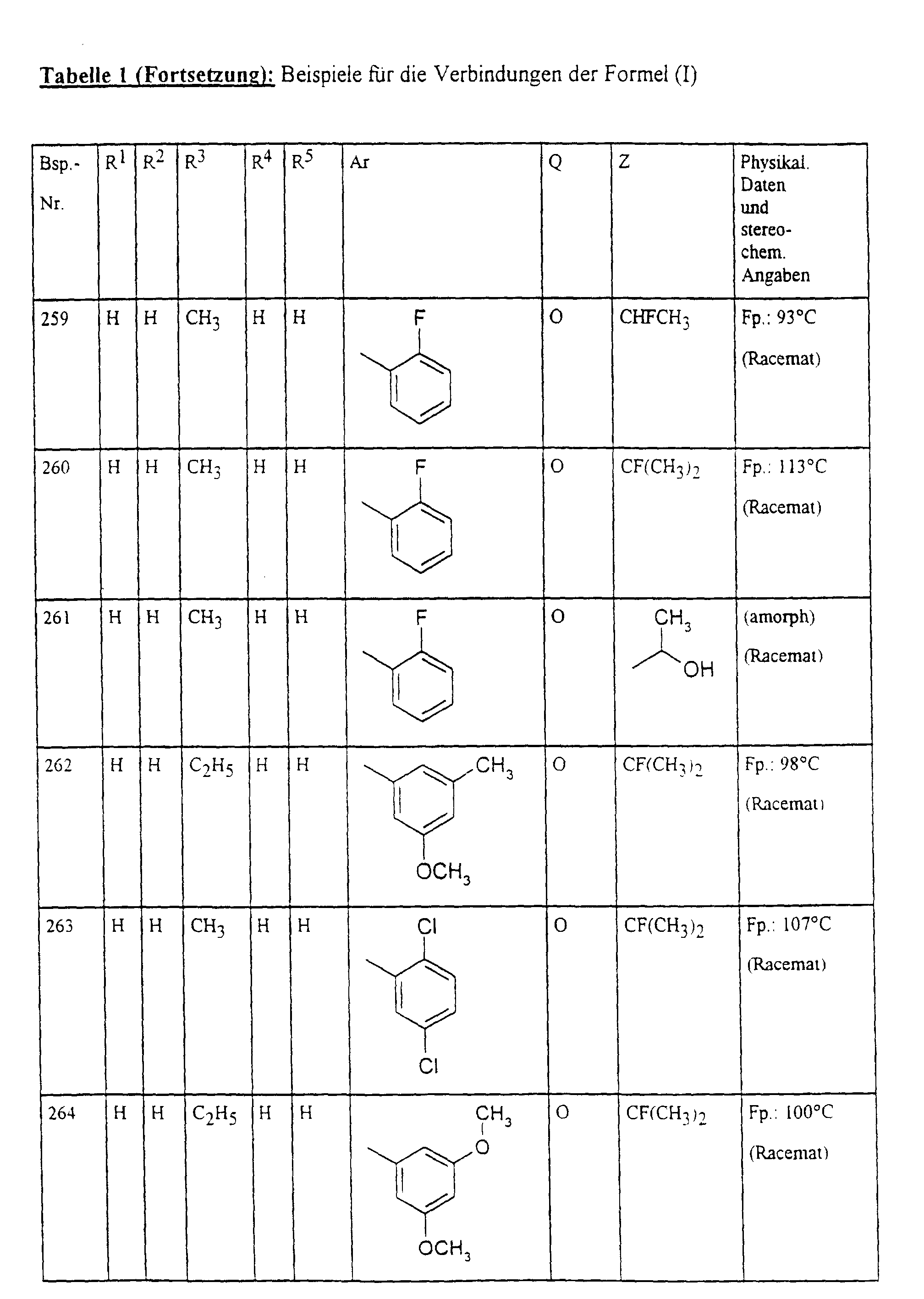

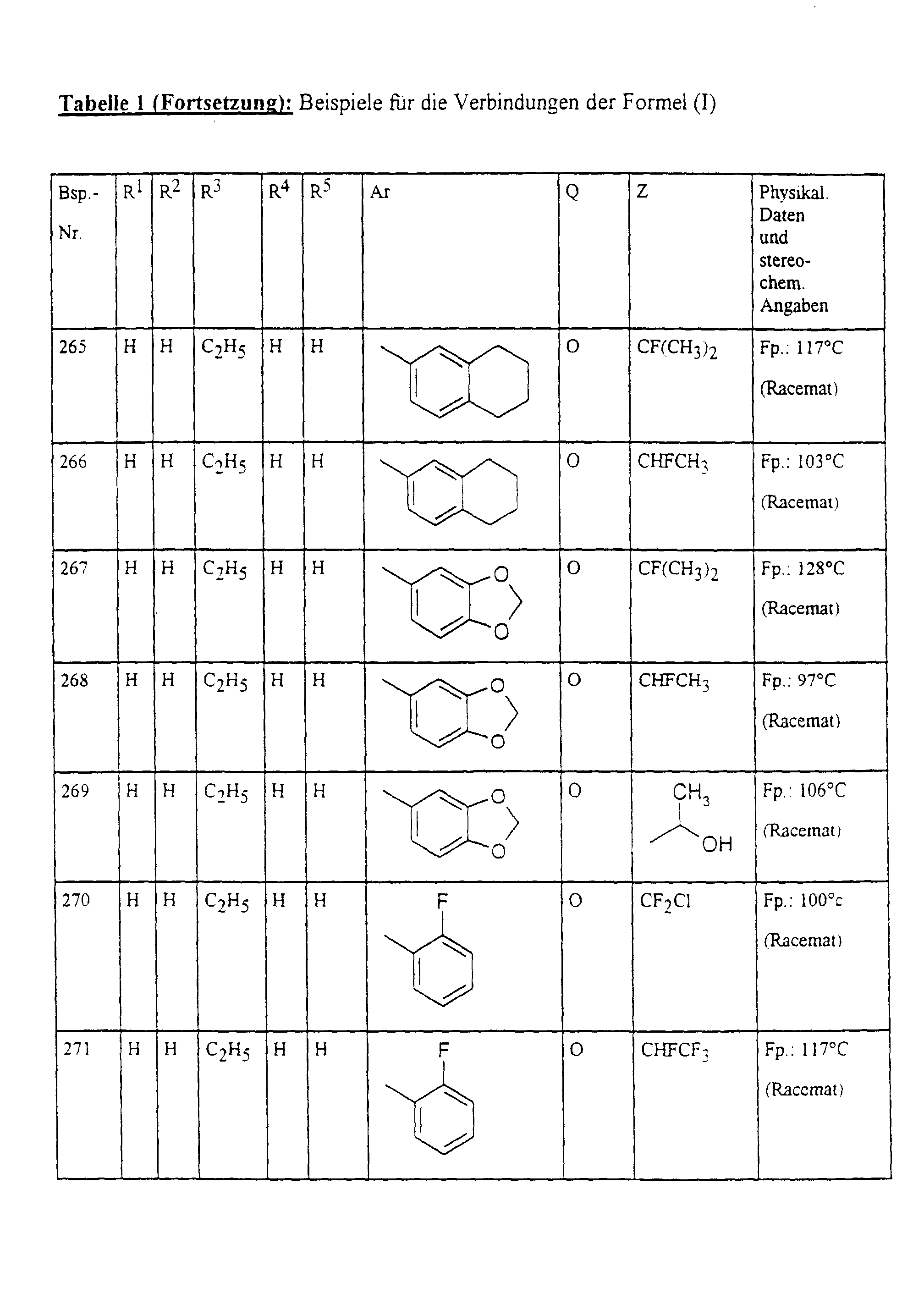

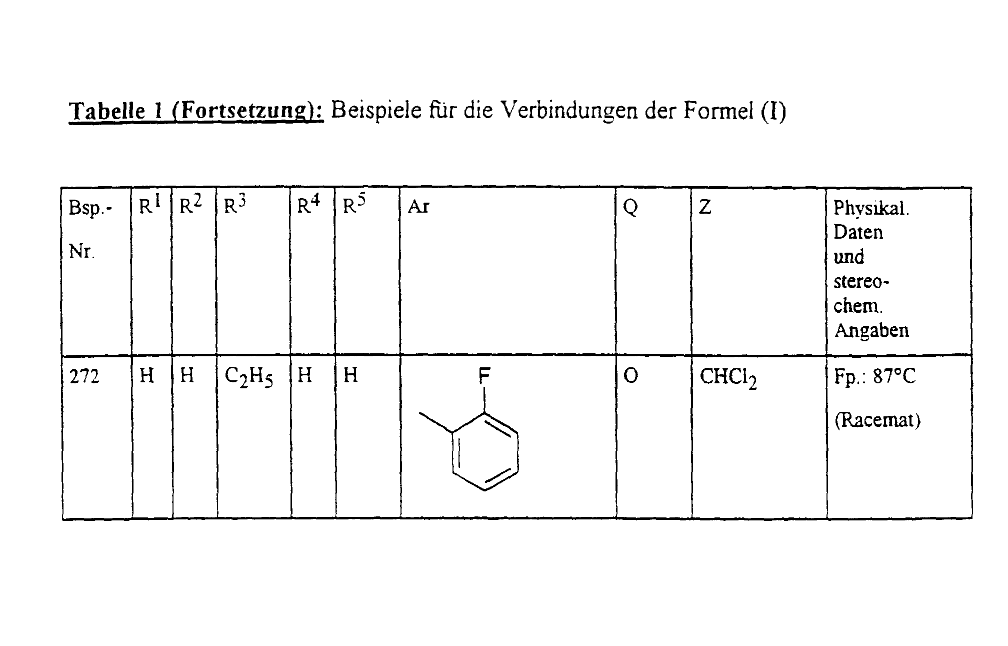

### Ausgangsstoffe der Formel (II):

### Beispiel (II-1)

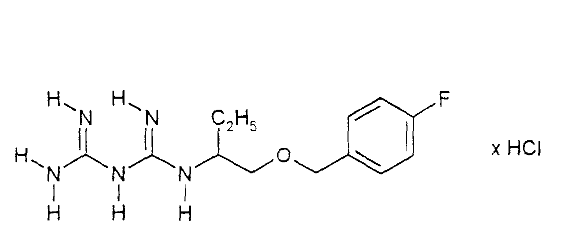

2,52 g (30 mmol) Cyanoguanidin und 7,01 g (30 mmol) 2-Amino-1-(4-fluor-benzyloxy)-butan-Hydrochlorid (racemisch) werden in einem Mörser innig verrieben und dann bei 160°C 30 Minuten zusammengeschmolzen. Während des Abkühlens wird die Mischung mit 30 ml Methanol versetzt. Die erhaltene Lösung von 1-[1-(4-Fluorbenzyloxymethyl)-propyl]-biguanid-Hydrochlorid wird gemäß Beispiel 1 umgesetzt.

Analog Beispiel (II-1) können beispielsweise auch die in der nachstehenden Verbindungen der Formel (II) hergestellt werden.

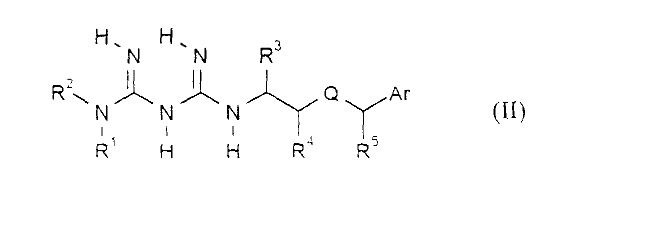

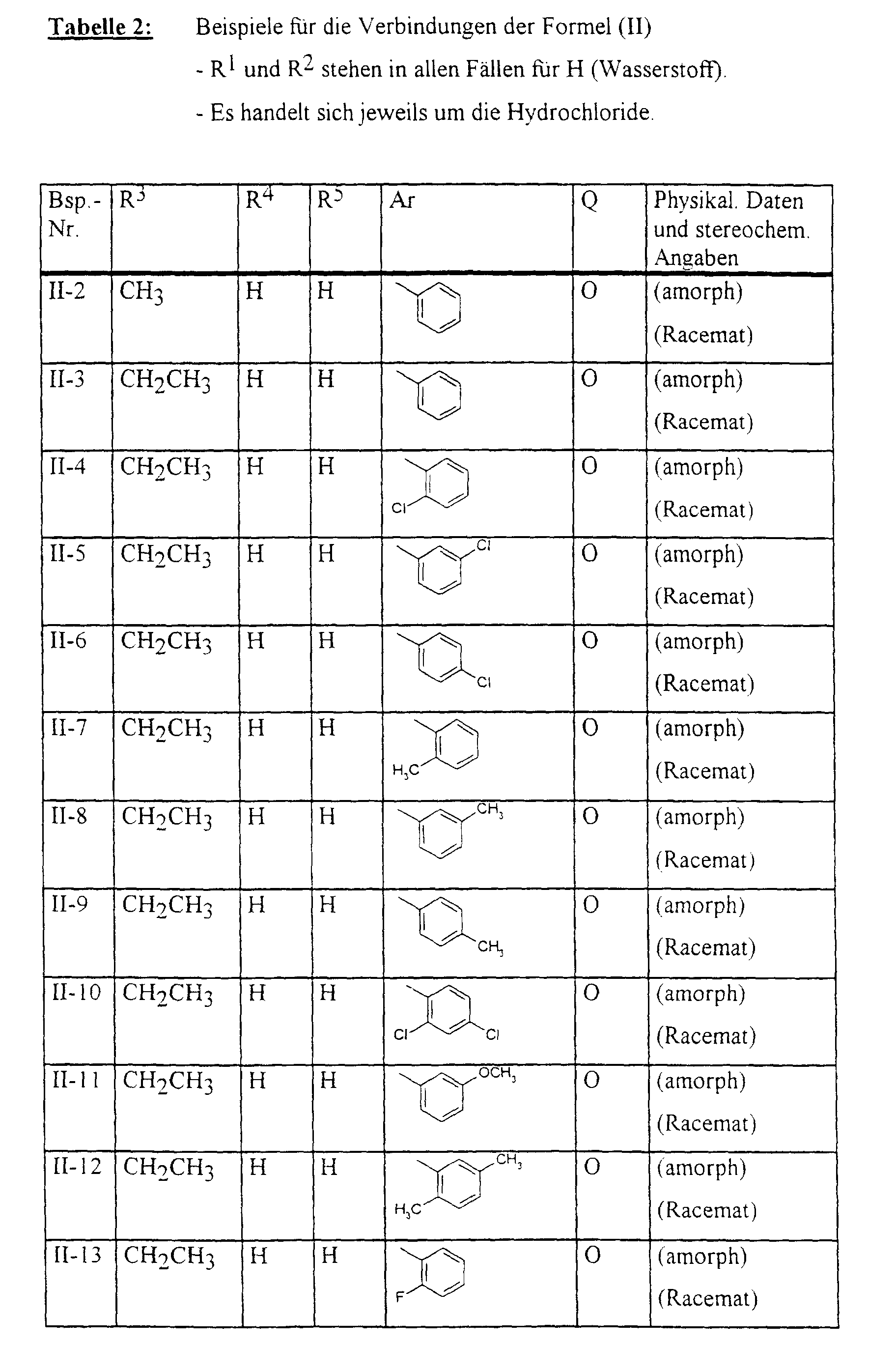

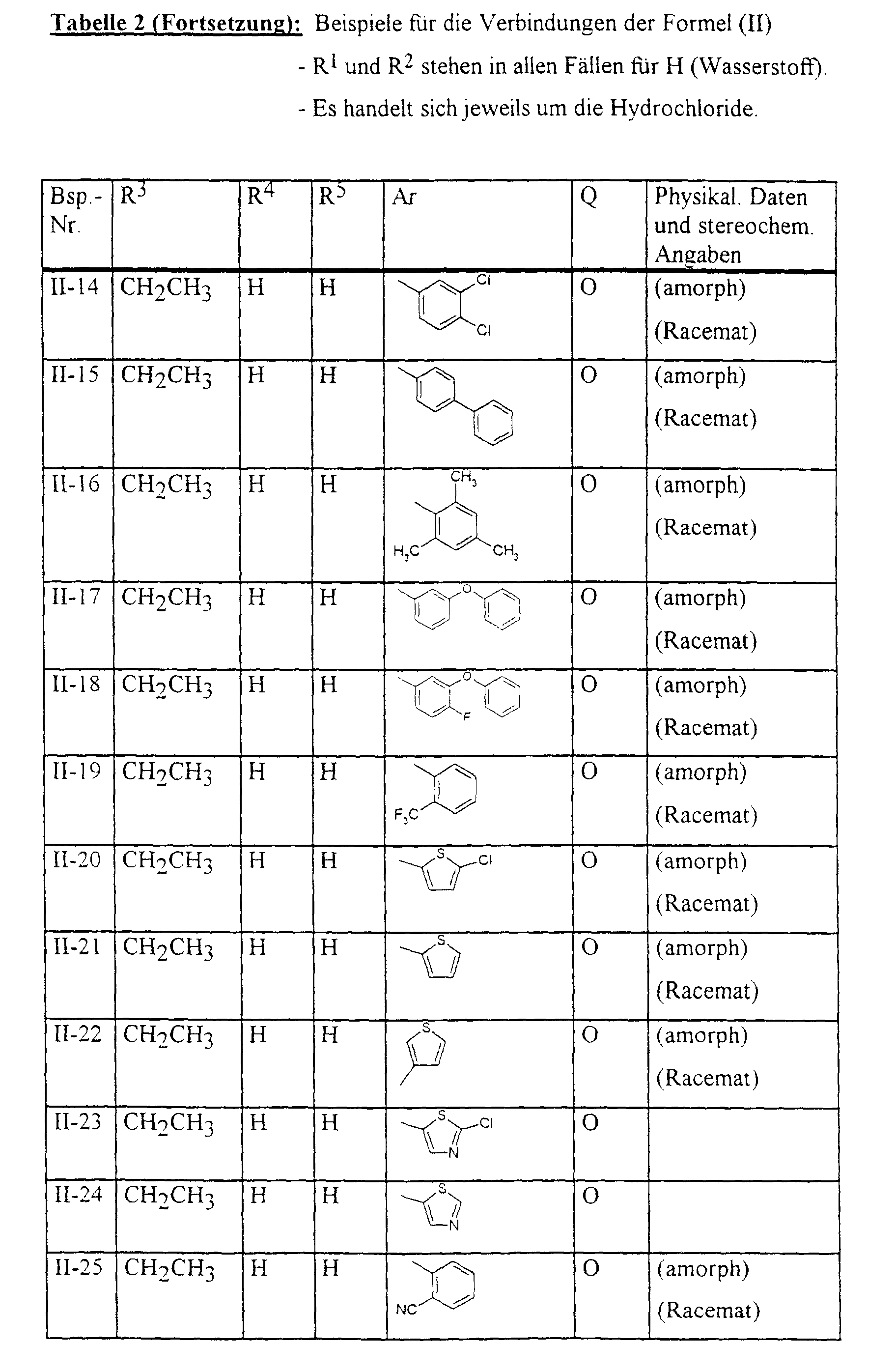

### Ausgangsstoffe der Formel (VII):

### Beispiel (VII-1)

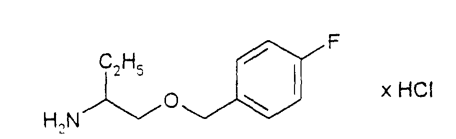

### Stufe 1

Eine Lösung von 7,5 ml (80 mmol) 2-Amino-1-butanol in 90 ml Tetrahydrofuran wird bei Raumtemperatur (ca. 20°C) portionsweise mit 2,4 g (80 mmol) Natriumhydrid (80%ig) versetzt und anschließend 30 Minuten unter Rückfluß erhitzt. Nach dem Abkühlen werden 10,3 g (71 mmol) 4-Fluor-benzylchlorid hinzugegeben und die Mischung wird 2 Stunden unter Rückfluß erhitzt. Nach erneutem Abkühlen wird soviel Wasser hinzugesetzt, bis sich das ausgefallene Salz gelöst hat und aus der Mischung wird das Lösungsmittel im Wasserstrahlvakuum abdestilliert. Der Rückstand wird auf IN-Salzsäure gegeben und zweimal mit Dichlormethan extrahiert. Die Dichlormethan-Phase wird mit Wasser ruckextrahiert. Dann wird die wässrige Phase mit konz. Natronlauge auf pH 10 eingestellt und dreimal mit Dichlormethan extrahiert. Die organische Extraktionslösung wird über Natriumsulfat getrocknet und filtriert Vom Filtrat wird das Lösungsmittel im Wasserstrahlvakuum sorgfältig abdestilliert.

Man erhält 13,4 g (96 % der Theorie) 2-Amino-1-(4-fluor-benzyloxy)-butan als öligen Rückstand.

### Stufe 2

13,4 g (68 mmol) 2-Amino-1-(4-fluorbenzyloxy)butan werden in 40 ml Methanol gelöst und mit 7,7 g (68 mmol) konz. Salzsäure versetzt. Man entfernt das Losungsmittel im Wasserstrahlvakuum, versetzt den Ruckstand mit 20 ml Toluol und engt wiederum im Wasserstrahlvakuum ein. Man erhält 14,9 g (94% der Theorie) 2-Amino-1-(4-fluor-benzyloxy)-butan-Hydrochlorid als weißen Feststoff.

Analog Beispiel (VII-1) können beispielsweise auch die in der nachstehenden Tabelle 3 aufgeführten Verbindungen der Formel (VII) hergestellt werden.

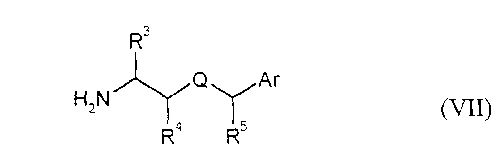

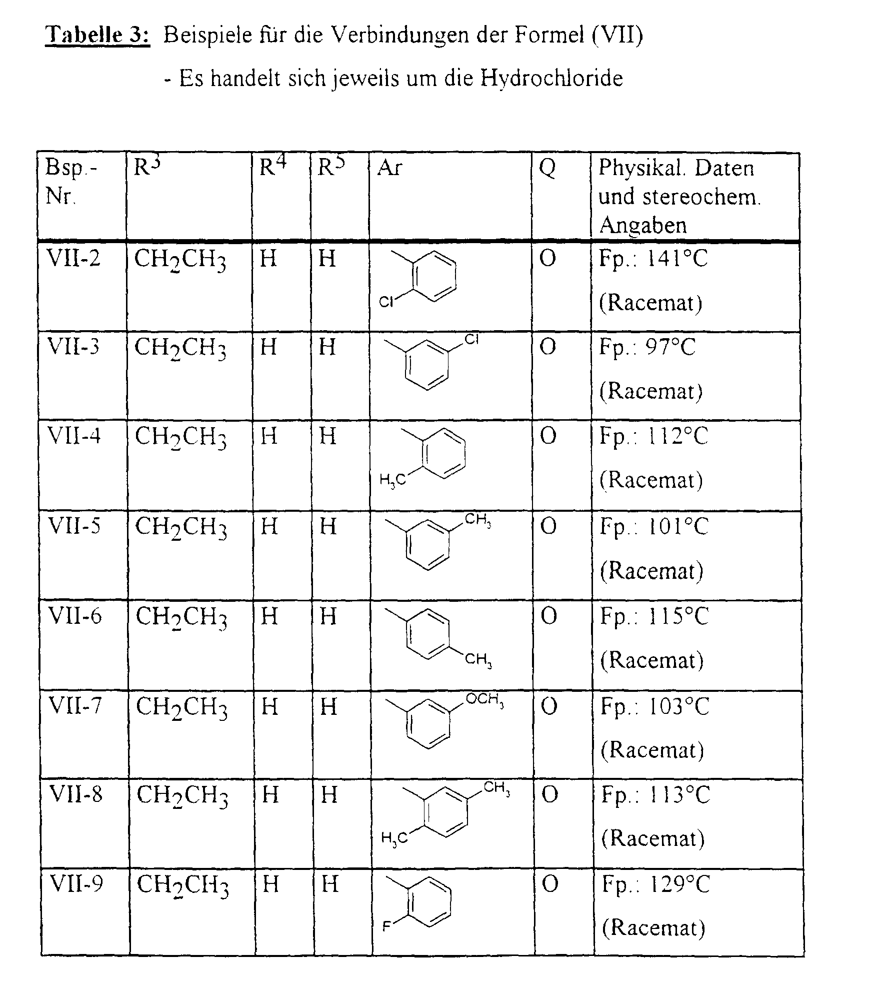

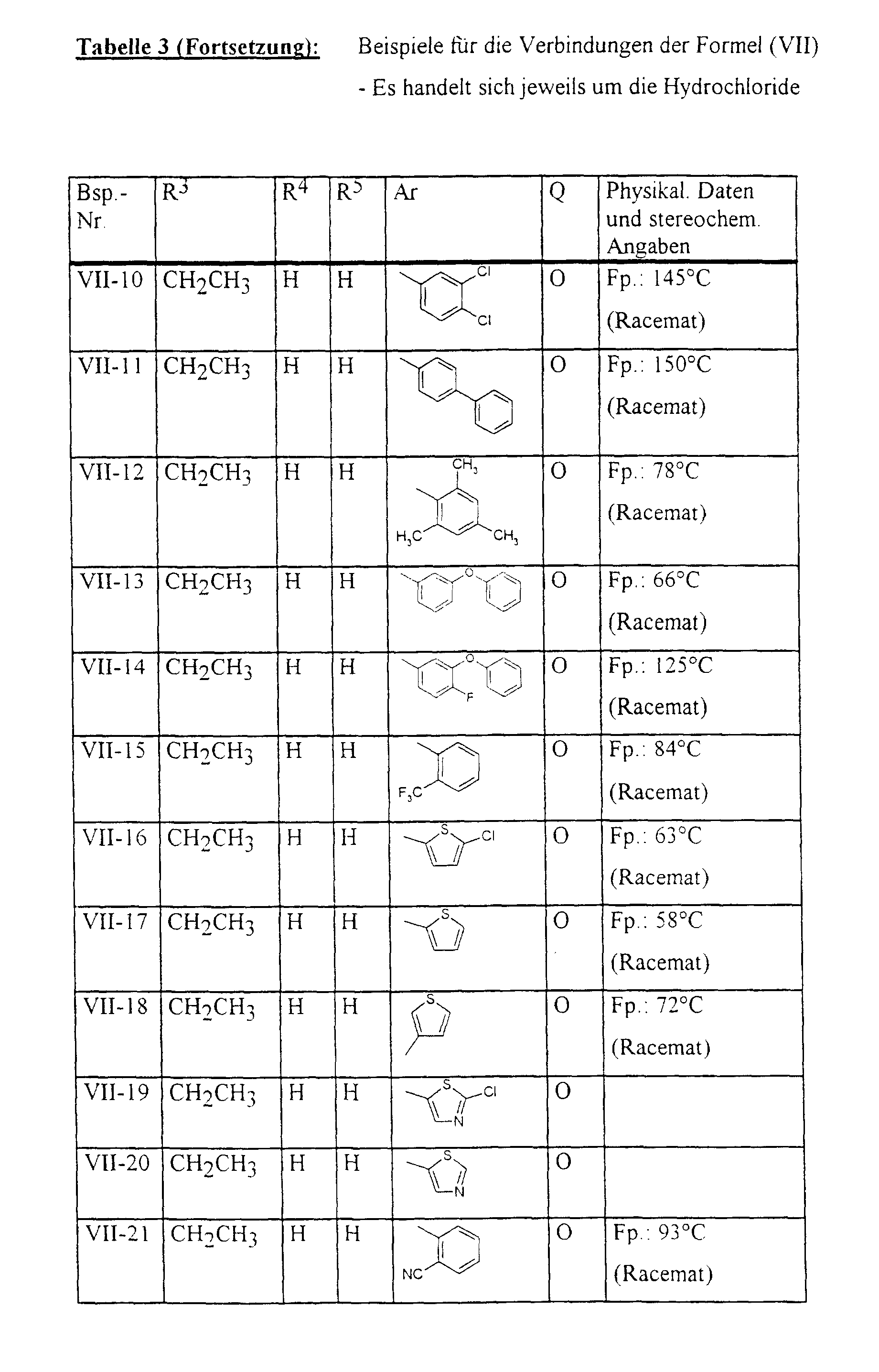

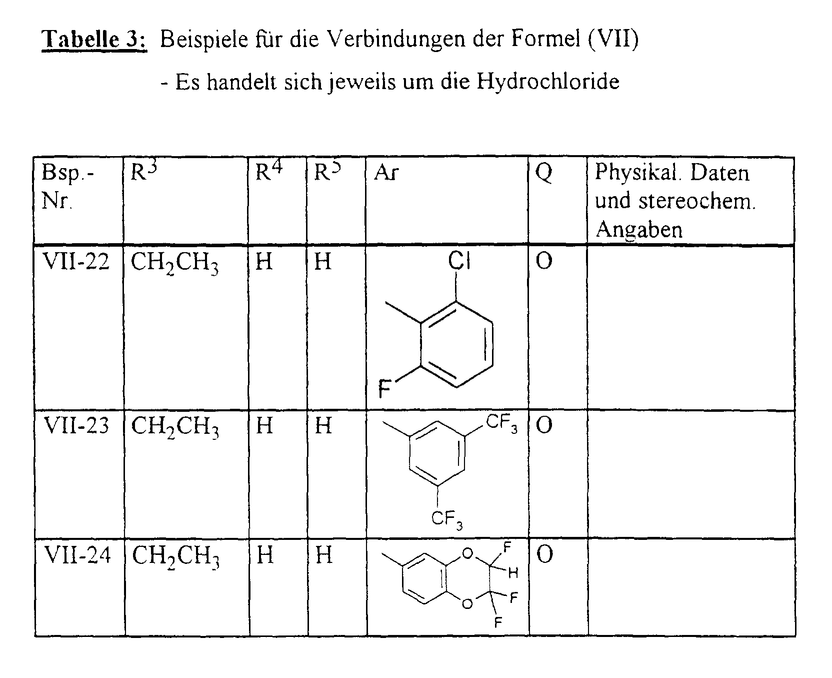

### Anwendungsbeispiele:

### Beispiel A

### Pre-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdunnt das Konzentrat mit Wasser auf die gewünschte Konzentration.

Samen der Testpflanzen werden in normalen Boden ausgesät. Nach ca. 24 Stunden wird der Boden mit der Wirkstoffzubereitung so gespritzt, daß die jeweils gewünschten Wirkstoffmengen pro Flächeneinheit ausgebracht werden. Die Konzentration der Spritzbrühe wird so gewählt, daß in 10001 Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:

| | |
|---|---|
| 0 % | keine Wirkung (wie unbehandelte Kontrolle) |
| 100 % | totale Vernichtung |

In diesem Test zeigen beispielsweise die Verbindungen gemäß Herstellungsbeispiel 8 und 14 bei teilweise guter Verträglichkeit gegenuber Kulturpflanzen, wie z.B. Mais, starke Wirkung gegen Unkräuter (vgl. Tabelle A, "ai." = Wirkstoff).

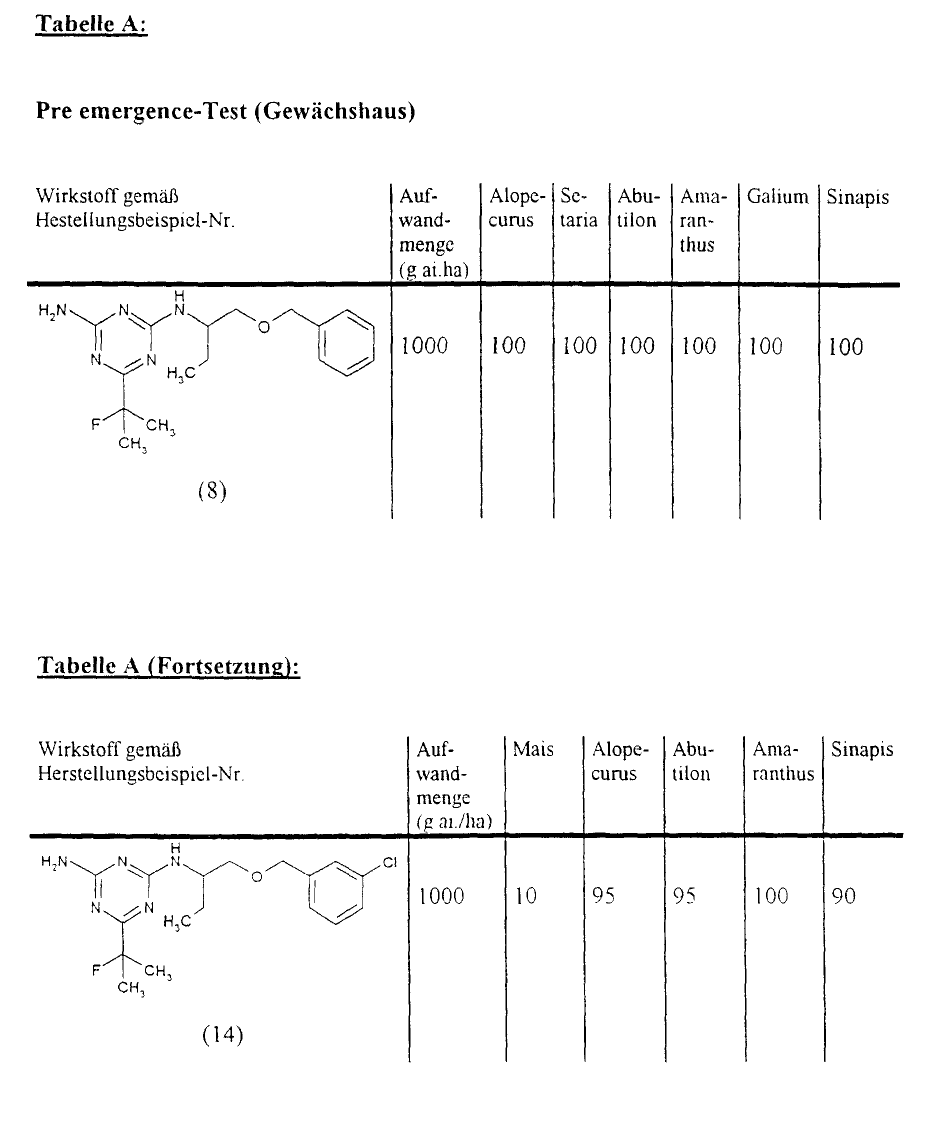

### Beispiel B

### Post-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 bis 15 cm haben so, daß die jeweils gewünschten Wirkstoffmengen pro Flächeneinheit ausgebracht werden. Die Konzentration der Spritzbrühe wird so gewählt, daß in 1000 1 Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:

| | |
|---|---|
| 0 % | keine Wirkung (wie unbehandelte Kontrolle) |
| 100 % | totale Vernichtung |

In diesem Test zeigen beispielsweise die Verbindungen gemäß Herstellungsbeispiel 8 und 14 starke Wirkung gegen Unkräuter (vgl. Tabelle B).

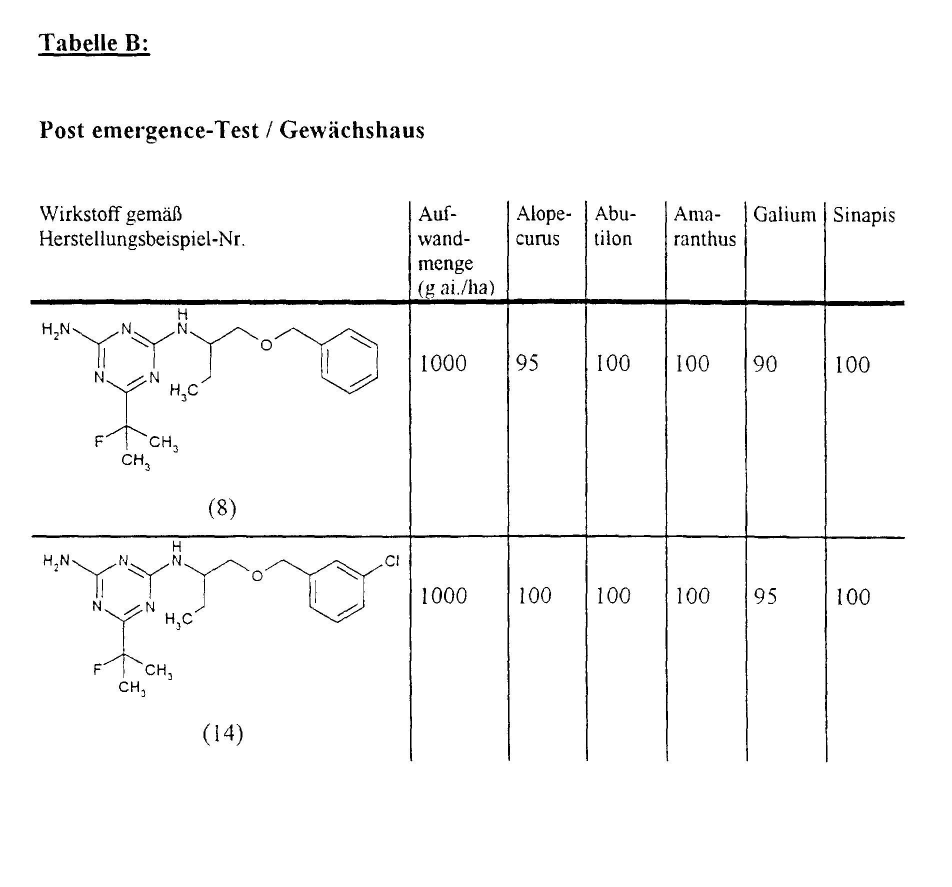

## Patentansprüche

1. Substituierte 2,4-Diamino-1,3,5-triazine der allgemeinen Formel (I) 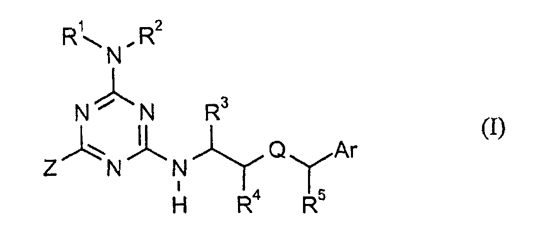 in welcher
Q für O (Sauerstoff), S (Schwefel), SO, SO₂, NH oder N(C₁-C₄-Alkyl) steht,
R¹ für Wasserstoff oder für gegebenenfalls durch Hydroxy, Cyano, Halogen oder C₁-C₄-Alkoxy substituiertes Alkyl mit 1 bis 6 Kohlenstoffatomen steht,
R² für Wasserstoff, für Formyl oder für jeweils gegebenenfalls durch Cyano, Halogen oder C₁-C₄-Alkoxy substituiertes Alkyl, Alkylcarbonyl, Alkoxycarbonyl oder Alkylaminocarbonyl mit jeweils 1 bis 6 Kohlenstoffatomen in den Alkylgruppen steht,
R³ für gegebenenfalls durch Hydroxy, Cyano, Halogen oder C₁-C₄-Alkoxy substituiertes Alkyl mit 1 bis 6 Kohlenstoffatomen oder für gegebenenfalls durch Cyano, Halogen oder C₁-C₄-Alkyl substituiertes Cycloalkyl mit 3 bis 6 Kohlenstoffatomen steht,
R⁴ für Wasserstoff oder Alkyl mit 1 bis 4 Kohlenstoffatomen steht,
R⁵ für Wasserstoff oder Alkyl mit 1 bis 4 Kohlenstoffatomen steht,
Ar für jeweils gegebenenfalls substituiertes Phenyl, Naphthyl, Tetralinyl oder Heteroaryl steht,
wobei die möglichen Heteroarylgruppierungen aus folgender Gruppe ausgewählt sind:
Furyl, Benzofuryl, Thienyl, Benzothienyl, Thiazolyl, Benzthiazolyl, Oxazolyl, Benzoxazolyl, Isoxazolyl, Thiadiazolyl, Oxadiazolyl, Pyrazolyl, Imidazolyl, Pyrrolyl, Pyridinyl, Pyrimidinyl,
und wobei die möglichen Substituenten jeweils aus folgender Gruppe ausgewählt sind:
Hydroxy, Cyano, Carbamoyl, Thiocarbamoyl, Nitro, Halogen, jeweils gegebenenfalls durch Hydroxy, Cyano oder Halogen substituiertes Alkyl oder Alkoxy mit jeweils 1 bis 6 Kohlenstoffatomen, jeweils gegebenenfalls durch Halogen substituiertes Alkylcarbonyl, Alkoxycarbonyl, Alkylthio, Alkylsulfinyl, Alkylsulfonyl, Dialkylamino, Alkylcarbonylamino, Alkylsulfonylamino, Bis-alkylcarbonyl-amino, Bis-alkylsulfonyl-amino, N-Alkyl-N-alkylcarbonyl-amino oder N-Alkyl-N-alkylsulfonyl-amino mit jeweils 1 bis 6 Kohlenstoffatomen in den Alkylgruppen, jeweils gegebenenfalls durch Hydroxy, Cyano, Nitro, Halogen, C₁-C₄-Alkyl, C₁-C₄-Halogenalkyl, C₁-C₄-Alkoxy oder C₁-C₄-Halogenalkoxy substiuiertes Phenyl oder Phenoxy, sowie jeweils gegebenenfalls durch Halogen substituiertes Methylendioxy oder Ethylendioxy,
und
Z für jeweils gegebenenfalls durch Hydroxy, Cyano, Halogen, C₁-C₄-Alkoxy, C₁-C₄-Alkyl-carbonyl, C₁-C₄-Alkoxy-carbonyl, C₁-C₄-Alkylthio, C₁-C₄-Alkylsulfinyl oder C₁-C₄-Alkylsulfonyl substituiertes Alkyl, mit 1 bis 6 Kohlenstoffatomen in den Alkylgruppen, oder für gegebenenfalls durch Cyano, Halogen oder C₁-C₄-Alkyl substituiertes Cycloalkyl mit 3 bis 6 Kohlenstoffatomen steht.

2. Verbindungen der Formel (I) gemäß Anspruch 1, **dadurch gekennzeichnet, dass** darin
R¹ für Wasserstoff oder für jeweils gegebenenfalls durch Hydroxy, Cyano, Fluor, Chlor, Methoxy oder Ethoxy substituiertes Methyl, Ethyl, n- oder i-Propyl, n-, i-, s- oder t-Butyl steht,
R² für Wasserstoff, für Formyl, oder für jeweils gegebenenfalls durch Cyano, Fluor, Chlor, Brom, Methoxy oder Ethoxy substituiertes Methyl, Ethyl, Acetyl, Propionyl, n- oder i-Butyroyl, Methoxycarbonyl, Ethoxycarbonyl, n- oder i-Propoxycarbonyl, Methylaminocarbonyl, Ethylaminocarbonyl, n- oder i-Propylaminocarbonyl steht,
R³ für jeweils gegebenenfalls durch Hydroxy, Cyano, Fluor, Chlor, Methoxy oder Ethoxy substituiertes Ethyl, n- oder i-Propyl, n-, i-, s- oder t-Butyl oder für jeweils gegebenenfalls durch Cyano, Fluor, Chlor, Methyl oder Ethyl substituiertes Cyclopropyl, Cyclobutyl, Cyclopentyl oder Cyclohexyl steht,
R⁴ für Wasserstoff oder Methyl steht,
R⁵ für Wasserstoff oder Methyl steht,
Ar für jeweils gegebenenfalls substituiertes Phenyl, Naphthyl, Tetralinyl oder Heteroaryl steht,
wobei die möglichen Heteroarylgruppierungen aus folgender Gruppe ausgewählt sind:
Furyl, Benzofuryl, Thienyl, Benzothienyl, Thiazolyl, Benzthiazolyl, Oxazolyl, Benzoxazolyl, Isoxazolyl, Thiadiazolyl, Oxadiazolyl, Pyrazolyl, Imidazolyl, Pyrrolyl, Pyridinyl, Pyrimidinyl,
und wobei die möglichen Substituenten jeweils aus folgender Gruppe ausgewählt sind:
Hydroxy, Cyano, Carbamoyl, Thiocarbamoyl, Nitro, Fluor, Chlor, Brom, jeweils gegebenenfalls durch Hydroxy, Cyano, Fluor oder Chlor substituiertes Methyl, Ethyl, n- oder i-Propyl, n-, i-, s- oder t-Butyl, Methoxy, Ethoxy, n- oder i-Propoxy, n-, i-, s- oder t-Butoxy, jeweils gegebenenfalls durch Fluor oder Chlor substituiertes Acetyl, Propionyl, n- oder i-Butyroyl, Methoxycarbonyl, Ethoxycarbonyl, n-oder i-Propoxycarbonyl, Methylthio, Ethylthio, n- oder i-Propylthio, Methylsulfinyl, Ethylsulfinyl, n- oder i-Propylsulfinyl, Methylsulfonyl, Ethylsulfonyl, n- oder i-Propylsulfonyl, Dimethylamino, Diethylamino, Acetylamino, Propionylamino, Methylsulfonylamino, Ethylsulfonylamino, Bis-acetyl-amino, Bis-methylsulfonyl-amino, N-Methyl-N-acetyl-amino oder N-Methyl-N-methylsulfonylamino, jeweils gegebenenfalls durch Hydroxy, Cyano, Nitro, Fluor, Chlor, Brom, Methyl, Ethyl, n- oder i-Propyl, n-, i-, s- oder t-Butyl, Trifluormethyl, Methoxy, Ethoxy, n- oder i-Propoxy, n-, i-, s- oder t-Butoxy, Difluormethoxy oder Trifluormethoxy substiuiertes Phenyl oder Phenoxy, sowie jeweils gegebenenfalls durch Fluor oder Chlor substituiertes Methylendioxy oder Ethylendioxy,
und
Z für jeweils gegebenenfalls durch Hydroxy, Cyano, Nitro, Fluor, Chlor, Methoxy, Ethoxy, n- oder i-Propoxy, n-, i-, s- oder t-Butoxy, Methylthio, Ethylthio, n- oder i-Propylthio, Methylsulfinyl, Ethylsulfinyl, n-oder i-Propylsulfinyl, Methylsulfonyl, Ethylsulfonyl, n- oder i-Propylsulfonyl substituiertes Methyl, Ethyl, n- oder i-Propyl, n-, i-, s- oder t-Butyl, oder für jeweils gegebenenfalls durch Cyano, Fluor, Chlor, Methyl oder Ethyl substituiertes Cyclopropyl, Cyclobutyl, Cyclopentyl oder Cyclohexyl steht.

3. Verfahren zur Herstellung von substituierten 2,4-Diaminotriazinen der Formel (I) gemäß Anspruch 1, **dadurch gekennzeichnet, daß** man
(a) Biguanide der allgemeinen Formel (II) 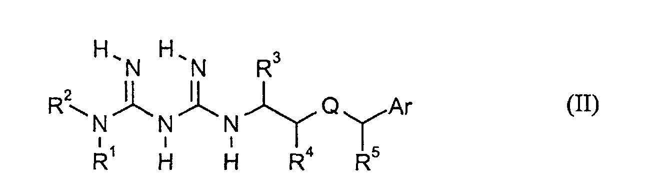 in welcher
Q, R¹, R², R³, R⁴, R⁵ und Ar die in Anspruch 1 angegebene Bedeutung haben,
- und/oder Säureaddukte von Verbindungen der allgemeinen Formel (II) -
mit Alkoxycarbonylverbindungen der allgemeinen Formel (III)
Z-CO-OR' (III)
in welcher
Z die in Anspruch 1 angegebene Bedeutung hat und
R' für Alkyl steht,
gegebenenfalls in Gegenwart eines Reaktionshilfsmittels und gegebenenfalls in Gegenwart eines Verdünnungsmittels umsetzt,
oder dass man
(b) substituierte Halogentriazine der allgemeinen Formel (IV) 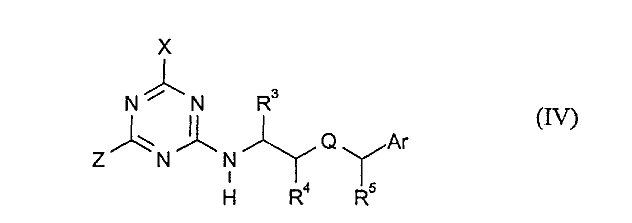 in welcher
Q, R³, R⁴, R⁵, Ar und Z die oben angegebene Bedeutung haben und
X für Halogen steht,
mit Stickstoffverbindungen der allgemeinen Formel (V) 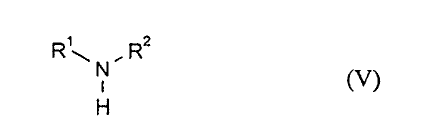 in welcher
R¹ und R² die oben angegebene Bedeutung haben,
gegebenenfalls in Gegenwart eines Reaktionshilfsmittels und gegebenenfalls in Gegenwart eines Verdünnungsmittels umsetzt,
oder dass man
(c) substituierte Aminotriazine der allgemeinen Formel (VI) 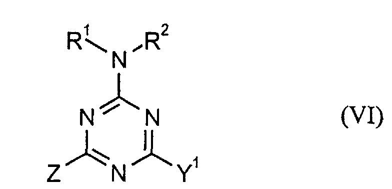 in welcher
R¹, R² und Z die oben angegebene Bedeutung haben und
Y¹ für Halogen oder Alkoxy steht,
mit substituierten Alkylaminen der allgemeinen Formel (VII) 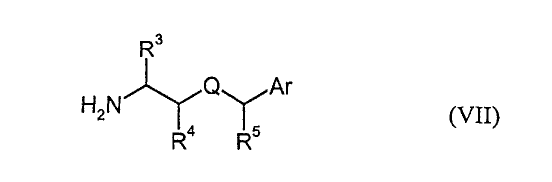 in welcher
Q, Ar, R³, R⁴ und R⁵ die oben angegebene Bedeutung haben,
gegebenenfalls in Gegenwart eines Reaktionshilfsmittels und gegebenenfalls in Gegenwart eines Verdünnungsmittels umsetzt,
oder dass man
(d) substituierte 2,4-Diamino-1,3,5-triazine der allgemeinen Formel (Ia) 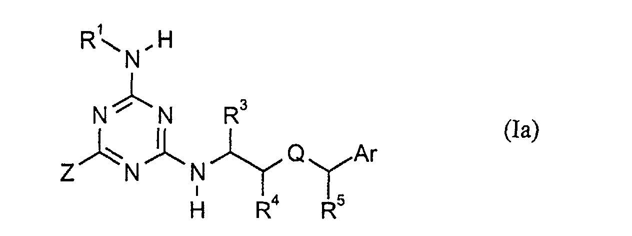 in welcher
Q, R¹, R³, R⁴, R⁵, Ar und Z die oben angegebene Bedeutung haben,
mit Alkylierungs- oder Acylierungsmitteln der allgemeinen Formel (VIII)
Y²-R² (VIII)
in welcher
R² mit Ausnahme von Wasserstoff die oben angegebene Bedeutung hat und
Y² für Halogen, -O-R² oder -O-CO-R² steht,
gegebenenfalls in Gegenwart eines Reaktionshilfsmittels und gegebenenfalls in Gegenwart eines Verdünnungsmittels umsetzt,
und gegebenenfalls an den gemäß den unter (a), (b), (c) oder (d) beschriebenen Verfahren erhaltenen Verbindungen der allgemeinen Formel (I) im Rahmen der obigen Substituentendefinition weitere Umwandlungen nach üblichen Methoden durchführt.

4. Herbizide Mittel, **gekennzeichnet durch** einen Gehalt an mindestens einer Verbindung der Formel (I) gemäß Anspruch 1.

5. Verwendung von Verbindungen der Formel (I) gemäß Anspruch 1 zur Bekämpfung von unerwünschtem Pflanzenwachstum.

6. Verfahren zur Bekämpfung von Unkräutern, **dadurch gekennzeichnet, dass** man Verbindungen der Formel (I) gemäß Anspruch 1 auf die Unkräuter oder ihren Lebensraum einwirken lässt.

7. Verfahren zur Herstellung von herbiziden Mitteln, **dadurch gekennzeichnet, daß** man Verbindungen der Formel (I) gemäß Anspruch 1 mit Streckmitteln und/oder oberflächenaktiven Mitteln vermischt.

8. Biguanide der allgemeinen Formel (II) 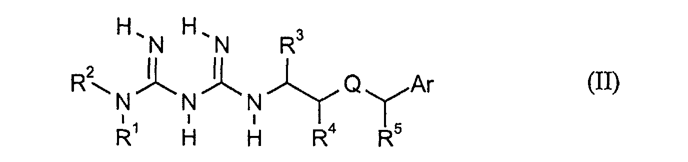 in welcher
Q, R¹, R², R³, R⁴, R⁵ und Ar die in Anspruch 1 angegebene Bedeutung haben,
und Säureaddukte von Verbindungen der allgemeinen Formel (II).

9. Substituierte Halogentriazine der allgemeinen Formel (IV) 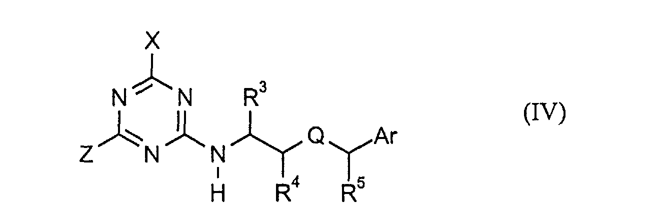 in welcher
Q, R³, R⁴, R⁵, Ar und Z die in Anspruch 1 angegebene Bedeutung haben und
X für Halogen steht.

## Claims

1. Substituted 2,4-diamino-1,3,5-triazines of the general formula (I) 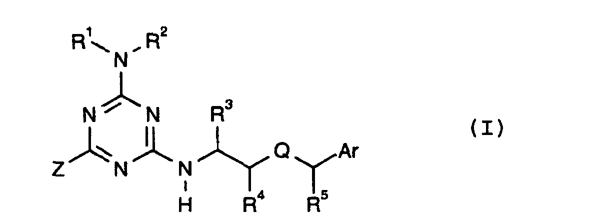 in which
Q represents O (oxygen), S (sulphur), SO, SO₂, NH or N(C₁-C₄-alkyl),
R¹ represents hydrogen or represents optionally hydroxyl-, cyano-, halogen- or C₁-C₄-alkoxy-substituted alkyl having 1 to 6 carbon atoms,
R² represents hydrogen, represents formyl or represents in each case optionally cyano-, halogen- or C₁-C₄-alkoxy-substituted alkyl, alkylcarbonyl, alkoxycarbonyl or alkylaminocarbonyl having in each case 1 to 6 carbon atoms in the alkyl groups,
R³ represents optionally hydroxyl-, cyano-, halogen- or C₁-C₄-alkoxy-substituted alkyl having 1 to 6 carbon atoms or represents optionally cyano-, halogen- or C₁-C₄-alkyl-substituted cycloalkyl having 3 to 6 carbon atoms,
R⁴ represents hydrogen or alkyl having 1 to 4 carbon atoms,
R⁵ represents hydrogen or alkyl having 1 to 4 carbon atoms,
Ar represents in each case optionally substituted phenyl, naphthyl, tetralinyl or heteroaryl,
where the possible heteroaryl groupings are selected from the following group:
furyl, benzofuryl, thienyl, benzothienyl, thiazolyl, benzothiazolyl, oxazolyl, benzoxazolyl, isoxazolyl, thiadiazolyl, oxadiazolyl, pyrazolyl, imidazolyl, pyrrolyl, pyridinyl, pyrimidinyl,
and where the possible substituents are in each case selected from the following group:
hydroxyl, cyano, carbamoyl, thiocarbamoyl, nitro, halogen, in each case optionally hydroxyl-, cyano- or halogen-substituted alkyl or alkoxy having in each case 1 to 6 carbon atoms, in each case optionally halogen-substituted alkylcarbonyl, alkoxycarbonyl, alkylthio, alkylsulphinyl, alkylsulphonyl, dialkylamino, alkylcarbonylamino, alkylsulphonylamino, bis-alkylcarbonyl-amino, bis-alkylsulphonylamino, N-alkyl-N-alkylcarbonyl-amino or N-alkyl-N-alkylsulphonyl-amino having in each case 1 to 6 carbon atoms in the alkyl groups, in each case optionally hydroxyl-, cyano-, nitro-, halogen-, C₁-C₄-alkyl-, C₁-C₄-halogenoalkyl-, C₁-C₄-alkoxy- or C₁-C₄-halogenoalkoxy-substituted phenyl or phenoxy, and in each case optionally halogen-substituted methylenedioxy or ethylenedioxy,
and
Z represents in each case optionally hydroxyl-, cyano-, halogen-, C₁-C₄-alkoxy-, C₁-C₄-alkylcarbonyl-, C₁-C₄-alkoxy-carbonyl-, C₁-C₄-alkylthio-, C₁-C₄-alkylsulphinyl- or C₁-C₄-alkylsulphonyl-substituted alkyl having 1 to 6 carbon atoms in the alkyl groups, or represents optionally cyano-, halogen- or C₁-C₄-alkyl-substituted cycloalkyl having 3 to 6 carbon atoms.

2. Compounds of the formula (I) according to Claim 1, **characterized in that**, therein,
R¹ represents hydrogen or represents in each case optionally hydroxyl-, cyano-, fluorine-, chlorine-, methoxy- or ethoxy-substituted methyl, ethyl, n- or i-propyl, n-, i-, s- or t-butyl,
R² represents hydrogen, represents formyl, or represents in each case optionally cyano-, fluorine-, chlorine-, bromine-, methoxy- or ethoxy-substituted methyl, ethyl, acetyl, propionyl, n- or i-butyroyl, methoxycarbonyl, ethoxycarbonyl, n- or i-propoxycarbonyl, methylaminocarbonyl, ethylaminocarbonyl, nor i-propylaminocarbonyl,
R³ represents in each case optionally hydroxyl-, cyano-, fluorine-, chlorine-, methoxy- or ethoxy-substituted ethyl, n- or i-propyl, n-, i-, s- or t-butyl or represents in each case optionally cyano-, fluorine-, chlorine-, methyl- or ethyl-substituted cyclopropyl, cyclobutyl, cyclopentyl or cyclohexyl,
R⁴ represents hydrogen or methyl,
R⁵ represents hydrogen or methyl,
Ar represents in each case optionally substituted phenyl, naphthyl, tetralinyl or heteroaryl,
where the possible heteroaryl groupings are selected from the following group:
furyl, benzofuryl, thienyl, benzothienyl, thiazolyl, benzothiazolyl, oxazolyl, benzoxazolyl, isoxazolyl, thiadiazolyl, oxadiazolyl, pyrazolyl, imidazolyl, pyrrolyl, pyridinyl, pyrimidinyl,
and where the possible substituents are in each case selected from the following group:
hydroxyl, cyano, carbamoyl, thiocarbamoyl, nitro, fluorine, chlorine, bromine, in each case optionally hydroxyl-, cyano-, fluorine- or chlorine-substituted methyl, ethyl, n- or i-propyl, n-, i-, s- or t-butyl, methoxy, ethoxy, n- or i-propoxy, n-, i-, s- or t-butoxy, in each case optionally fluorine- or chlorine-substituted acetyl, propionyl, n- or i-butyroyl, methoxycarbonyl, ethoxycarbonyl, n- or i-propoxycarbonyl, methylthio, ethylthio, n- or i-propylthio, methylsulphinyl, ethylsulphinyl, n- or i-propylsulphinyl, methylsulphonyl, ethylsulphonyl, n- or i-propylsulphonyl, dimethylamino, diethylamino, acetylamino, propionylamino, methylsulphonylamino, ethylsulphonylamino, bis-acetyl-amino, bis-methylsulphonyl-amino, N-methyl-N-acetylamino or N-methyl-N-methylsulphonylamino, in each case optionally hydroxyl-, cyano-, nitro-, fluorine-, chlorine-, bromine-, methyl-, ethyl-, n- or i-propyl-, n-, i-, s-or t-butyl-, trifluoromethyl-, methoxy-, ethoxy-, n- or i-propoxy-, n-, i-, s- or t-butoxy-, difluoromethoxy- or trifluoromethoxy-substituted phenyl or phenoxy, and in each case optionally fluorine- or chlorine-substituted methylenedioxy or ethylenedioxy,
and
Z represents in each case optionally hydroxyl-, cyano-, nitro-, fluorine-, chlorine-, methoxy-, ethoxy-, n- or i-propoxy-, n-, i-, s-or t-butoxy-, methylthio-, ethylthio-, n- or i-propylthio-, methylsulphinyl-, ethylsulphinyl-, n- or i-propylsulphinyl-, methylsulphonyl-, ethylsulphonyl-, n- or i-propylsulphonyl-substituted methyl, ethyl, nor i-propyl, n-, i-, s- or t-butyl, or represents in each case optionally cyano-, fluorine-, chlorine-, methyl- or ethyl-substituted cyclopropyl, cyclobutyl, cyclopentyl or cyclohexyl.

3. Process for preparing substituted 2,4-diaminotriazines of the formula (I) according to Claim 1, **characterized in that**
(a) biguanides of the general formula (II) 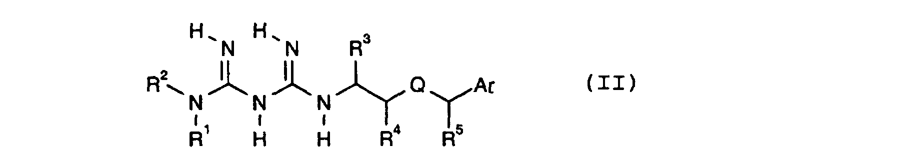 in which
Q, R¹, R², R³, R⁴, R⁵ and Ar are as defined in Claim 1
- and/or acid adducts of compounds of the general formula (II) -
are reacted with alkoxycarbonyl compounds of the general formula (III)
Z-CO-OR' (III)
in which
Z is as defined in Claim 1 and
R' represents alkyl,
if appropriate in the presence of a reaction auxiliary and if appropriate in the presence of a diluent,
or that
(b) substituted halogenotriazines of the general formula (IV) 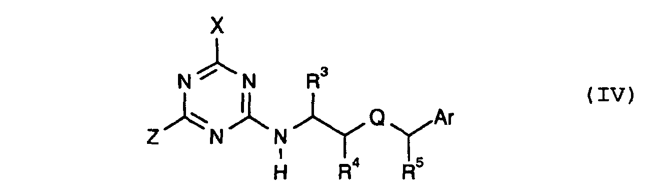 in which
Q, R³, R⁴, R⁵, Ar and Z are as defined above and
X represents halogen
are reacted with nitrogen compounds of the general formula (V) 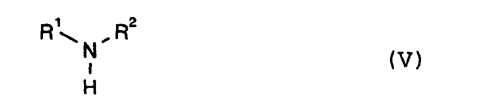 in which
R¹ and R² are as defined above,
if appropriate in the presence of a reaction auxiliary and if appropriate in the presence of a diluent,
or that
(c) substituted aminotriazines of the general formula (VI) 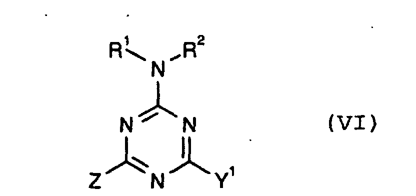 in which
R¹, R² and Z are as defined above and
Y¹ represents halogen or alkoxy,
are reacted with substituted alkyl amines of the general formula (VII) - 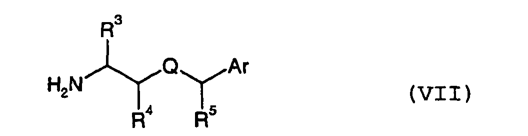 in which
Q, Ar, R³, R⁴ and R⁵ are as defined above,
if appropriate in the presence of a reaction auxiliary and if appropriate in the presence of a diluent, or that
(d) substituted 2,4-diamino-1,3,5-triazines of the general formula (Ia) 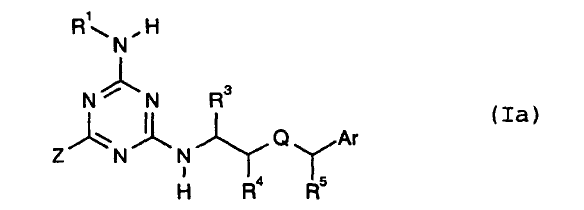 in which
Q, R¹, R³, R⁴, R⁵, Ar and Z are as defined above
are reacted with alkylating or acylating agents of the general formula (VIII)
Y²-R² (VIII)
in which
R² is as defined above, except for hydrogen, and
Y² represents halogen, -O-R² or -O-CO-R²,
if appropriate in the presence of a reaction auxiliary and if appropriate in the presence of a diluent,
and further conversions within the scope of the above definition of substituents are carried out by customary methods on the compounds of the general formula (I) obtained by the processes described under (a), (b), (c) or (d).

4. Herbicidal compositions, **characterized in that** they comprise at least one compound of the formula (I) according to Claim 1.

5. Use of compounds of the formula (I) according to Claim 1 for controlling undesirable vegetation.

6. Method for controlling weeds, **characterized in that** compounds of the formula (I) according to Claim 1 are allowed to act on the weeds or their habitat.

7. Process for preparing herbicidal compositions, **characterized in that** compounds of the formula (I) according to Claim 1 are mixed with extenders and/or surfactants.

8. Biguanides of the general formula (II) 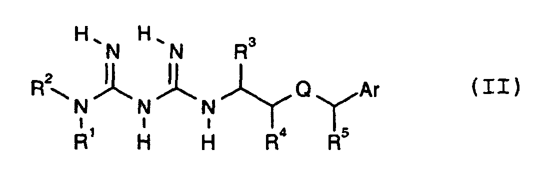 in which
Q, R¹, R², R³, R⁴, R⁵ and Ar are as defined in Claim 1
and acid adducts of compounds of the general formula (II).

9. Substituted halogenotriazines of the general formula (IV) 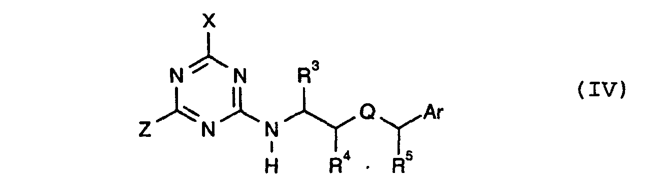 in which
Q, R³, R⁴, R⁵, Ar and Z are as defined in Claim 1 and
X represents halogen.

## Revendications

1. 2,4-diamino-1,3,5-triazines substituées de formule générale (I) 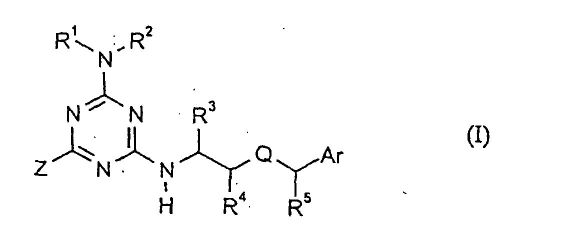 dans laquelle
Q représente O (oxygène), S (soufre), SO, SO₂, NH ou N(alkyle en C₁ à C₄),
R¹ est l'hydrogène ou un groupe alkyle ayant 1 à 6 atomes de carbone éventuellement substitué par un radical hydroxy, cyano, halogéno ou alkoxy en C₁ à C₄,
R² représente l'hydrogène, le groupe formyle ou un groupe alkyle, alkylcarbonyle, alkoxycarbonyle ou alkylaminocarbonyle ayant chacun 1 à 6 atomes de carbone dans les groupes alkyle et chacun portant éventuellement un substituant cyano, halogéno ou alkoxy en C₁ à C₄,
R³ désigne un reste alkyle ayant 1 à 6 atomes de carbone portant éventuellement un substituant hydroxy, cyano, halogéno ou alkoxy en C₁ à C₄, ou un reste cycloalkyle ayant 3 à 6 atomes de carbone portant éventuellement un substituant cyano, halogéno ou alkyle en C₁ à C₄,
R⁴ représente l'hydrogène ou un reste alkyle ayant 1 à 4 atomes de carbone,
R⁵ représente l'hydrogène ou un reste alkyle ayant 1 à 4 atomes de carbone,
Ar désigne un reste phényle, naphtyle, tétralinyle ou hétéroaryle dont chacun est éventuellement substitué, les groupements hétéroaryle possibles étant choisis dans le groupe suivant :
furyle, benzofuryle, thiényle, benzothiényle, thiazolyle, benzothiazolyle, oxazolyle, benzoxezolyle, isoxazolyle, thiadiazolyle, oxadiazolyle, pyrazolyle, imidazolyle, pyrrolyle, pyridinyle, pyrimidinyle,
et les substituants possibles étant choisis chacun dans le groupe suivant :
hydroxy, cyano, carbamoyle, thiocarbamoyle, nitro, halogéno, alkyle ou alkoxy ayant chacun 1 à 6 atomes de carbone et portant chacun un. substituant hydroxy, cyano ou halogéno, alkylcarbonyle, alkoxycarbonyle, alkylthio, alkylsulfinyle, alkylsulfonyle, dialkylamino, alkylcarbonylamino, alkylsulfonylamino, bis-alkylcarbonylamino, bis-alkylsulfonylamino, N-alkyl-N-alkylcarbonylamino ou N-alkyl-N-alkylsulfor,ylamino ayant chacun 1 à 6 atomes de carbone dans les groupes alkyle et portant chacun éventuellement un substituant halogéno, phényle ou phénoxy portant chacun, le cas échéant, un substituant hydroxy, cyano, nitro, halogéno, alkyle en C₁ à C₄, halogénalkyle en C₁ à C₄, alkoxy en C₁ à C₄ ou halogénalkoxy en C₁ à C₄, ainsi que méthylènedioxy ou éthylènedioxy portant chacun un substituant halogéno,
et
Z désigne un reste alkyle ayant 1 à 6 atomes de carbone dans les groupes alkyle, portant éventuellement dans chaque cas un substituant hydroxy, cyano, halogéno, alkoxy en C₁ à C₄, (alkyle en C₁ à C₄)carbonyle, (alkoxy en C₁ à C₄)carbonyle, alkylthio en C₁ à C₄, alkylsulfinyle en C₁ à C₄ ou alkylsulfonyle en C₁ à C₄, ou un reste cycloalkyle ayant 3 à 6 atomes de carbone portant éventuellement un substituant cyano, halogéno ou alkyle en C₁ à C₄.

2. Composés de formule (I) suivant la revendication 1, **caractérisés en ce que** dans la formule :
R¹ désigne l'hydrogène ou un reste méthyle, éthyle, n-propyle, isopropyle, n-butyle, isobutyle, sec.-butyle ou tertio-butyle portant chacun, le cas échéant, un substituant hydroxy, cyano, fluoro, chloro, méthoxy ou éthoxy,
R² désigne l'hydrogène, le reste formyle, un reste méthyle, éthyle, acétyle, propionyle, n-butyroyle, isobutyroyle, méthoxycarbonyle, éthoxycarbonyle, n-propoxycarbonyle, isopropoxycarbonyle, méthylaminocarbonyle, éthylaminocarbonyle, n-propylaminocarbonyle ou isopropylaminocarbonyle portant chacun, le cas échéant, un substituant cyano, fluoro, chloro, bromo, méthoxy ou éthoxy,
R³ désigne un reste éthyle, n-propyle, isopropyle, n-butyle, isobutyle, sec.-butyle ou tertio-butyle portant chacun, le cas échéant, un substituant hydroxy, cyano, fluoro, chloro, méthoxy ou éthoxy, ou un reste cyclopropyle, cyclobutyle, cyclopentyle ou cyclohexyle portant chacun éventuellement un substituant cyano, fluoro, chloro, méthyle ou éthyle,
R⁴ est l'hydrogène ou le groupe méthyle,
R⁵ est l'hydrogène ou le groupe méthyle,
Ar est un reste phényle, naphtyle, tétralinyle ou hétéroaryle éventuellement substitué dans chaque cas,
les groupements hétéroaryle possibles étant choisis dans le groupe suivant :
furyle, benzofuryle, thiényle, benzothiényle, thiazolyle, benzothiazolyle, oxazolyle, benzoxazolyle, isoxazolyle, thiadiazolyle, oxadiazolyle, pyrazolyle, imidazolyle, pyrrolyle, pyridinyle, pyrimidinyle,
et les substituants possibles étant choisis chacun dans le groupe suivant :
hydroxy, cyano, carbamoyle, thiocarbamoyle, nitro, fluor, chlore, brome, méthyle, éthyle, n-propyle, isopropyle, n-butyle, isobutyle, sec.-butyle, tertio-butyle, méthoxy, éthoxy, n-propoxy, isopropoxy, n-butoxy, isobutoxy, sec.-butoxy ou tertio-butoxy portant chacun, le cas échéant, un substituant hydroxy, cyano, fluoro ou chloro, acétyle, propionyle, n-butyroyle, isobutyroyle, méthoxycarbonyle, éthoxycarbonyle, n-propoxycarbonyle, isopropoxycarbonyle, méthylthio, éthylthio, n-propylthio, isopropylthio, méthylsulfinyle, éthylsulfinyle, n-propylsulfinyle, isopropylsulfinyle, méthylsulfonyle, éthylsulfonyle, n-propylsulfonyle, isopropylsulfonyle, diméthylamino, diéthylamino, acétylamino, propionylamino, méthylsulfonylamino, éthylsulfonylamino, bis-acétylamino, bis-méthylsulfonylamino, N-méthyl-N-acétylamino ou N-méthyl-N-méhylsulfonylamino portant chacun, le cas échéant, un substituant fluoro ou chloro, phényle ou phénoxy portant chacun, le cas échéant, un substituant hydroxy, cyano, nitro, fluoro, chloro, bromo, méthyle, éthyle, n-propyle, isopropyle, n-butyle, isobutyle, sec.-butyle, tertio-butyle, trifluorométhyle, méthoxy, éthoxy, n-propoxy, isopropoxy, n-butoxy, isobutoxy, sec.-butoxy, tertio-butoxy, difluorométhoxy ou trifluorométhoxy, ainsi que méthylènedioxy ou éthylènedioxy portant chacun, le cas échéant, un substituant fluoro ou chloro,
et
Z désigne un reste méthyle, éthyle, n-propyle, isopropyle, n-butyle, isobutyle, sec.-butyle ou tertio-butyle portant chacun, le cas échéant, un substituant hydroxy, cyano, nitro, fluoro, chloro, méthoxy, éthoxy, n-propoxy, isopropoxy, n-butoxy, isobutoxy, sec-butoxy, tertio-butoxy, méthylthio, éthylthio, n-propylthio, isopropylthio, méthylsulfinyle, éthylsulfinyle, n-propylsulfinyle, isopropylsulfinyle, méthylsulfonyle, éthylsulfonyle, n-propylsulfonyle ou isopropylsulfonyle ou un reste cyclopropyle, cyclobutyle, cyclopentyle ou cyclohexyle portant éventuellement, dans chaque cas, un substituant cyano, fluoro, chloro, méthyle ou éthyle.

3. Procédé de production de 2,4-diaminotriazines substituées de formule (I) suivant la revendication 1, **caractérisé en ce que** :
(a) on fait réagir des biguanides de formule générale (II) 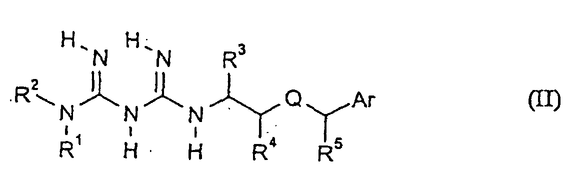 dans laquelle
Q, R¹, R², R³, R⁴, R⁵ et Ar ont la définition indiquée dans la revendication 1,
- et/ou des sels d'addition d'acides de composés de formule générale (II) -
avec des composés alkoxycarbonyliques de formule générale (III)
Z-CO-OR' (III)
dans laquelle
Z a la définition indiquée dans la revendication 1 et
R' est un reste alkyle,
éventuellement en présence d'un auxiliaire de réaction et, le cas échéant, en présence d'un diluant,
ou **en ce que** :
(b) on fait réagir des halogénotriazines substituées de formule générale (IV) 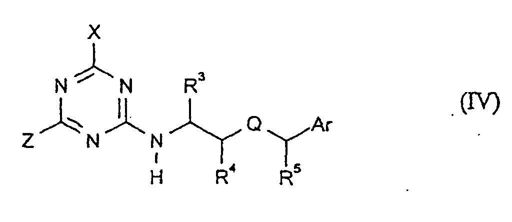 dans laquelle
Q, R³, R⁴, R⁵, Ar et Z ont la définition indiquée ci-dessus et
X représente un halogène,
avec des composés azotés de formule générale (V) 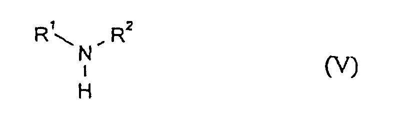 dans laquelle
R¹ et R² a la définition indiquée ci-dessus,
éventuellement en présence d'un auxiliaire de réaction et, le cas échéant, en présence d'un diluant,
ou **en ce que**
(c) on fait réagir des aminotriazines substituées de formule générale (VI) 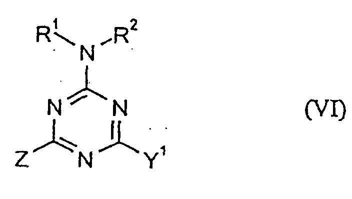 dans laquelle
R¹, R² et Z ont la définition indiquée ci-dessus et
Y¹ représente un halogène ou un reste alkoxy,
avec- des alkylamines substituées de formule générale (VII) 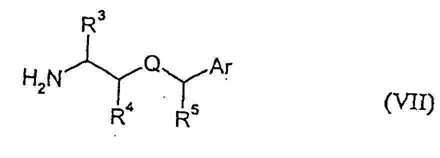 dans laquelle
Q, Ar, R³, R⁴ et R⁵ ont la définition indiquée ci-dessus,
éventuellement en présence d'un auxiliaire de réaction et, le cas échéant, en présence d'un diluant,
ou **en ce que**
(d) on fait réagir des 2,4-diamino-1,3,5-triazines substituées de formule générale (Ia) 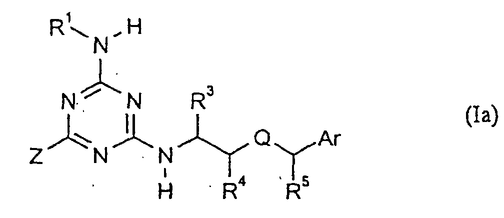
dans laquelle
Q, R¹, R³, R⁴, R⁵, Ar et Z ont la définition indiquée ci-dessus,
avec des agents d'alkylation ou d'acylation de formule générale (VIII)
Y²-R² (VIII)
dans laquelle
R² a la définition indiquée ci-dessus, à l'exception de l'hydrogène, et
Y² représente un halogène, un groupe -O-R² ou -O-CO-R²,
éventuellement en présence d'un auxiliaire de réaction et, le cas échéant, en présence d'un diluant,
et on conduit éventuellement d'autres transformations selon des modes opératoires usuels sur les composés de formule générale (I) obtenus selon les procédés décrits en (a), (b), (c) ou (d), dans le cadre des définitions des substituants indiquées ci-dessus.

4. Composition herbicide, **caractérisée par** une teneur en au moins un composé de formule (I) suivant la revendication 1.

5. Utilisation de composés de formule (I) suivant la revendication 1 pour combattre une végétation indésirable.

6. Procédé pour combattre des mauvaises herbes, **caractérisé en ce qu'**on fait agir des composés de formule (I) suivant la revendication 1 sur les mauvaises herbes ou sur leur milieu.

7. Procédé de préparation de compositions herbicides, **caractérisé en ce qu'**on mélange des composés de formule (I) suivant la revendication 1 avec des diluants et/ou des agents tensio-actifs.

8. Biguanides de formule générale (II) 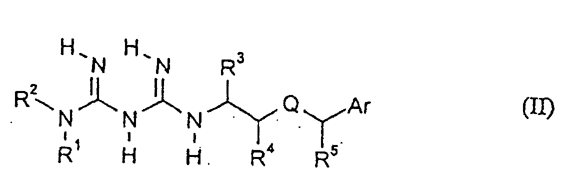 dans laquelle
Q, R¹, R², R³, R⁴, R⁵ et Ar ont la définition indiquée dans la revendication 1,
et sels d'addition d'acides de composés de formule générale (II).

9. Halogénotriazines substituées de formule générale (IV) 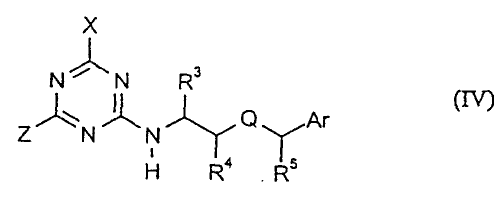 dans laquelle
Q, R³, R⁴, R⁵, Ar et Z ont la définition indiquée dans la revendication 1
et
X désigne un halogène.
